# EUROPEAN PATENT SPECIFICATION

(11) **EP 1 541 197 B1**
(45) Date of publication and mention of the grant of the patent: **18.03.2009**
(21) Application number: 04025035.9
(22) Date of filing: 21.10.2004
(51) Int. Cl.: A61K 31/343, A61K 31/4545, A61P 25/22, A61P 25/24

(54) **Use of pipamperone and an SNDRI, SNRI or SSRI for the treatment of mood or anxiety disorders**
Verwendung von Pipamperon und einem SNDRI, SNRI oder SSRI zur Behandlung von Stimmungs- oder Angststörungen
Utilisation de pipamperone et d'un SNDRI, SNRI ou SSRI pour le traitement des troubles de l'humeur ou anxieux

(30) Priority: 02.12.2003 EP 03447279; 05.01.2004 EP 04447001; 18.03.2004 EP 04447066
(43) Date of publication of application: 15.06.2005
(73) Proprietor: PharmaNeuroBoost N.V., 3570 Alken (BE)
(72) Inventor: Buntinx, Erik, 3750 Alken (BE)
(74) Representative: De Clercq, Ann G. Y.

(56) References cited:
- WO-A-98/43646
- DE-A- 4 039 631
- US-A- 5 762 960
- VOLMAT R ET AL: "The treatment of depressions by Cledial. Evolution and clinical state and handwriting" PSYCHOLOGIE MEDICALE 1986 FRANCE, vol. 18, no. 10, 1986, pages 1615-1622, XP009028776
- SQUELART P ET AL: "Pipamperone (Dipiperon), a useful sedative neuroleptic drug in troublesome chronic psychotic patients." ACTA PSYCHIATRICA BELGICA. BELGIUM 1977 MAR-APR, vol. 77, no. 2, March 1977 (1977-03), pages 284-293, XP009028314 ISSN: 0300-8967
- KOCH H J ET AL: "Successful therapy of tardive dyskinesia in a 71-year-old woman with a combination of tetrabenazine, olanzapine and tiapride." INTERNATIONAL JOURNAL OF CLINICAL PRACTICE. ENGLAND MAR 2003, vol. 57, no. 2, March 2003 (2003-03), pages 147-149, XP009030046 ISSN: 1368-5031
- DIEBOLD K. ET AL: "Are psychoactive-drug-induced changes in plasma lipid and lipoprotein levels of significance for clinical remission in psychiatric disorders?." PHARMACOPSYCHIATRY, (1998) 31/2 (60-67)., 1998, XP009029360
- PERUGI G ET AL: "EFFECTIVENESS OF ADJUNCTIVE GABAPENTIN IN RESISTANT BIPOLAR DISORDER: IS IT DUE TO ANXIOUS-ALCOHOL ABUSE COMORBIDITY?" JOURNAL OF CLINICAL PSYCHOPHARMACOLOGY, WILLIAMS AND WILKINS, US, vol. 22, no. 6, 2002, pages 584-591, XP009029358 ISSN: 0271-0749
- ADLER L ET AL: "PRAXIS DER STATIONAEREN AKUTBEHANDLUNG VON MANIEN RETROSPEKTIVE VERGLEICHSUNTERSUCHUNG AN JE 100 PATIENTEN ZWEIER PSYCHIATRISCHER ZENTREN PRACTICE OF IN-PATIENT ACUTE TREATMENT OF MANIAS" FORTSCHRITTE DER NEUROLOGIE PSYCHIATRIE, STUTTGART, DE, vol. 62, no. 12, 1994, pages 479-488, XP009029389 ISSN: 0720-4299
- ANSOMS C ET AL: "Sleep disorders in patients with severe mental depression: double-blind placebo-controlled evaluation of the value of pipamperone (Dipiperon)." ACTA PSYCHIATRICA SCANDINAVICA. FEB 1977, vol. 55, no. 2, February 1977 (1977-02), pages 116-122, XP009041169 ISSN: 0001-690X
- LEYSEN J E ET AL: "RECEPTOR INTERACTI0NS OF NEW ANTIPSYCHOTICS: RELATION TO PHARMACODYNAMIC AND CLINICAL EFFECTS" INTERNATIONAL JOURNAL OF PSYCHIATRY IN CLINICAL PRACTICE, MARTIN DUNITZ, LONDON, GB, vol. 2, no. 1, 1998, pages S03-S17, XP001009585 ISSN: 1365-1501
- DATABASE BIOSIS [Online] BIOSCIENCES INFORMATION SERVICE, PHILADELPHIA, PA, US; 1996, SCHOTTE A ET AL: "Risperidone compared with new and reference antipsychotic drugs: In vitro and in vivo receptor binding" XP002290498 Database accession no. PREV199698829913 & PSYCHOPHARMACOLOGY, vol. 124, no. 1-2, 1996, pages 57-73, ISSN: 0033-3158
- VANHOENACKER P ET AL: "EFFICIENT EXPRESSION OF THE HUMAN DOPAMINE D4.2 RECEPTOR: POSITIVE INFLUENCE OF PIPAMPERONE ON EXPRESSION LEVELS" ABSTRACTS OF THE SOCIETY FOR NEUROSCIENCE, SOCIETY FOR NEUROSCIENCE, WASHINGTON, DC, US, vol. 26, no. 1/2, 2000, page 1, XP001181469 ISSN: 0190-5295
- VIJVER VAN DE D A M C ET AL: "ANTIPSYCHOTICS AND PARKINSON'S DISEASE: ASSOCIATION WITH DISEASE AND DRUG CHOICE DURING THE FIRST 5 YEARS OF ANTIPARKINSONIAN DRUG TREATMENT" EUROPEAN JOURNAL OF CLINICAL PHARMACOLOGY, SPRINGER VERLAG, DE, vol. 58, no. 7, 2002, pages 157-161, XP009033980 ISSN: 0031-6970
- ENGELBORGHS S ET AL: "AMINO ACIDS AND BIOGENIC AMINES IN CEREBROSPINAL FLUID OF PATIENTS WITH PARKINSON'S DISEASE" NEUROCHEMICAL RESEARCH, PLENUM PRESS, NEW YORK, US, vol. 28, no. 8, August 2003 (2003-08), pages 1145-1150, XP009031514 ISSN: 0364-3190
- DATABASE EMBASE [Online] ELSEVIER SCIENCE PUBLISHERS, AMSTERDAM, NL; 1999, ETCHEPAREBORDA M C: "Neurocognitive and pharmacological approach to specific learning disorders" XP002312308 Database accession no. EMB-2000050033 & REVISTA DE NEUROLOGIA 1999 SPAIN, vol. 28, no. SUPPL. 2, 1999, pages S81-S93, ISSN: 0210-0010
- WIRZ-JUSTICE A ET AL: "HALOPERIDOL DISRUPTS, CLOZAPINE REINSTATES THE CIRCADIAN REST-ACTIVITY CYCLE IN A PATIENT WITH EARLY-ONSET ALZHEIMER DISEASE" ALZHEIMER DISEASE AND ASSOCIATED DISORDERS, RAVEN PRESS, NEW YORK, NY, US, vol. 14, no. 4, 2000, pages 212-215, XP009029353 ISSN: 0893-0341
- FAHS H ET AL: "THYMOREGULATEURS DANS L'AGITATION ET LES TROUBLES DU COMPORTEMENT CHEZ LE SUJET DEMENT A PROPOS DE HUIT CAS ANTICONVULSIVANTS AND AGGRESSIVE BEHAVIORS IN ALZHEIMER'S DISEASE. EIGHT CASES REPORTS" ENCEPHALE, PARIS, FR, vol. 25, no. 2, 1999, pages 169-174, XP009039295 ISSN: 0013-7006
- GRÖZINGER M ET AL: "Melperone is an inhibitor of the CYP2D6 catalyzed O-demethylation of venlafaxine." PHARMACOPSYCHIATRY. GERMANY JAN 2003, vol. 36, no. 1, January 2003 (2003-01), pages 3-6, XP009029363 ISSN: 0176-3679
- WERTH ESTHER ET AL: "Decline in long-term circadian rest-activity cycle organization in a patient with dementia." JOURNAL OF GERIATRIC PSYCHIATRY AND NEUROLOGY. 2002 SPRING, vol. 15, no. 1, April 2002 (2002-04), pages 55-59, XP009042127 ISSN: 0891-9887
- STAHL STEPHEN M ET AL: "Examination of nighttime sleep-related problems during double-blind, placebo-controlled trials of galantamine in patients with Alzheimer's disease." CURRENT MEDICAL RESEARCH AND OPINION. APR 2004, vol. 20, no. 4, April 2004 (2004-04), pages 517-524, XP009041652 ISSN: 0300-7995
- DATABASE BIOSIS [Online] BIOSCIENCES INFORMATION SERVICE, PHILADELPHIA, PA, US; 1995, MELTZER HERBERT Y ET AL: "Plasma clozapine levels and the treatment of L-DOPA-induced psychosis in Parkinson's disease. A high potency effect of clozapine" XP002290499 Database accession no. PREV199598192811 & NEUROPSYCHOPHARMACOLOGY, vol. 12, no. 1, 1995, pages 39-45, ISSN: 0893-133X
- MUENCHAU A ET AL: "PHARMACOLOGICAL TREATMENT OF PARKINSON'S DISEASE" POSTGRADUATE MEDICAL JOURNAL, MCMILLAN PRESS, BASINGSTOKE, GB, vol. 76, no. 900, October 2000 (2000-10), pages 602-610, XP009034316 ISSN: 0032-5473
- FALTRACO F ET AL: "AKTUELLE THERAPIEMOEGLICHKEITEN DER ALZHEIMER DEMENZ CURRENT THERAPEUTICAL STRATEGIES IN DEMENTIA" NEUROLOGIE UND REHABILITATION, BONN, DE, vol. 9, no. 1, 2003, pages 15-22, XP009041267 ISSN: 0947-2177
- MONTGOMERY S.A.; ANDERSEN H.F.: 'Escitalopram versus venlafaxine XR in the treatment of depression' INTERNATIONAL CLINICAL PHARMACOLOGY vol. 21, no. 5, 2006, pages 297 - 309
- KHAN ARIF; WARNER HEATHER A.; BROWN WALTER A.: 'Symptom reduction and suicide risk in patients treated with placebo in antidepressant clinical trials: An analysis of the Food and Drug Administration database' ARCHIVES OF GENERAL PSYCHIATRY vol. 57, no. 4, 2000, pages 311 - 317
- MOORE NICHOLAS; VERDOUX HÉLÈNE; FANTINO BRUNO: 'Prospective, multicentre, randomized, double-blind study of the efficacy of escitalopram versus citalopram in outpatient treatment of major depressive disorder' INTERNATIONAL CLINICAL PSYCHOPHARMACOLOGY vol. 20, no. 3, 2005, pages 131 - 137
- WIELING W. ET AL: 'INITIAL ORTHOSTATIC HYPOTENSION AS A CAUSE OF RECURRENT SYNCOPE: A CASE REPORT' CLINICAL AUTONOMIC RESEARCH vol. 11, no. 4, 2001, page 270, XP009039296
- FOUKS D. ET AL: 'TRAITEMENT DES TROUBLES CARACTERIELS PAR UNE NOUVELLE BUTYROPHENOME: LE R. 3345 OU PIPAMPERONE//TREATMENT OF CHARACTER DISORDERS WITH A NEW BUTYROPHENONE: R. 3345 OR PIPAMPERONE' ANNALES MEDICO-PSYCHOLOGIQUES vol. 124, no. 5, 1966, pages 677 - 681, XP009028328
- "Pipamperone dichlorhydrate"[Online] 2000, pages 1-4, Retrieved from the Internet: URL:http://www.biam2.org/www/Sub2783.html> [retrieved on 2007]
- 'Médicaments psychotropes: posologies chez l'enfant et l'adolescent', [Online] 2001, pages 1 - 3 Retrieved from the Internet: <URL:http://www.adiph.org/pic/pedia-neurole ptiques.pdf> [retrieved on 2007-00-00]
- HEISER P.; REMSCHMIDT H.: 'SELECTIVE SEROTONIN REUPTAKE INHIBITORS AND NEWER ANTIDEPRESSIVE SUBSTANCES IN CHILD AND ADOLESCENT PSYCHIATRY. DIE SELEKTIVEN SEROTONIN-WIEDERAUFNAHMEHEMMER UND DIE NEUEREN ANTIDEPRESSIVA-SUBSTANZEN IN DER KINDER- UND JUGENDPSYCHIATRIE' ZEITSCHRIFT FUER KINDER- UND JUGENDPSYCHIATRIE UNDPSYCHOTHERAPIE vol. 30, no. 3, 2002, pages 173 - 183, XP009076880
- CARLIER P.R. ET AL: 'Synthesis of a Potent Wide-Spectrum Serotonin-, Norepinephrine-, Dopamine-Reuptake Inhibitor (SNDRI) and a Species-Selective Dopamine-Reuptake Inhibitor Based on the Gamma-Amino Alcohol Functional Group' BIOORGANIC & MEDICINAL CHEMISTRY LETTERS vol. 8, no. 5, 1998, pages 487 - 492
- MAINA GIUSEPPE ET AL: 'ANTIPSYCHOTIC AUGMENTATION FOR TREATMENT RESISTANT OBSESSIVE-COMPULSIVE DISORDER: WHAT IF ANTIPSYCHOTIC IS DISCONTINUED?' INTERNATIONAL CLINICAL PSYCHOPHARMACOLOGY vol. 18, no. 1, 01 January 2003, pages 23 - 28, XP009078546

## Description

### Field of the invention

The invention relates to the field of neuropsychiatry. More specifically, the invention relates to the use of compounds, which have D4 and 5-HT2A antagonist, inverse agonist or partial agonist activity, for the preparation of medicaments:

### Background of the invention

Conventionally, mental disorders are divided into types based on criteria sets with defining features. DSM-IV (*American Psychiatric Association,* (1993 - ISBN 0 - 89042 - 061 - 0)) is the in the art well-known golden standard of such a categorical classification. In DSM-IV, there is no assumption that each category of mental disorder is a completely discrete entity with absolute boundaries dividing it from other mental disorders or from no mental disorder. There is also no assumption that all individuals described as having the same mental disorder are alike in all important ways. Individuals sharing a diagnosis are likely to be heterogeneous even in regard to the defining features of the diagnosis. Thus, the categorical defined mental disorders as mood and anxiety disorders are having an external and even internal variable co-incidence of symptoms concerning e.g. mood, anxiety, perception, feeding, somatic sensations, sexual functions, sleep, cognitive functioning, impulse control, attention, substance use, personality, bereavement, identity, phase of life, abuse or neglect and other aspects of behavior.

In a dimensional system, clinical presentations are classified based on quantification of attributes i.e. dysfunctions rather than the assignment to categories and works best in describing phenomena that are distributed continuously and that do not have clear boundaries.

Emotion dysregulation is known as such an attribution or dysfunction that plays an important role in the development and course of mental disorders (Gross, J. J. & Munoz, R. F., 1995, Emotion regulation and mental health, Clinical Psychology: Science and Practice, 2, 151-164*;* Mennin, D.S., Heimberg, R. G., Turk, C. L. & Fresco, D. M., 2002, Applying an emotion regulation framework to integrative approaches to generalized anxiety disorder, Clinical Psychology: Science and Practice, 9, 85-90*;* Linehan, M. M., 1993, Cognitive-behavioral treatment of borderline personality disorder, New York, The Guilford Press*;* Gratz, K. L., Roemer, L., 2001 & 2004, Multidimensional assessment of emotion regulation and dysregulation: development, factor structure, and initial validation of the Difficulties in Emotion Regulation Scale, Annual meeting of the Association for Advancement of Behavior Therapy, Nov. 2001 *&* Journal of Psychopathology and Behavioral Assessment, Vol. 26, No. 1, March 2004) besides behavioural and cognitive dysfunctions. D4 dopamine receptors (D4DR), almost exclusively present in the mesocortical and mesolimbic systems (O'Malley, K. L., Harmon, S., Tang, L., Todd, R. D., The rat dopamine D4 receptor: sequence, gene structure, and demonstration of expression in the cardiovascular system, New Biol., 4, 137-46, 1992), are in the art known as modulators of emotion and cognition. D4DR agonistic activity gives a behavioural sensitisation; D4DR antagonistic activity leads to an emotion modulation (Svensson, T. H., Mathé, A. A., Monoaminergic Transmitter Systems, Biological Psychiatry (eds. D'Haenen, H., et al.), 45-66, 2002, John Wiley & Sons, Ltd*)*. Data demonstrate that agonism of the dopamine D4 receptors play an important role in the induction of behavioral sensitization to amphetamine and accompanying adaptations in pre- and postsynaptic neural systems associated with the mesolimbocortical dopamine projections (D. L. Feldpausch et al.; The Journal of Pharmacology and Experimental Therapeutics Vol. 286, Issue 1, 497-508, July 1998).

Results suggest that the antagonisms of cortical D2 dopamine receptors are a common target of traditional and atypical antipsychotics for therapeutic action. Higher *in vivo* binding to the D2 receptors in the cortex than in the basal ganglia is suggested as an indicator of favorable profile for a putative antipsychotic compound (X. Xiberas and J.L. Martinot; The British Journal of Psychiatry (2001) 179: 503-508). Results show that dopamine D4 receptor antagonism in the brain does not result in the same neurochemical consequences (increased dopamine metabolism or hyperprolactinemia) observed with typical neuroleptics (Smita Patel et al., The Journal of Pharmacology and Experimental Therapeutics Vol. 283, Issue 2, 636-647,1997). The selective D4 dopamine receptor antagonist L-745,870 was ineffective as an antipsychotic for the treatment of neuroleptic responsive patients with acute schizophrenia (Kramer, M.S. et al., Arch. Gen. Psychiatry 1997 Dec; 54(12):1080).

Finally, in the biological system, mental disorders are defined on other levels of abstraction than in the categorical and dimensional system. Structural pathology (e.g. amyloid plaques in Alzheimer Disease), etiology (e.g. HIV Dementia) and deviance from a physiological norm (e.g. reduced cerebral blood flow) are often used as indicative biological markers for a mental disorder. The underlying dysregulation of various neurotransmittor systems (glutaminergic, GABAergic, cholinergic, monoaminergic (noradrenergic, dopaminergic, serotonergic), etc.) is the in the art used model for the explanation of the biological determinants of the clinical presentation of mental disturbances. It is known that the Serotonin 2A Receptor (5-HT2A receptor) - which is widespread in the Central Nervous System (CNS) - has a regulating role on the dysregulation of various neuro-transmittor systems. 5-HT2A agonism gives several behavioural disturbances; 5-HT2A antagonism leads to a governance of mood, social behaviour, anxiety, cognitive function, stress, sleep functions, nociception, sexual functions, feeding and other aspects of behaviour (J.E. Leysen (2004) 5-HT2 Receptors; Current Drug Targets - CNS & Neurological Disorders, 2004, 3, 11-26*)*.

Dysregulation of the HPA axis (hypothalamic - pituitary - adrenal axis) has frequently been reported in patients with psychiatric disorders, and is among the most robustly demonstrated neurobiological changes among psychiatric patients *(*D.A. Gutman and C.B. Nemeroff, Neuroendocrinology, Biological Psychiatry (eds. D'Haenen, H., et al), 99, 2002, John Wiley & Sons, Ltd*).* The resulting elevated plasma cortisol concentrations leads to an enhanced binding of serotonin for the 5-HT2A receptor (E. A. Young, Mineralocorticoid Receptor Function in Major Depression, Arch Gen Psychiatry, Jan 2003; 60: 24 - 28) and thus agonism.

Additionally 5-HT2A antagonism gives a des-inhibiting of the inhibitory effect of the 5-HT2A receptor on (i) the 5-HT1A receptor stimulation by serotonin (S. M. Stahl, Newer Antidepressants and Mood Stabilizers, Essential Psychopharmacology, 265, University Press; 2 edition (June 15, 2000); ISBN: 0521646154*)* and on (ii) the dopamine release in the mesocortical systems (S. M. Stahl, Classical Antidepressants, Serotonin Selective and Noradrenargic Reuptake Inhibitors, Essential Psychopharmacology, 233 , University Press; 2 edition (June 15, 2000); ISBN: 0521646154).

Clinical or real effectiveness of psychopharma is very rare via common pooping-out; many treatment-refractory patients and up to half of patients fail to attain remission (S. M. Stahl, Essential Psychopharmacology, Depression and Bipolar Disorders, 151, University Press; 2 edition (June 15, 2000); ISBN: 0521646154*) .* Implications of not attaining remission for Mental Disorders are increased relapse rates, continuing functional impairment and increased suicide rate (S. M. Stahl, Essential Psychopharmacology, Depression and Bipolar Disorders, 152 , University Press; 2 edition (June 15, 2000); ISBN: 0521646154). Clinical causes of not attaining remission by the Current Psychopharmacological Compounds are inadequate early treatment, underlying emotion dysregulation (affecting instability - hypersensitivity - hyperaesthesia - dissociative phenomena, etc.) and competitive antagonism. There is thus a growing need for a more efficient therapy and more efficient, selective and efficacious medicaments for treating mental disorders.

### Summary of the invention

The present invention describes the use of compounds and pharmaceutical compositions having D4 and 5-HT2A antagonistic, partial agonistic or inverse agonistic activity for the treatment of the underlying emotion dysregulation of mental disorders (e.g. affecting instability - hypersensitivity - hyperaesthesia - dissociative phenomena - etc.) and to methods entailing administering to a patient diagnosed as having a mental disorder a pharmaceutical composition containing (i) compounds having specific high selective D4 and 5-HT2A antagonistic, partial agonistic or inverse agonistic activity and (ii) a known medicinal compound and/or compositions of compounds. The combined D4 and 5-HT2A antagonistic, partial agonistic or inverse agonistic effects may reside within the same chemical or biological compound.

Taken into account the above mentioned (i) rare clinical or real effectiveness of psycho tropics, (ii) the governance of the features and dysfunctions responsible - in a variable coincidentally - for the clinical state of the mental disorders by D4 dopamine receptor (D4DR) and 2A serotonin receptor (5-HT2A) antagonism and (iii) the fact that 5-HT2A antagonism gives a des-inhibiting of the inhibitory effect of the 5-HT2A receptor on (a) the 5-HT1A receptor stimulation by serotonin and on (b) the dopamine release in the mesocortical systems, the present invention relates to the use of a compound for the preparation of a medicament for treating a disease or disorder with an underlying emotion dysregulation, characterised in that said compound has (i) a selective affinity for the Dopamine-4 (D4) receptor with a pKi value equal to or higher than 8 towards the D4 receptor and less than 8 towards other Dopamine receptors, and (ii) a selective affinity for the 5-HT2A receptor with a pKi value equal to or higher than 8 towards the 5-HT2A receptor and less than 8 towards other 5-HT receptors and wherein said compound is administered to a patient in a dose ranging between 5 and 15 mg of the active ingredient. According to the present invention said compound is pipamperon.

According to a first embodiment, the present invention relates to the use of pipamperone or a pharmaceutically acceptable salt thereof for the preparation of a medicament for treating a mood disorder or anxiety disorder, wherein said pipamperone or a pharmaceutically acceptable salt is to be administered to a patient in a daily dose ranging between 5 and 15 mg of the active ingredient, and wherein a second compound is to be administered simultaneously with, separate from or sequential to said pipamperone to augment the therapeutic effect of said second compound, said second compound is selected from the group consisting of: selective serotonin, nor-adrenaline and dopamine re-uptake inhibitor (SNDRI), selective serotonin and nor-adrenaline re-uptake inhibitor (SNRI) and selective serotonin re-uptake inhibitor (SSRI).

More particularly, wherein said pipamperone is to be administered daily at least one day before administering said second compound.

More preferably the present invention relates to the use of pipamperone or a pharmaceutically acceptable salt thereof as defined above, wherein said second compound is a selective serotonin, noradrenaline and dopamine re-uptake inhibitor (SNDRI) compound.

More preferably said selective serotonin, nor-adrenalins and dopamine re-uptake inhibitor (SNDRI) compound is selected from the group consisting of NS 2330, McN 5652, DOV 216,303 and DOV 21,947, or a pharmaceutically acceptable salt thereof.

According to a further embodiment, the present invention relates to a pharmaceutical composition comprising (a) pipamperone, and a selective serotonin, nor-adrenaline and dopamine re-uptake inhibitor (SNDRI) compound, as a combined preparation for simultaneous, separate or sequential use for treating the underlying emotion dysregulation of mental disease or disorder which is chosen from the group consisting of mood disorders and anxiety disorders, wherein said pipamperone is to be administered to a patient in a daily dose of between 5 and 15 mg of the active ingredient.

Preferably, said selective serotonin, nor-adrenaline and dopamine re-uptake inhibitor (SNDRI) compound is selected from the group consisting of NS 2330, McN 5652, DOV 216,303 and DOV 21,947, or a pharmaceutically acceptable salt thereof

Preferably, the use of pipamperone or a pharmaceutically acceptable salt thereof is as defined above, wherein said second compound is a selective serotonin, and nor-adrenaline re-uptake inhibitor (SNRI) compound.

More preferably, the invention relates to the use of pipamperone or a pharmaceutically acceptable salt thereof as defined above, wherein said selective serotonin and nor-adrenatine re-uptake inhibitor (SNRI) compound is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof.

For instance, the invention relates to the use of pipamperone or a pharmaceutically acceptable salt thereof as defined above, wherein said venlafaxine, or a pharmaceutically acceptable salt thereof, is to be administered in a daily dose ranging between 75 and 300 mg of the active ingredient.

Also, the invention relates to the use of pipamperone or a pharmaceutically acceptable salt thereof as defined above, wherein said tomoxetine, or a pharmaceutically acceptable salt thereof, is to be administered in a daily dose ranging between 0.475 and 3.8 mg/kg of the active ingredient

Also the invention relates to the use of pipamperone or a pharmaceutically acceptable salt thereof as defined above, wherein said milnacipran, or a pharmaceutically acceptable salt thereof, is to be administered in a daily dose ranging between 50 and 200 mg of the active ingredient.

Further, the invention relates to the use of pipamperone or a pharmaceutically acceptable salt thereof as defined above, wherein said duloxetine, or a pharmaceutically acceptable salt thereof, is to be administered in a daily dose ranging between 40 and 60 mg of the active ingredient.

According to a further embodiment, the present invention relates to a pharmaceutical composition comprising (a) pipamperone and (b) a selective serotonin and nor-adrenaline re-uptake inhibitor (SNRI) compound as a combined preparation for simultaneous, separate or sequential use for treating the underlying emotion dysregulation of mental disease or disorder which is chosen from the group to be administered to a patient in a daily consisting of mood disorders and anxiety disorders, wherein said pipamperone is dose of between 5 and 15 mg of the active ingredient.

Preferably, the said selective serotonin and nor-adrenaline re-uptake inhibitor (SNRI) compound is selected from the group consisting of venlafaxine, tomoxetine, tandamine, talsupram, talopram, nefazodond, milnacipran, LY 113.821, duloxetine, desvenlafaxine and amoxapine, or a pharmaceutically acceptable salt thereof.

Also, the invention relates to the pharmaceutical composition as defined above, wherein said venlafaxine, or a pharmaceutically acceptable salt thereof, provided in a unitary dose of between 75 and 300 mg of the active ingredient.

Also, the invention relates to the pharmaceutical composition as defined above, wherein said tomoxetine, or a pharmaceutically acceptable salt thereof, is provided in a unitary dose of between 0.475 and 3.8 mg/kg of the active ingredient.

Also, the invention relates to the pharmaceutical composition as defined above, wherein said milnacipran, or a pharmaceutically acceptable salt thereof, is provided in a unitary dose of between 50 and 200 mg of the active ingredient.

Further, the invention relates to the pharmaceutical composition as defined above, wherein said duloxetine, or a pharmaceutically acceptable salt thereof, is provided in a unitary dose of between 40 and 60 mg of the active ingredient.

The invention also relates to the use of pipamperone or a pharmaceutical acceptable salt thereof as defined above, wherein said second compound is a selective serotonin reuptake inhibitor (SSRI) compound.

Preferably the invention relates to the use of pipamperone or a pharmaceutically acceptable salt thereof as defined above, wherein said selective serotonin re-uptake inhibitor (SSRI) compound is selected from the group consisting of YM 992, VPI-013 (OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, cericlamine and ademethionine preferably s-adenosylmethionine, or a pharmaceutically acceptable salt thereof.

Preferably, the invention relates to the use of pipamperone or a pharmaceutically acceptable salt thereof as defined above, wherein said selective serotonin re-uptake inhibitor (SSRI) compound is fluvoxamine (controlled release formulation), or a pharmaceutically acceptable salt thereof, is to be administered in a daily dose ranging between 100 and 300 mg of the active ingredient.

Preferably, the invention relates to the use of pipamperone or a pharmaceutically acceptable salt thereof as defined above, wherein said selective serotonin re-uptake inhibitor (SSRI) compound is escitalopram, and is to be administered in a daily dose ranging between 10 and 20 mg of the active ingredient.

Preferably, the invention relates to the use of pipamperone or a pharmaceutically acceptable salt thereof as defined above, wherein said selective serotonin re-uptake inhibitor (SSRI) compound is citalopram, and is to be administered in a daily dose ranging between 10 and 40 mg of the active ingredient.

Further, the invention relates to a pharmaceutical composition comprising (a) pipamperone and (b) a selective serotonin re-uptake inhibitor (SSRI) compound as a combined preparation for simultaneous, separate or sequential use for treating the underlying emotion dysregulation of mental disease or disorder which is chosen from the group consisting of mood disorders and anxiety disorders, wherein, said pipamperone is to be administered to a patient in a daily dose of between 5 and 15 mg of the active ingredient.

Further the invention relates to the pharmaceutical composition as defined above, wherein said selective serotonin re-uptake inhibitor (SSRI) compound is selected from the group consisting of YM 992, VPI-013 (also known as OPC-14523), sertraline, paroxetine, LY 214.281, LU AA 21-004, Lu 35-138, litoxetine, ifoxetine, fluvoxamine (controller release formulation), fluvoxamine, fluoxetine, femoxetine, escitalopram, EMD 68843, cyanodothepine, citalopram, venlafaxine, milnacipran, duloxetine, cericlamine and ademethionine (preferably s-adenosylmethionine), or a pharmaceutically acceptable salt thereof.

Further the invention relates to the pharmaceutical composition as defined above, wherein said fluvoxamine (controlled release formulation), or a pharmaceutically acceptable salt thereof, is provided in a unitary dose of between 100 and 300 mg of the active ingredient.

Further, the invention relates to the pharmaceutical composition as defined above, wherein said escitalopram, or a pharmaceutically acceptable salt thereof, is provided in a unitary dose of between 10 and 20 mg of the active ingredient.

Further, the invention relates to the pharmaceutical composition as defined above, wherein said citalopram, or a pharmaceutically acceptable salt thereof, is provided in a unitary dose of between 10 and 40 mg of the active ingredient.

According to a further embodiment the invention relates to the use of a first compound as defined above for the preparation of a medicament for treating a mental disease or disorder with an underlying emotion dysregulation whereby a second compound is administered simultaneously with, separate from or sequential to said first compound to augment the therapeutic effect of said second compound on said disease, or to provide a faster onset of the therapeutic effect of said second compound on said disease.

The mental diseases or disorders characterized by an underlying emotion dysregulation can be grouped into subclasses as follows: (i) non-cognitive mental disorders comprising mood disorders, anxiety disorders, psychotic disorders, eating disorders, premenstrual syndrome, somatoform disorders (excluding pain disorders), factitious disorders, dissociative disorders, sexual and gender identity disorders, sleep disorders, adjustment disorders, impulse control disorders, pervasive development disorders, attention-deficit disorders, disruptive behaviour disorders, substance-related disorders, personality disorders, psychological factors affecting medical conditions, malingering, antisocial behaviour, bereavement, occupational problems, identity problem, phase of life problem, academic problem and problems related to abuse or neglect; (ii) cognitive diseases comprising delirium, Alzheimer Disease, substance-related persisting dementia, vascular dementia, dementia due to HIV disease, dementia due to head trauma, dementia due to Parkinson Disease, dementia due to Huntington Disease, dementia due to Pick Disease, dementia due to Creutzfeldt-Jacob Disease, amnestic disorders due to a general medical condition, substance-induced persisting amnestic disorder, mild cognitive impairment disorder, other cognitive disorders; (iii) pain disorders; and (iv) Parkinson Disease.

In a preferred embodiment, the first compound is administered daily at least one day before administering said second compound.

Preferably, said second compound is characterized by the physiological property of influencing positively the activity of the Central Nervous System.

### Detailed description of the invention

The present inventors surprisingly found that compounds which have a high selective affinity towards the 5-HT2A receptor and which, at the same time have a high selective affinity towards the dopamine-4 (D4) receptor show an improved effect in treating underlying emotion dysregulation of mental disorders.

The compounds according to the invention may be chemical or biological in nature, or may be chemically synthesised. Preferably, the compounds of the invention are provided as a pharmaceutically acceptable salt.

One example of such a compound which has both a selective affinity for the 5-HT2A receptor with a pKi value equal to or higher than 8 towards the 5-HT2A receptor and less than 8 towards other 5HT receptors, and a selective affinity for the D4 receptor with a pKi value equal to or higher than 8 towards the D4 receptor and less than 8 towards other dopamine receptors is pipamperon. Pipamperon is the conventional name given for the compound of the formula 1'-[3-(p-Fluorobenzoyl)propyl]-[1,4'-bipiperidine]-4'-carboxamide. Pipamperon is also the active ingredient of for instance the commercially available Dipiperon (Janssen, Cilag B.V).

Further, the present inventors surprisingly found that the dosage of active ingredient for pipamperon in treatment (in combination therapy as described in more detail further) could be very low compared to conventionally used dosages. Preferred dosages which, according to the invention, have been shown to be effective for treating these mental disorders, range between 5 and 15 mg per day or between 5 and 10 mg per day. More preferably, dosages of 5, 6, 7, 8, 9, 10, 11, 12, 13, 14 or 15 mg per day are used in treatment of the diseases of the invention. In conventional pipamperon treatment, the active ingredient is available in tablets of 40 mg per tablet or in solutions of 2 mg per drop. Conventional usage of high doses ranging from 40 to 360 mg is prescribed. For instance, for children up to the age of 14, doses corresponding with 2 to 6 mg per kg body weight are conventionally prescribed. The high selective affinity of pipamperon towards the 5-HT2A receptor and the D4 receptor is reflected in the low dosage which is needed for the treatment of the mental diseases listed below and also contributes to the efficacy of the treatment.

Mental disorders such as depression are commonly treated with serotonin re-uptake inhibitors. Unfortunately, however, these compounds can give rise to side effects in use. Moreover, a substantial problem in most treatment of mental disorders is the non-response to selective serotonin re-uptake inhibitors (SSRIs). Also the onset of the therapeutic effect can be delayed undesirable.

A problem to be solved by the present invention is thus the provision of a more efficient therapy and efficient, highly selective and efficacious medicaments for treating mental disorders.

The inventors found that, for instance, the non-response to selective serotonin re-uptake inhibitors (SSRIs) in depression may be declared by (partial) inhibition of the 5-HT1A stimulation via 5-HT2A stimulation. Des-inhibition thereof via 5-HT2A antagonism seems to be an answer to this problem.

The present inventors found that a simultaneous or foregoing treatment with a compound having a high selective 5-HT2A antagonist, inverse agonist or partial agonist activity, could lead to a greater response towards, for instance, SSRIs. However, not all compounds exhibiting 5-HT2A antagonism are useful: competition between 5-HT2A stimulation via serotonin and 5-HT2A antagonism via the compound could be responsible for the lack of more efficacy of compounds which have both a selective serotonin re-uptake inhibitory and 5-HT2A antagonist profile, such as trazodone and nefazodone.

The present inventors further surprisingly found that a simultaneous or foregoing treatment with a compound having a high selective D4 antagonist, inverse agonist or partial agonist activity in combination with a compound having a high selective 5-HT2A antagonist, inverse agonist or partial agonist activity could lead to a greater response towards, for instance, SSRIs.

In this invention, the term "antagonist" refers to an interaction between chemicals in which one partially or completely inhibits the effect of the other, in particular agents having high affinity for a given receptor, but which do not activate this receptor.

In this invention, the term "inverse agonist" refers to a ligand which produces an effect opposite to that of the agonist by occupying the same receptor.

In this invention, the term "agonist" relates to an agent which both binds to a receptor and has an intrinsic effect.

In this invention, the term "partial agonist" relates to an agent with lower intrinsic activity than a full agonist, and which produces a lower maximum effect.

The present inventors found that a compound which binds to the 5-HT2A receptor with a pKi of at least 8 but for which the binding affinity, i.e. pKi, towards other 5HT receptors is less than 8 in combination with a high selective affinity for the D4 receptor, i.e. which bind to the D4 receptor with a pKi of at least 8 but for which the binding affinity, i.e. pKi, towards other dopamine receptors is less than 8 also show such an improved effect in treatment. These effects, i.e. D4 antagonism, inverse agonism or partial agonism and 5-HT2A antagonism, inverse agonism or partial agonism, may reside in the same compound.

The term "other 5HT receptors" as used herein relate to for instance 5-HT1 receptors (e.g. 5-HT1A, 5-HT1B, 5-HT1D, 5-HT1E, 5-HT1 F), 5-HT2B, 5-HT2C, 5-HT6 (rat) and 5-HT7 (rat).

By the expression "selective affinity for the 5-HT2A receptor" is meant that the receptor has a higher affinity for the 5-HT2A receptor than for other 5-HT receptors.

The expression "selective affinity for the D4 receptor" means that the receptor has a higher affinity for the dopamine D4 receptor than for other dopamine receptors.

The term "other dopamine receptors" are, for instance, D1, D2 and D3 dopamine receptors.

pKi values of test compounds for dopamine receptors as well as 5-HT2A receptors can be measured using commonly known assays.

Compounds which have a selective affinity for the D4 receptor preferably have a pKi value equal to or higher than 8 towards the D4 receptor and less than 8 towards other dopamine receptors.

Preferably, the compounds of the invention which have a selective affinity for the 5-HT2A receptor (or the D4 receptor), are compounds which have a pKi value equal to or higher than 8 towards the 5-HT2A receptor and the D4 receptor, and less than 8 towards other 5-HT receptors or dopamine receptors, respectively, as can be measured, for instance by methods known in the art. For instance, the "NIMH Psychoactive Drug Screening Program (PDSP)" Kᵢ database (http://kidb.cwru.edu/nimh/5htp.php), is a unique resource in the public domain which provides information on the abilities of drugs to interact with an expanding number of molecular targets. The PDSP K_{¡} database serves as a data warehouse for published and internally-derived pKi, or affinity, values for a large number of drugs and drug candidates at an expanding number of G-protein coupled receptors, ion channels, transporters and enzymes. The PDSP internet site also provides for commonly used protocols and assays for measuring pKi values of 5-HT and dopamine receptors.

A preferred example of a compound which has both a selective affinity for the 5-HT2A receptor with a pKi value equal to or higher than 8 towards the 5-HT2A receptor and less than 8 towards other 5-HT receptors, and a selective affinity for the D4 receptor with a pKi value equal to or higher than 8 towards the D4 receptor and less than 8 towards other Dopamine receptors and which is therefore useful in a combination therapy is pipamperon.

Table 1 illustrates the selective affinity of for instance pipamperon for the 5-HT2A and for the D4 receptor. In addition, Table 1 also illustrates the low or absence of affinity of pipamperon for other receptors such as the adrenergic receptors Alpha 1A, Alpha 2A, Alpha 2B, Alpha 2C, Beta 1, Beta 2, and the histamine receptor H1. As such, treating patients with pipamperon will provide for less side effects which otherwise result from simultaneous stimulation of other receptors. Therefore, and according to preferred embodiments, useful compounds according to the invention not only have a selective 5-HT2A and/or D4 affinity but also a low affinity for other receptors such as the adrenergic and histamine receptors.

The low dosage which can be used in pipamperon treatment, as already described earlier, contributes to the high selective affinity of the compound towards the 5-HT2A receptor and the D4 receptor and therefore also to the efficacy of the treatment.

The mental diseases or disorders characterized by an underlying emotion dysregulation can be grouped into subclasses as follows: (i) the non-cognitive mental disorders comprising mood disorders, anxiety disorders, psychotic disorders, eating disorders, premenstrual syndrome, somatoform disorders (excluding pain disorders), factitious disorders, dissociative disorders, sexual and gender identity disorders, sleep disorders, adjustment disorders, impulse control disorders, pervasive development disorders, attention-deficit disorders, disruptive behaviour disorders, substance-related disorders, personality disorders, psychological factors affecting medical conditions, malingering, antisocial behaviour, bereavement, occupational problems, identity problem, phase of life problem, academic problem and problems related to abuse or neglect; (ii) cognitive diseases comprising delirium, Alzheimer Disease, substance-related persisting dementia, vascular dementia, dementia due to HIV disease, dementia due to head trauma, dementia due to Parkinson Disease, dementia due to Huntington Disease, dementia due to Pick Disease, dementia due to Creutzfedt-Jacob Disease, amnestic disorders due to a general medical condition, substance-induced persisting amnestic disorder, mild cognitive impairment disorder, other cognitive disorders; (iii) the pain disorders; and (iv) Parkinson Disease. In Table 5, this classification has been used for summarizing the diseases and disorders relative to known psychotropics. In Table 6, an overview of pharmacological grouping is provided, indicating the pharmalogical profile numbering, the pharmalogical profile, the main disease or disorder indication(s), the name of the compound, the dose range, and the company producing or selling said compound.

These diseases and their diagnosis are very clearly defined in the "Diagnostic and Statistical Manual of Mental Disorders (DSM-IV)" published by the American Psychiatric Association. This manual sets forth diagnostic criteria, descriptions and other information to guide the classification and diagnosis of mental disorders and is commonly used in the field of neuropsychiatry. It is for instance available on the internet under: http://www.behavenet.com/ capsules/disorders/ dsm4tr.htm.

The expression "non-cognitive diseases or disorders" used in some of the embodiments of the invention comprises the following group of diseases or disorders: mood disorders, anxiety disorders, psychotic disorders, eating disorders, premenstrual syndrome, somatoform disorders (excluding pain disorders), factitious disorders, dissociative disorders, sexual and gender identity disorders, sleep disorders, adjustment disorders, impulse control disorders, pervasive development disorders, attention-deficit disorders, disruptive behaviour disorders, substance-related disorders, personality disorders, psychological factors affecting medical conditions, malingering, antisocial behaviour, bereavement, occupational problems, identity problem, phase of life problem, academic problem and problems related to abuse or neglect.

The expression "(related) cognitive diseases or disorders" according to the invention comprises, the following group of diseases or disorders: delirium (F05), dementia (such as Alzheimer Disease (F00), vascular dementia (F01), dementia due to other general medical conditions (HIV disease (F02.4), head trauma (F06.8), Parkinson Disease (F02.3), Huntington Disease (F02.2), Pick Disease (F02.0), Creutzfeldt-Jacob Disease (F02.1) and other (F02.8)), substance-induced persisting dementia (F1x.6)), amnestic disorders due to a general medical condition (F06.8) or a substance-induced persisting amnestic disorder (F1x.6), mild cognitive impairment disorder (F06.7) and other cognitive disorders (F04). The above list of diseases is provided by way of example and is not intended to limit the invention.

For instance, Alzheimer Disease is a degenerative brain disease of unknown cause that is the most common form of dementia. Alzheimer Disease usually starts in late middle age or in old age as a memory loss for recent events spreading to memories for more distant events and progresses over the course of five to ten years to a profound intellectual decline characterized by dementia and personal helplessness. The disease is marked histologically by the degeneration of brain neurons especially in the cerebral cortex and by the presence of neurofibrillary tangles and plaques containing beta-amyloid. Because dopamine receptor D4 (DRD4) antagonism can inhibit the behavioral disturbances - merely aggression and confusion - caused by the degeneration of dopamine D2 receptors (Esiri, M.M., The basis for behavioural disturbances in dementia, J. Neurol. Neurosurg. Psychiatry, 1996; 61(2):127-130.2) accompanied with Alzheimer disease and 5-HT2A antagonism has an important boosting effect towards the effect of cholinesterase inhibitors such as used in the treatment by facilitating the affected dopamine release in the mesocortical dopamine pathways, a high selective D4/5-HT2A-antagonist would be a more preferable compound to combine with a cholinesterase inhibitor since this avoids the counteracting effect of the in the art used SDAs on the cognitive functioning by its dopamine receptor D2-antagonism.

These diseases and their diagnoses are very clearly defined in the *"International Statistical Classification of Diseases and Related Health Problems, 1989 Revision, Geneva, World Health Organization, 1992 (ICD-10).* This manual sets forth diagnostic criteria, descriptions and other information to guide the classification and diagnosis of neurodegenerative disorders and is commonly used in the field of neurology. According to the ICD-10 classification, the cognitive disorders are classified under several classes of disorders, i.e. dispersed under categories F00 to F19 (see above: respective classification between parentheses). Following the DSM classification, however, they are grouped in one class of diseases or disorders.

The terms "treatment", "treating" as used herein include amelioration or elimination of a developed mental disease or condition once it has been established or alleviation of the characteristic symptoms of such disease or condition. As used herein these terms also encompass, depending on the condition of the patient, preventing the onset of a disease or condition or of symptoms associated with a disease or condition, including reducing the severity of a disease or condition or symptoms associated therewith prior to affliction with said disease or condition. Such prevention or reduction prior to affliction refers to administration of the compound or composition of the invention to a patient that is not at the time of administration afflicted with the disease or condition. "Preventing" also encompasses preventing the recurrence or relapse-prevention of a disease or condition or of symptoms associated therewith, for instance after a period of improvement. It should be clear that mental conditions may be responsible for physical complaints. In this respect, the term "treating" also includes prevention of a physical disease or condition or amelioration or elimination of the developed physical disease or condition once it has been established or alleviation of the characteristic symptoms of such conditions.

As used herein, the term "medicament" also encompasses the terms "drug", "therapeutic", "potion" or other terms which are used in the field of medicine to indicate a preparation with therapeutic or prophylactic effect.

The present inventors not only found that the selective 5-HT2A and D4 antagonists, inverse agonists or partial agonists have an effect in augmenting the therapeutic effect or in providing a faster onset of the therapeutic effect of a diversity of other pharmaceutical compounds, i.e. also named "second compounds" in the present invention, in the treatment of specific diseases or disorders.

Second compounds useful according to the invention are SSRI, SNRI and SNDRI compounds provided in Table 5. It should be clear that in the present invention, pipamperon is never to be seen as a "second compound".

According to the invention, it thus has been found that the compounds having a selective 5-HT2A and D4 antagonist, inverse agonist or partial agonist activity as described above are useful for augmenting the therapeutic effect of a second compound on a disease.

According to another embodiment of the invention, it has also been found that the compounds having a selective 5-HT2A and D4 antagonist, inverse agonist or partial agonism, activity as described above are useful for providing a faster onset of the therapeutic effect of a second compound on a disease.

From the above it should be clear that the selective 5-HT2A and D4 antagonist, inverse agonist or partial agonist is also named 'the first compound' in the embodiments of the invention.

According to the invention, when the 5-HT2A and D4 antagonist, inverse agonist or partial agonist activity reside in separate compounds, the term "composition" may be used. Compositions of the invention comprise a first element having (i) a selective affinity for the D4 receptor with a pKi value equal to or higher than 8 towards the D4 receptor and less than 8 towards other dopamine receptors, and a second element having (ii) a selective affinity for the 5-HT2A receptor with a pKi value equal to or higher than 8 towards the 5-HT2A receptor and less than 8 towards other 5-HT receptors.

The expression "the 5-HT2A and D4 antagonist, inverse agonist or partial agonist" is used herein to indicate a single compound having both activities or to indicate the composition comprising the activities in separate elements.

It should be clear that when, in the present invention, a composition of separate elements is used instead of a single compound, this composition of separate elements may be used in combination with another, i.e. a second, compound to augment the therapeutic effect of the other, i.e. the second, compound on the same or another disease.

When the 5-HT2A and D4 antagonist, inverse agonist or partial agonist or the composition comprising both elements and the second compound are administered simultaneously, the compounds or active ingredients may be present in a single pharmaceutical composition or formulation. Alternatively the compounds or active ingredients are administered in separate pharmaceutical compositions or formulations for simultaneous or separate use. The invention thus also relates to pharmaceutical compositions comprising pipamperon and a second compound of the invention and to the uses of these pharmaceutical compositions.

When the 5-HT2A and D4 antagonist, inverse agonist or partial agonist or the composition comprising both elements of the invention are administered prior to the second compound as defined, the 5-HT2A and D4 antagonist, inverse agonist or partial agonist or the composition comprising both elements is administered at least during 1 day prior to said second compound. Preferably, the 5-HT2A and D4 antagonist, inverse agonist or partial agonist (e.g. pipamperon) or the composition comprising both elements is administered for at least 1, 2, 3, 4, 5, 6, 7, 8, 9 or 10 days prior to the administration of the second compound. Preferably, the 5-HT2A and D4 antagonist, inverse agonist or partial agonist (e.g. pipamperon) or the composition comprising both elements is administered for at least 2, 3, 4 or 5 weeks prior to the administration of the second compound, or even for at least 1, 2, 3, 4, 5, 6, 7, 8, 9, 10, 11 or 12 months prior to the administration of the second compound.

According to a preferred embodiment of the invention, the above described compounds or the composition comprising both elements having a 5-HT2A and D4 antagonist, inverse agonist or partial agonist activity are useful for augmenting the therapeutic effect of citalopram or for providing a faster onset of the therapeutic effect of citalopram.

Citalopram or citalopram hydrobromide is a selective serotonin (5-hydroxytryptamine / 5-HT) re-uptake inhibitor (SSRI) and is the conventional name given for the compound of the formula (RS)-1-[3-(dimethylamino)propyl]-1-(p-flurophenyl)-5-phthalancarbonitrile hydro-bromide.

According to an embodiment, a daily dose of active ingredient of SSRI, preferably citalopram, ranges between 10 and 40 mg per day. Preferably, daily doses of active ingredient ranging between 20 and 30 mg per day are administered. More preferably, a daily dose of 10, 15, 20, 25, 30, 35 or 40 mg per day is administered.

According to another preferred embodiment of the invention, the above described compounds or the composition comprising both elements having a 5-HT2A and D4 antagonist, inverse agonist or partial agonist activity are useful for augmenting the therapeutic effect of citalopram or for providing a faster onset of the therapeutic effect of fluvoxamine.

Fluvoxamine or fluvoxamine maleate (luvox, fevarin) is a selective serotonin (5-HT) reuptake inhibitor (SSRI) belonging to a new chemical series, the 2-aminoethyl oxime ethers of aralkylketones. It is chemically unrelated to other SSRIs and clomipramine. It is chemically designated as 5-methoxy-4'-(trifluoromethyl) valerophenone (E)-O-(2-aminoethyl) oxime maleate (1:1).

According to an embodiment, a daily dose of active ingredient of fluvoxamine maleate ranges between 100 and 300 mg per day. Preferably, daily doses of active ingredient ranging between 150 and 200 mg per day are administered. More preferably, a daily dose of 100, 150, 200, 250 or 300 mg per day is administered.

Most of the second compounds herein described are known in the art and may be used in doses according to the supplier's or physician's prescription, or may be used according to specific embodiments described herein.

The present invention also encompasses the use of these second compounds, administered in the form of a pharmaceutically acceptable salt in admixture with a suitable pharmaceutically acceptable excipient.

To prepare the pharmaceutical compositions, comprising the compounds or the combination of the first and second compound described herein, an effective amount of the active ingredients, in acid or base addition salt form or base form, is combined in admixture with a pharmaceutically acceptable carrier, which can take a wide variety of forms depending on the form of preparation desired for administration. These pharmaceutical compositions are desirably in unitary dosage form suitable, for administration orally, nasal, rectally, percutaneously or by parenteral injection. For example, in preparing the compositions in oral dosage form, any of the usual pharmaceutical media may be employed, such as, for example, water, glycols, oils, alcohols in the case of oral liquid preparations such as suspensions, syrups, elixirs and solutions; or solid carriers such as starches, sugars, kaolin, lubricants, binders, disintegrating agents in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included.

The pharmaceutical compounds for treatment are intended for parenteral, topical, oral or local administration and generally comprise a pharmaceutically acceptable carrier and an amount of the active ingredient sufficient to reverse or prevent the bad effects of mental disorders. The carrier may be any of those conventionally used and is limited only by chemico-physical considerations, such as solubility and lack of reactivity with the compound, and by the route of administration.

Examples of pharmaceutically acceptable acid addition salts for use in the present inventive pharmaceutical composition include those derived from mineral acids, such as hydrochloric, hydrobromic, phosphoric, metaphosphoric, nitric and sulfuric acids, and organic acids, such as tartaric, acetic, citric, malic, lactic, fumaric, benzoic, glycolic, gluconic, succinic, p-toluenesulphonic acids, and arylsulphonic, for example.

The pharmaceutically acceptable excipients described herein, for example, vehicles, adjuvants, carriers or diluents, are well-known to those who are skilled in the art and are readily available to the public. It is preferred that the pharmaceutically acceptable carrier be one that is chemically inert to the active compounds and one that has no detrimental side effects or toxicity under the conditions of use.

The following formulations for oral, aerosol, parenteral, subcutaneous, intravenous, intramuscular, interperitoneal, rectal, and vaginal administration are merely exemplary and are in no way limiting. Overall, the requirements for effective pharmaceutical carriers for parenteral compositions are well known to those of ordinary skill in the art. See Pharmaceutics and Pharmacy Practice, J.B. Lippincott Company, Philadelphia, PA, Banker and Chalmers, eds., pages 238-250, (1982), and ASHP Handbook on Injectable Drugs, Toissel, 4th ed., pages 622-630 (1986). Topical formulations, including those that are useful for transdermal drug release, are well-known to those of skill in the art and are suitable in the context of the present invention for application to skin.

Formulations suitable for oral administration require extra considerations considering the nature of the compounds and the possible breakdown thereof if such compounds are administered orally without protecting them from the digestive secretions of the gastrointestinal tract. Such a formulation can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, containing, in addition to the active ingredient, such excipients as are known in the art.

The compounds of the present invention, alone or in combination with other suitable components, can be made into aerosol formulations to be administered via inhalation. For aerosol administration, the compounds are preferably supplied in finely divided form along with a surfactant and propellant. Typical percentages of compounds are 0.01 %-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the compounds, preferably 0.25-5%. The balance of the compounds is ordinarily propellant. A carrier can also be included as desired, e.g., lecithin for intranasal delivery. These aerosol formulations can be placed into acceptable pressurized propellants, such as dichlorodifluoromethane, propane, nitrogen. They also may be formulated as pharmaceuticals for non-pressured preparations, such as in a nebulizer or an atomizer. Such spray formulations may be used to spray mucosa.

It will be understood that, apart from daily doses, the compounds can be administered by other schedules. For instance, the present invention also contemplates depot injection, in which a long acting form of the active compound is injected into the body, such as the muscles. From there the active compound slowly enters the rest of the body, so one injection can last from 1 to 4 weeks or even multiple months. Other form of dosage administrations relate to "once-a-week" pills, in which the ingredient is slowly released over a period of a week, and slow-realese patches, e.g. a CDS (Continuous Delivery System), or Once-a-Day Transdermal Patches.

It should be clear that the compounds and compositions described herein are useful for treating any patient in need thereof. As used herein the term "patient" is not restricted to humans but also to other mammals, for instance, domestic animals which may also suffer from any form of a mental disease or disorder described herein.

The second compounds of the invention can be further grouped according to their pharmacological profile, which is summarized in Table 6.

The present invention is now described in more detail by the following embodiments. The compounds belonging to different pharmacological profiles can be further grouped according to their action on the same pathway or system as follows.

### 90: combination therapy with a selective SNDRI compound

The mental disorders which can be treated using compounds having a high selective affinity for the 5-HT2A and D4 receptor, for instance pipamperon, in a combination therapy with a selective serotonin, nor-adrenaline and dopamine re-uptake inhibitor (SNDRI) compound, are chosen from the group of diseases or disorders consisting of mood disorders, anxiety disorders.

The present invention thus relates to the use of pipamperon or a pharmaceutically acceptable salt thereof for the preparation of a medicament for treating the underlying emotion dysregulation of a non-cognitive mental disease or disorder selected from the group of diseases and disorders consisting of mood disorders, anxiety disorders, characterized in that pipamperon or said pharmaceutically acceptable salt thereof is administered simultaneously with, separate from or prior to the administration of a selective serotonin, nor-adrenaline and dopamine re-uptake inhibitor (SNDRI) compound to augment the therapeutic effect or to provide a faster onset of the therapeutic effect of said selective serotonin, nor-adrenaline and dopamine re-uptake inhibitor (SNDRI) compound, further characterized in that pipamperon is to be administered to a patient in a daily dose ranging between 5 and 15 mg of the active ingredient.

According to a preferred embodiment, the invention relates to the uses as described above, wherein said selective serotonin, nor-adrenaline and dopamine re-uptake inhibitor (SNDRI) compound is selected from the group consisting of NS 2330; McN 5652; DOV 216,303 and DOV 21,947; more preferably NS 2330 or DOV 216,303; or a pharmaceutically acceptable salt thereof.

The invention also relates to a pharmaceutical composition comprising (a) pipamperon, and (b) a selective serotonin, nor-adrenaline and dopamine re-uptake inhibitor (SNDRI) compound, preferably selected from the group consisting of NS 2330; McN 5652; DOV 216,303 and DOV 21,947, more preferably NS 2330 or DOV 216,303, or a pharmaceutically acceptable salt thereof, as a combined preparation for simultaneous, separate or sequential use for treating the underlying emotion dysregulation of mental disease or disorder which is chosen from the group consisting of mood disorders, anxiety disorders.

### 91: combination therapy with a selective SNRI compound

The mental disorders which can be treated using compounds having a high selective affinity for the 5-HT2A and D4 receptor, for instance pipamperon, in a combination therapy with a selective serotonin and nor-adrenaline re-uptake inhibitor (SNRI) compound, are chosen from the group of diseases or disorders consisting of mood disorders, anxiety disorders.

The present invention thus relates to the use of pipamperon or a pharmaceutically acceptable salt thereof for the preparation of a medicament for treating the underlying emotion dysregulation of a non-cognitive mental disease or disorder selected from the group of diseases and disorders consisting of mood disorders, anxiety disorders, characterized in that pipamperon or said pharmaceutically acceptable salt thereof is administered simultaneously with, separate from or prior to the administration of a selective serotonin and nor-adrenaline re-uptake inhibitor (SNRI) compound to augment the therapeutic effect or to provide a faster onset of the therapeutic effect of said selective serotonin and nor-adrenaline re-uptake inhibitor (SNRI) compound, further characterized in that pipamperon is to be administered to a patient in a daily dose ranging between 5 and 15 mg of the active ingredient.

According to a preferred embodiment, the invention relates to the uses as described above, wherein said selective serotonin and nor-adrenaline re-uptake inhibitor (SNRI) compound is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof. Even more preferably, said selective serotonin and nor-adrenaline re-uptake inhibitor (SNRI) compound is chosen from the group consisting of venlafaxine, tomoxetine, milnacipran, duloxetine and desvenlafaxine, or a pharmaceutically acceptable salt thereof. More preferably, said selective serotonin and nor-adrenaline re-uptake inhibitor (SNRI) compound is venlafaxine and is to be administered in a daily dose ranging between 75 and 300 mg of the active ingredient. More preferably, said selective serotonin and nor-adrenaline re-uptake inhibitor (SNRI) compound is tomoxetine and is to be administered in a daily dose ranging between 0.475 and 3.8 mg/kg of the active ingredient. More preferably, said selective serotonin and nor-adrenaline re-uptake inhibitor (SNRI) compound is milnacipran and is to be administered in a daily dose ranging between 50 and 200 mg of the active ingredient. More preferably, said selective serotonin and nor-adrenaline re-uptake inhibitor (SNRI) compound is duloxetine and is to be administered in a daily dose ranging between 40 and 60 mg of the active ingredient.

The invention also relates to a pharmaceutical composition comprising (a) pipamperon, and (b) a selective serotonin and nor-adrenaline re-uptake inhibitor (SNRI) compound, preferably selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof, as a combined preparation for simultaneous, separate or sequential use for treating the underlying emotion dysregulation of mental disease or disorder which is chosen from the group consisting of mood disorders, anxiety disorders,

The invention also relates to a pharmaceutical composition as described above wherein pipamperon is provided in a unitary dose of between 5 and 15 mg of the active ingredient and wherein said selective serotonin and nor-adrenaline re-uptake inhibitor (SNRI) compound is venlafaxine, preferably provided in a unitary dose of between between 75 and 300 mg of the active ingredient.

The invention also relates to a pharmaceutical composition as described above wherein pipamperon is provided in a unitary dose of between 5 and 15 mg of the active ingredient and wherein said selective serotonin and nor-adrenaline re-uptake inhibitor (SNRI) compound is tomoxetine, preferably provided in a unitary dose of between 0.475 and 3.8 mg/kg of the active ingredient.

The invention also relates to a pharmaceutical composition as described above wherein pipamperon is provided in a unitary dose of between 5 and 15 mg of the active ingredient and wherein said selective serotonin and nor-adrenaline re-uptake inhibitor (SNRI) compound is milnacipran, preferably provided in a unitary dose of between 50 and 200 mg of the active ingredient.

The invention also relates to a pharmaceutical composition as described above wherein pipamperon is provided in a unitary dose of between 5 and 15 mg of the active ingredient and wherein said selective serotonin and nor-adrenaline re-uptake inhibitor (SNRI) compound is duloxetine, preferably provided in a unitary dose of between 40 and 60 mg of the active ingredient.

### 92: combination therapy with a selective serotonin re-uptake inhibitor (SSRI) compound

The mental disorders which can be treated using compounds having a high selective affinity for the 5-HT2A and D4 receptor, for instance pipamperon, in a combination therapy with a selective serotonin re-uptake inhibitor (SSRI) compound, are chosen from the group of diseases or disorders consisting of mood disorders, anxiety disorders.

The present invention thus relates to the use of pipamperon or a pharmaceutically acceptable salt thereof for the preparation of a medicament for treating the underlying emotion dysregulation of a non-cognitive mental disease or disorder selected from the group of diseases and disorders consisting of mood disorders, anxiety disorders, characterized in that pipamperon or said pharmaceutically acceptable salt thereof is administered simultaneously with, separate from or prior to the administration of a selective serotonin re-uptake inhibitor (SSRI) compound to augment the therapeutic effect or to provide a faster onset of the therapeutic effect of said selective serotonin re-uptake inhibitor (SSRI) compound, further characterized in that pipamperon is to be administered to a patient in a daily dose ranging between 5 and 15 mg of the active ingredient.

According to a preferred embodiment, the invention relates to the uses as described above, wherein said selective serotonin re-uptake inhibitor (SSRI) compound is selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, cericlamine and ademethionine (preferably s-adenosylmethionine), or a pharmaceutically acceptable salt thereof. Even more preferably, said selective serotonin re-uptake inhibitor (SSRI) compound is chosen from the group consisting of litoxetine, fluvoxamine (controlled release formulation) and escitalopram, or a pharmaceutically acceptable salt thereof.

More preferably, said selective serotonin re-uptake inhibitor (SSRI) compound is fluvoxamine (controlled release formulation) and is to be administered in a daily dose ranging between 100 and 300 mg of the active ingredient. More preferably, said selective serotonin re-uptake inhibitor (SSRI) compound is escitalopram and is to be administered in a daily dose ranging between 10 and 20 mg of the active ingredient. More preferably, said selective serotonin re-uptake inhibitor (SSRI) compound is citalopram and is to be administered in a daily dose ranging between 10 and 40 mg of the active ingredient.

The invention also relates to a pharmaceutical composition comprising (a) pipamperon, and (b) a selective serotonin re-uptake inhibitor (SSRI) compound, preferably selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, cericlamine and ademethionine (preferably s-adenosylmethionine), or a pharmaceutically acceptable salt thereof, as a combined preparation for simultaneous, separate or sequential use for treating the underlying emotion dysregulation of mental disease or disorder which is chosen from the group consisting of mood disorders, anxiety disorders.

The invention also relates to a pharmaceutical composition as described above wherein pipamperon is provided in a unitary dose of between 5 and 15 mg of the active ingredient and wherein said selective serotonin re-uptake inhibitor (SSRI) compound is fluvoxamine (controlled release formulation), preferably provided in a unitary dose of between between 100 and 300 mg of the active ingredient.

The invention also relates to a pharmaceutical composition as described above wherein pipamperon is provided in a unitary dose of between 5 and 15 mg of the active ingredient and wherein said selective serotonin re-uptake inhibitor (SSRI) compound is escitalopram, preferably provided in a unitary dose of between 10 and 20 mg of the active ingredient.

The invention also relates to a pharmaceutical composition as described above wherein pipamperon is provided in a unitary dose of between 5 and 15 mg of the active ingredient and wherein said selective serotonin re-uptake inhibitor (SSRI) compound is citalopram, preferably provided in a unitary dose of between 10 and 40 mg of the active ingredient.

Citalopram or citalopram hydrobromide is a selective serotonin (5-hydroxytryptamine / 5-HT) re-uptake inhibitor (SSRI) and is the conventional name given for the compound of the formula (RS)-1-[3-(dimethylamino)propyl]-1-(p-flurophenyl)-5-phthalancarbonitrile-hydro-bromide. According to an embodiment, a daily doses of active ingredient of SSRI, preferably citalopram, ranges between 10 and 40 mg per day. Preferably, daily doses of active ingredient ranging between 20 and 30 mg per day are administered. More preferably, a daily dose of 10, 15, 20, 25, 30, 35 or 40 mg per day is administered.

Fluvoxamine or fluvoxamine maleate (luvox, fevarin) is a selective serotonin (5-HT) reuptake inhibitor (SSRI) belonging to a new chemical series, the 2-aminoethyl oxime ethers of aralkylketones. It is chemically unrelated to other SSRIs and clomipramine. It is chemically designated as 5-methoxy-4'-(trifluoromethyl) valerophenone (E)-O-(2-aminoethyl) oxime maleate (1:1).

According to an embodiment, a daily dose of active ingredient of fluvoxamine in a controlled release mode ranges between 100 and 300 mg per day. Preferably, daily doses of active ingredient ranging between 150 and 200 mg per day are administered in a controlled release mode. More preferably, a daily dose of 100, 150, 200, 250 or 300 mg per day is administered by controlled release.

The invention, now being generally described, will be more readily understood by reference to the following tables and examples.

### Short description of the Tables and Figures

**Table 1**: In Table 1, the pKi values of test compounds are given for each of the dopamine receptors, 5HT receptors, adrenergic receptors and the histamine1 receptor.
**Table 2**: Set-up of a clinical trial comprising for treatment groups.
**Table 3**: Overview of a placebo, active and period controlled clinical trial in a fore-going pipamperon - citalopram treatment in Major Depressive Disorder.
**Table 4**: POC process for major depressive disorder.
**Table 5**: Summary of diseases and disorders relative to known psycho-tropics.
**Table 6**: Overview of Pharmacological grouping, indicating pharmacological profile numbering (column 2), pharmacological profile (column 3), main indication(s) (column 4), name of the compound (column 4), the dose range (column 5), and the company producing or selling said compound (column 6). Compounds indicated by hatching are preferred.

**Figure 1**: Add-on treatment with pipamperon after treatment with citalopram .
**Figure 2**: HDRS-17 change from baseline: combo treatment pipamperon as add-on - citalopram vs SNRI (duloxetine) in Major Depression.
**Figure 3**: Remission rates (HDRS-17 <=7): combo treatment pipamperon as add-on - citalopram vs SNRI (venlafaxine) vs SSRIs vs placebo in Major Depression.
**Figure 4**: Fore-going treatment during 1-5 days with pipamperon followed with the combination treatment of pipamperon and citalopram.
**Figure 5**: HDRS-17 change from baseline: combo treatment pipamperon - citalopram with a fore-going treatment of 4 days with pipamperon vs SNRI (duloxetine) in Major Depression.
**Figure 6**: Remission rates (HDRS-17 <=7): combo pipamperon - citalopram with a fore-going treatment of 4 days with pipamperon vs SNRI (venlafaxine) in Major Depression.
**Figure 7**: Fore-going treatment during 6-8 days with pipamperon followed with the combination treatment of pipamperon and citalopram.
**Figure 8**: HDRS-17 change from baseline: combo treatment pipamperon - citalopram with a fore-going treatment of 7 days with pipamperon vs SNRI (duloxetine) in Major Depression.
**Figure 9**: Fore-going and add-on treatment with pipamperon in MDD.
**Figure 10**: HDRS-17 change from baseline: fore-going and add-on treatment with pipamperon and citalopram in comparison with the SNRI duloxetine in Major Depression.
**Figure 11**: Remission rates (HDRS-17 <=7): fore-going and add-on treatment with pipamperon and citalopram in comparison with the SNRI venlafaxine in Major Depression.
**Figure 12**: Y-BOCS total score: fore-going and add-on treatment with pipamperon and citalopram in comparison with the SSRI fluvoxamine in OCD.
**Figure 13**: Y-BOCS obsession score: fore-going and add-on treatment with pipamperon and citalopram in comparison with the SSRI fluvoxamine in OCD.
**Figure 14**: Y-BOCS compulsion score: fore-going and add-on treatment with pipamperon and citalopram in comparison with the SSRI fluvoxamine in OCD.
**Figure 15**: CGI-severity score: fore-going and add-on treatment with pipamperon and citalopram in comparison with the SSRI in panic disorder.

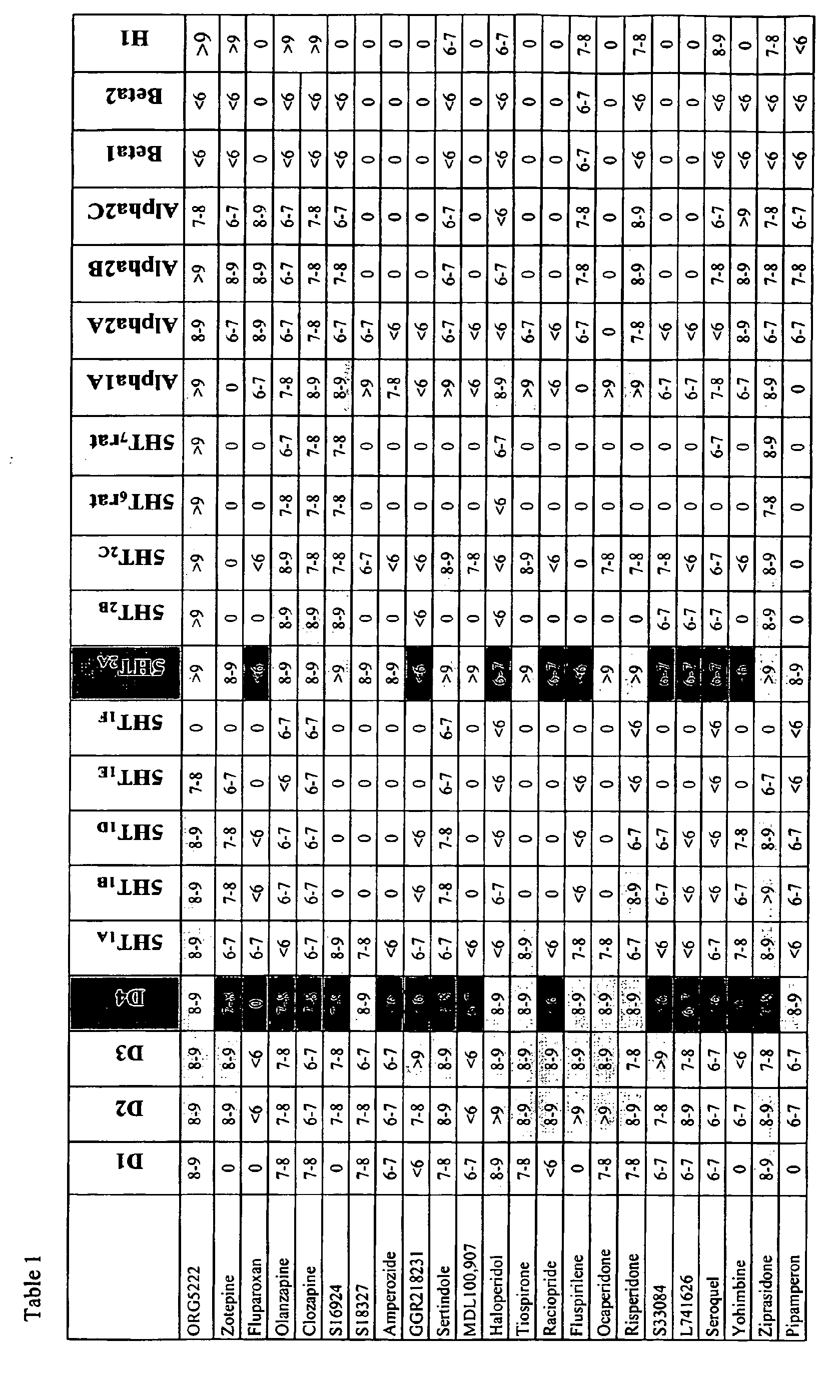

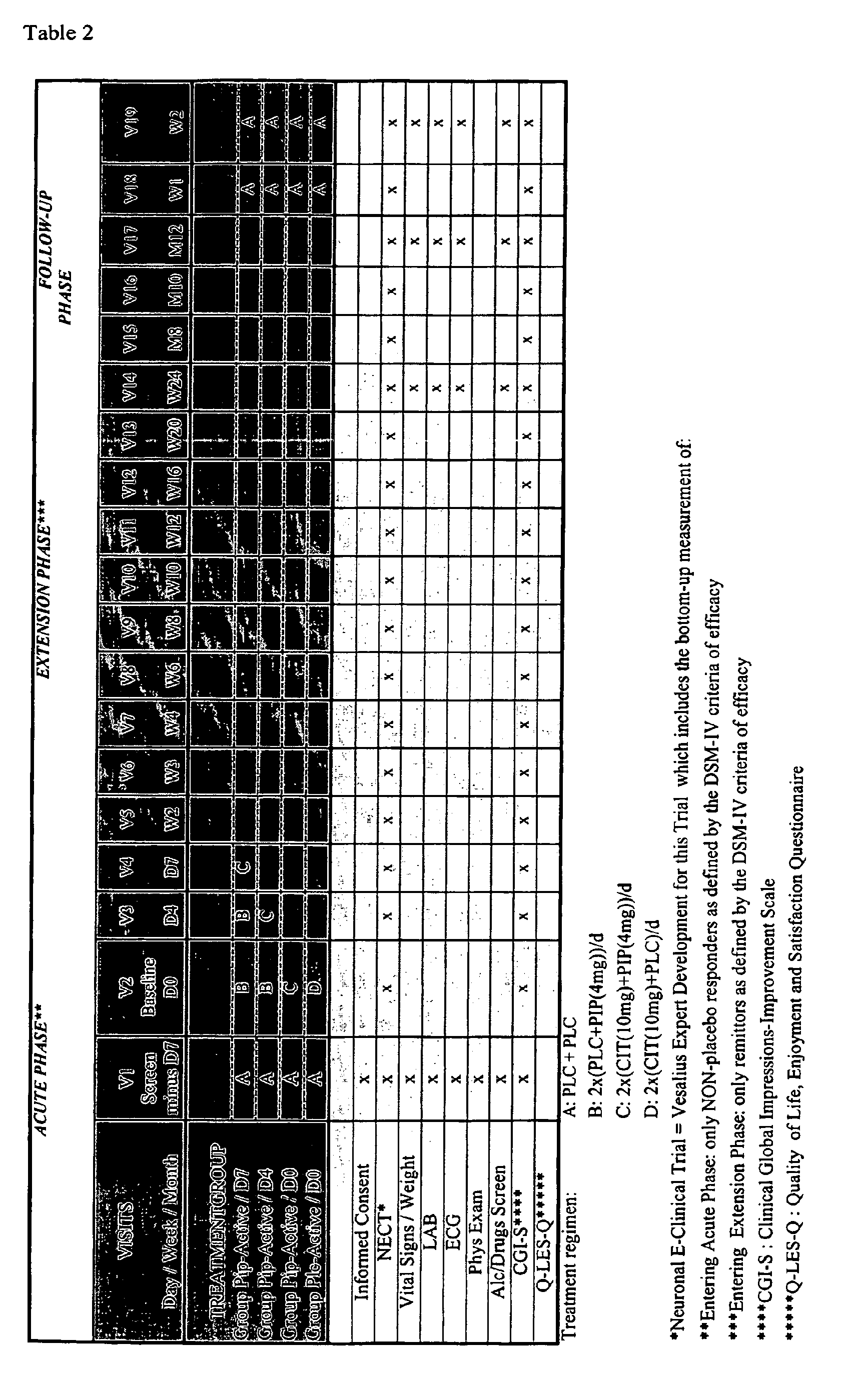

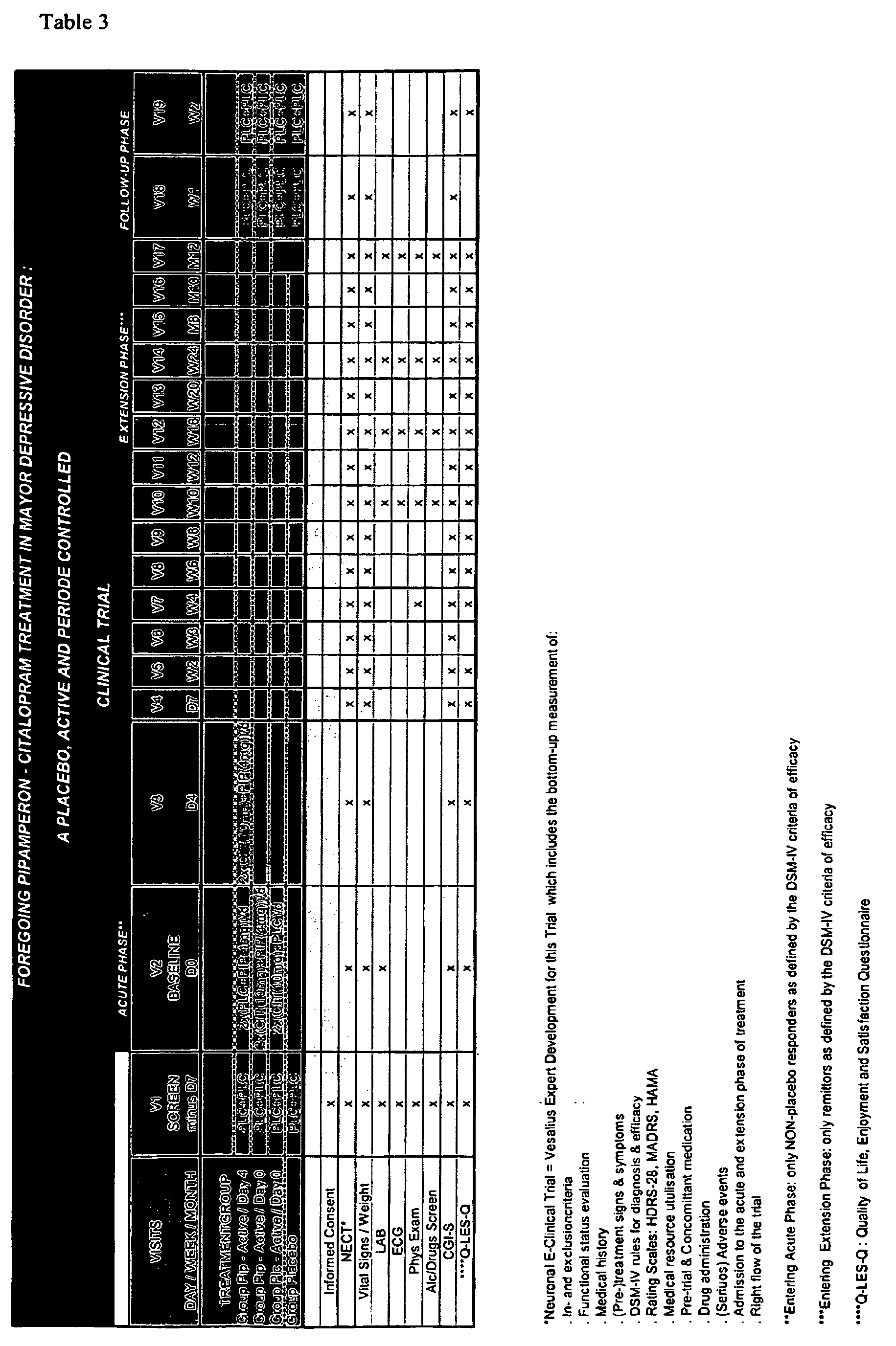

**Table 4**

| DAY | minus D7 | D0 | =>D4 |
|---|---|---|---|
| TREATMENTGROUP | | | |
| Placebo (PLC) | PLC+PLC | 2x(PLC+PLC) | 2x(PLC+PC) |
| PIP - Active / Day 4 | PLC+PLC | 2x(PLC+PIP (4mg))/d | 2x(CIT (10mg)+PIP (4mg))/d |
| PIP - Active / Day 0 | PLC+PLC | 2x(CIT (10mg)+PIP (4mg))/d | 2x(CIT (10mg)+PIP (4mg))/d |
| PLC - Active / Day 0 | PLC+PLC | 2x(CIT (10mg)+PLC)/d | 2x(CIT (10mg)+PLC)/d |

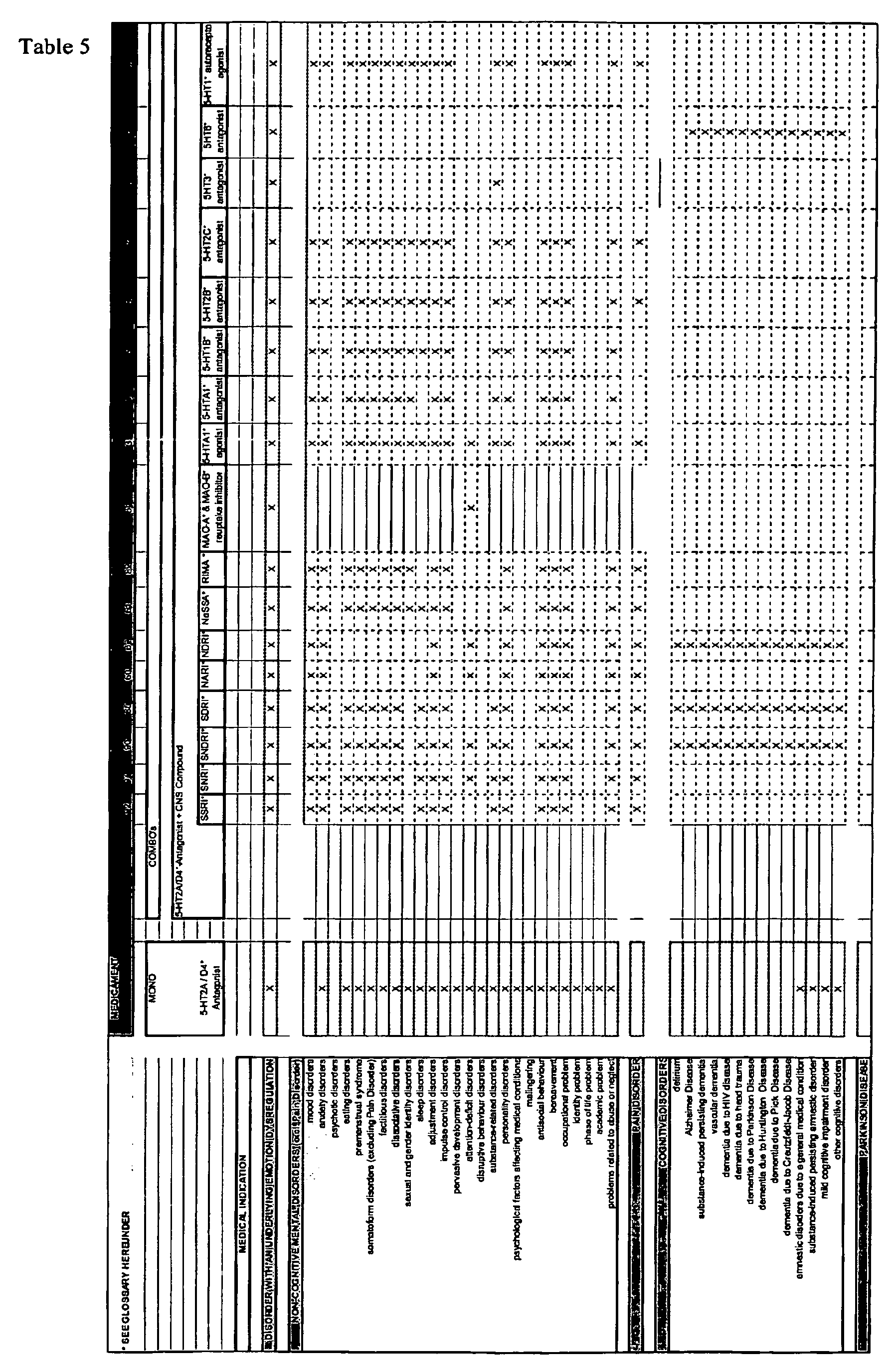

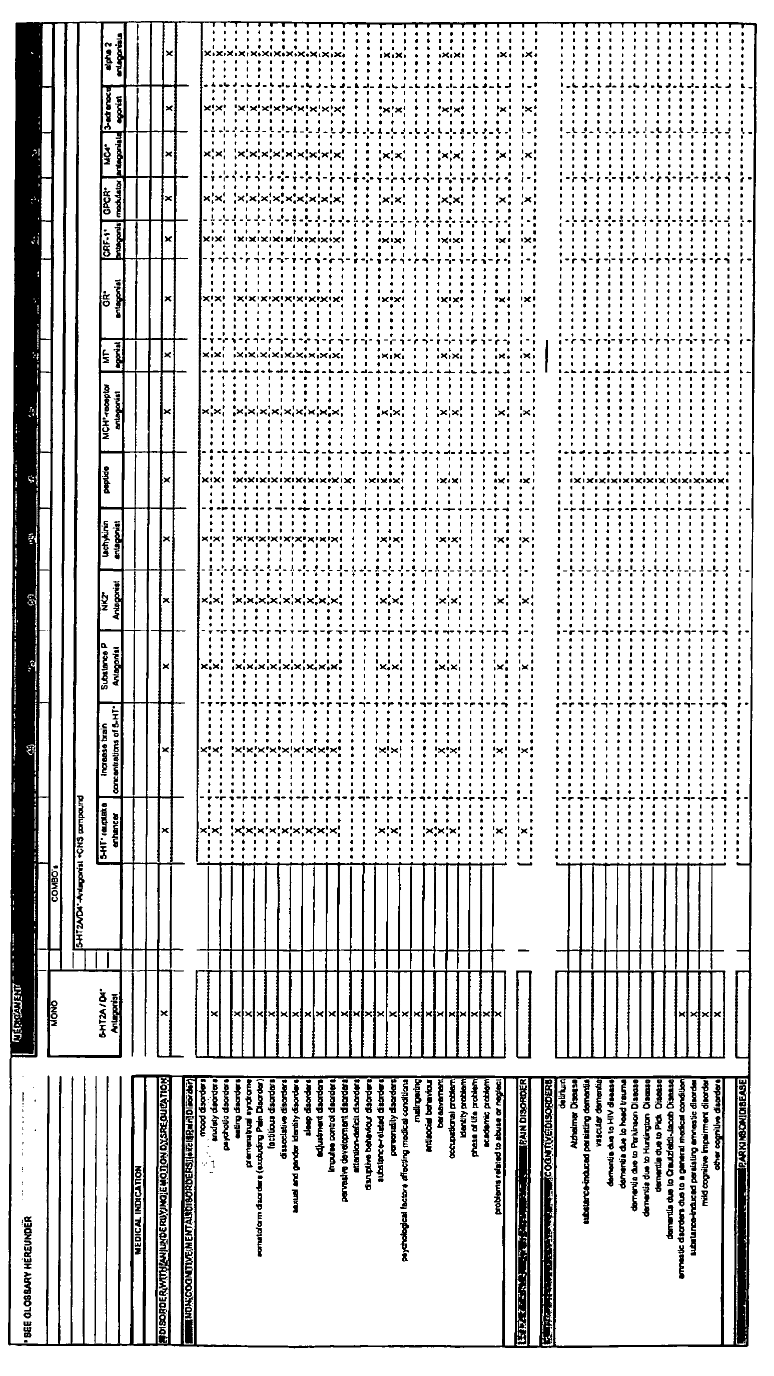

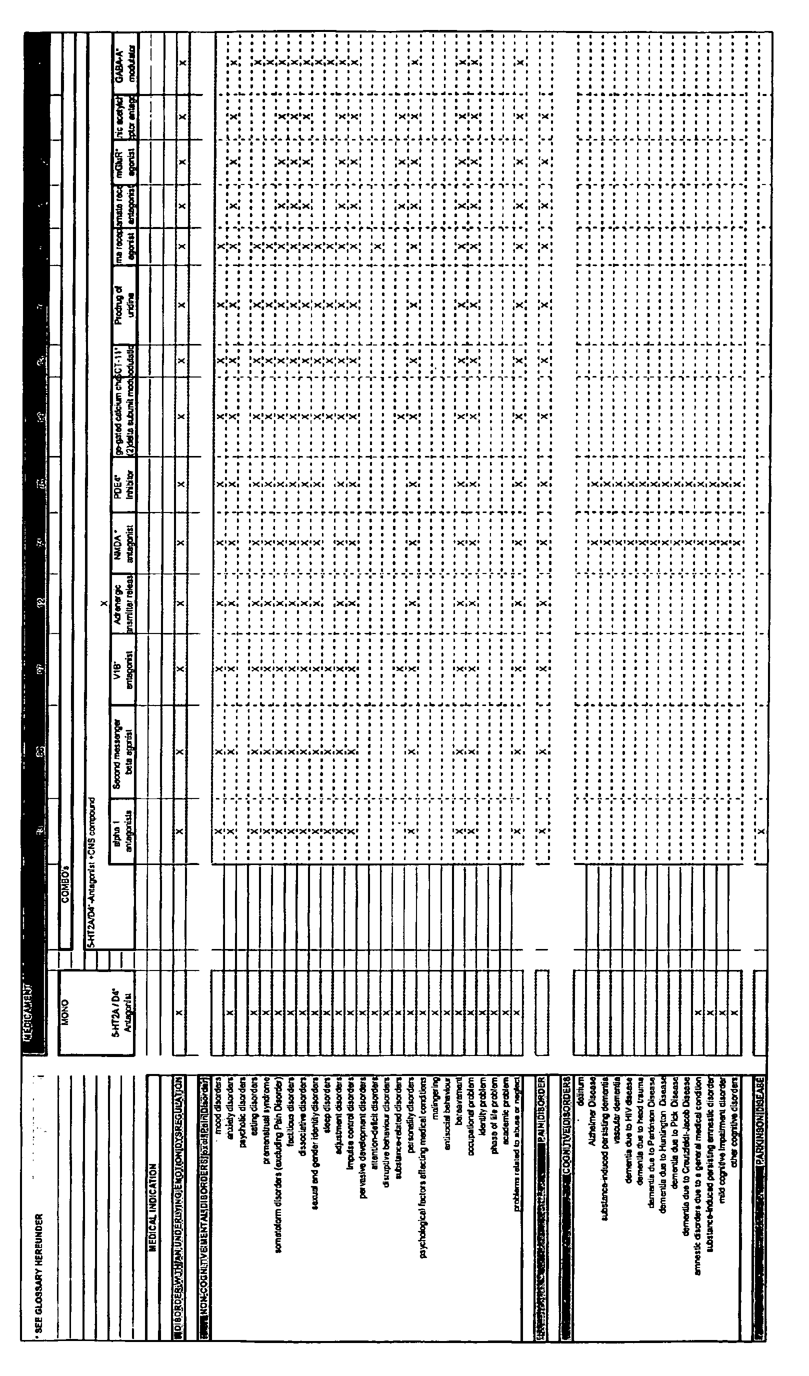

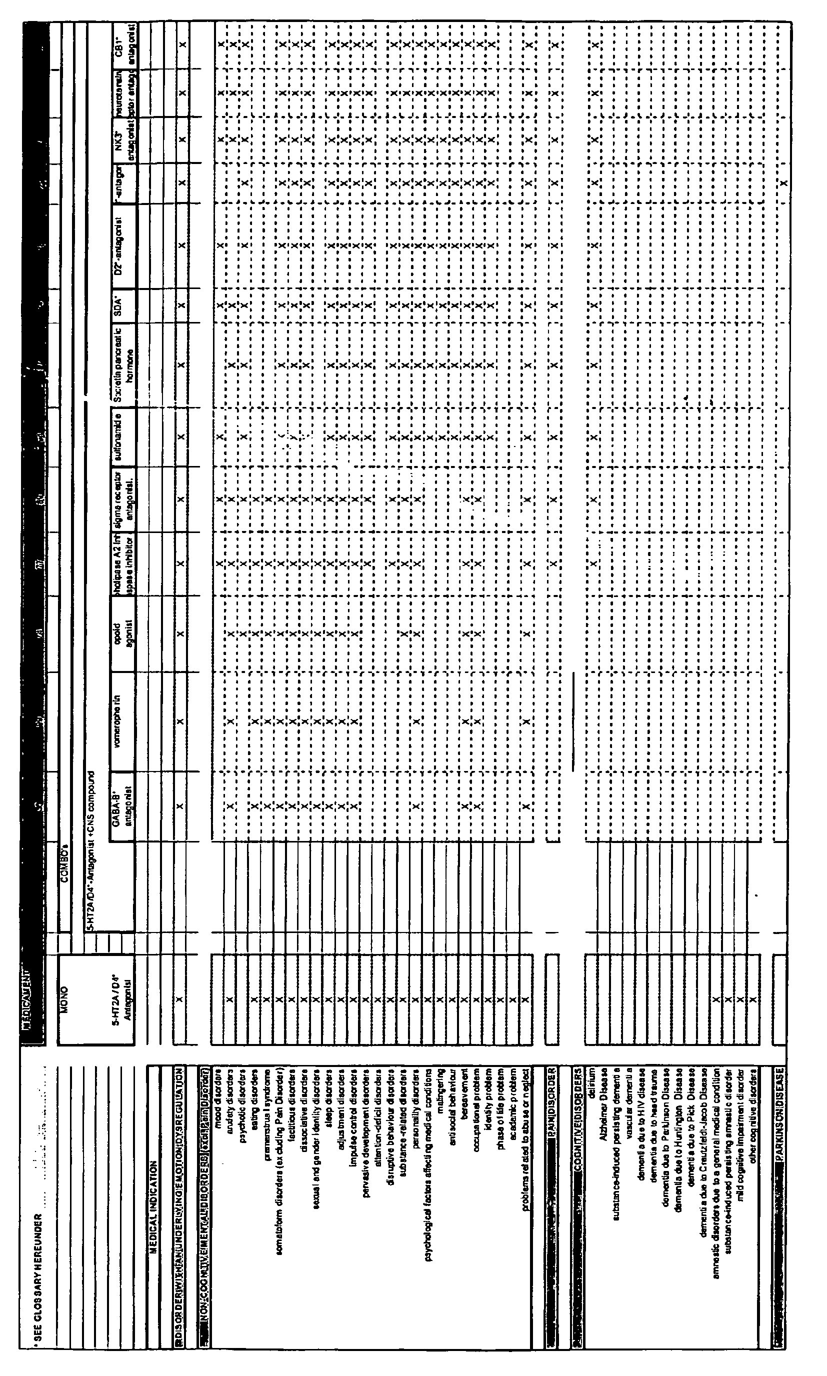

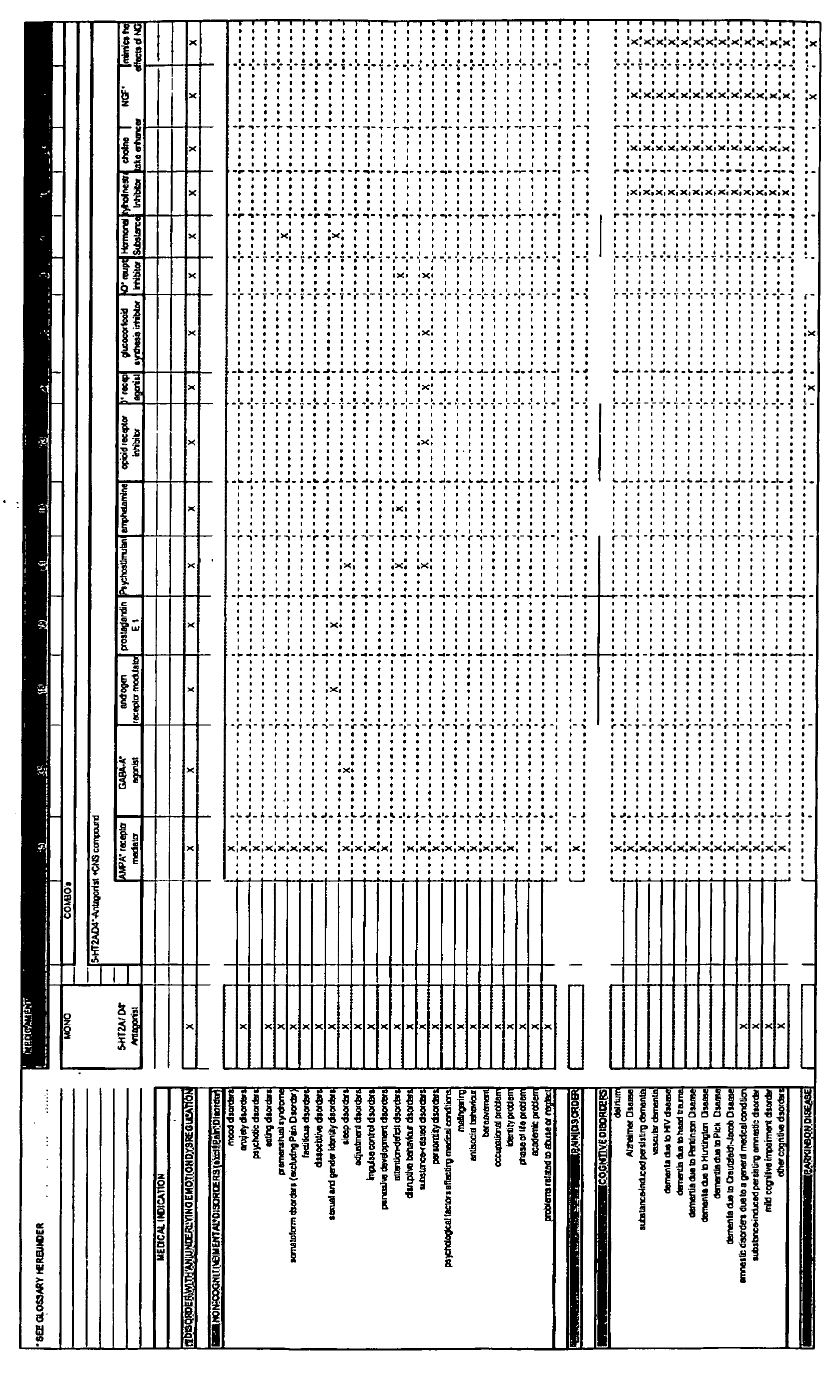

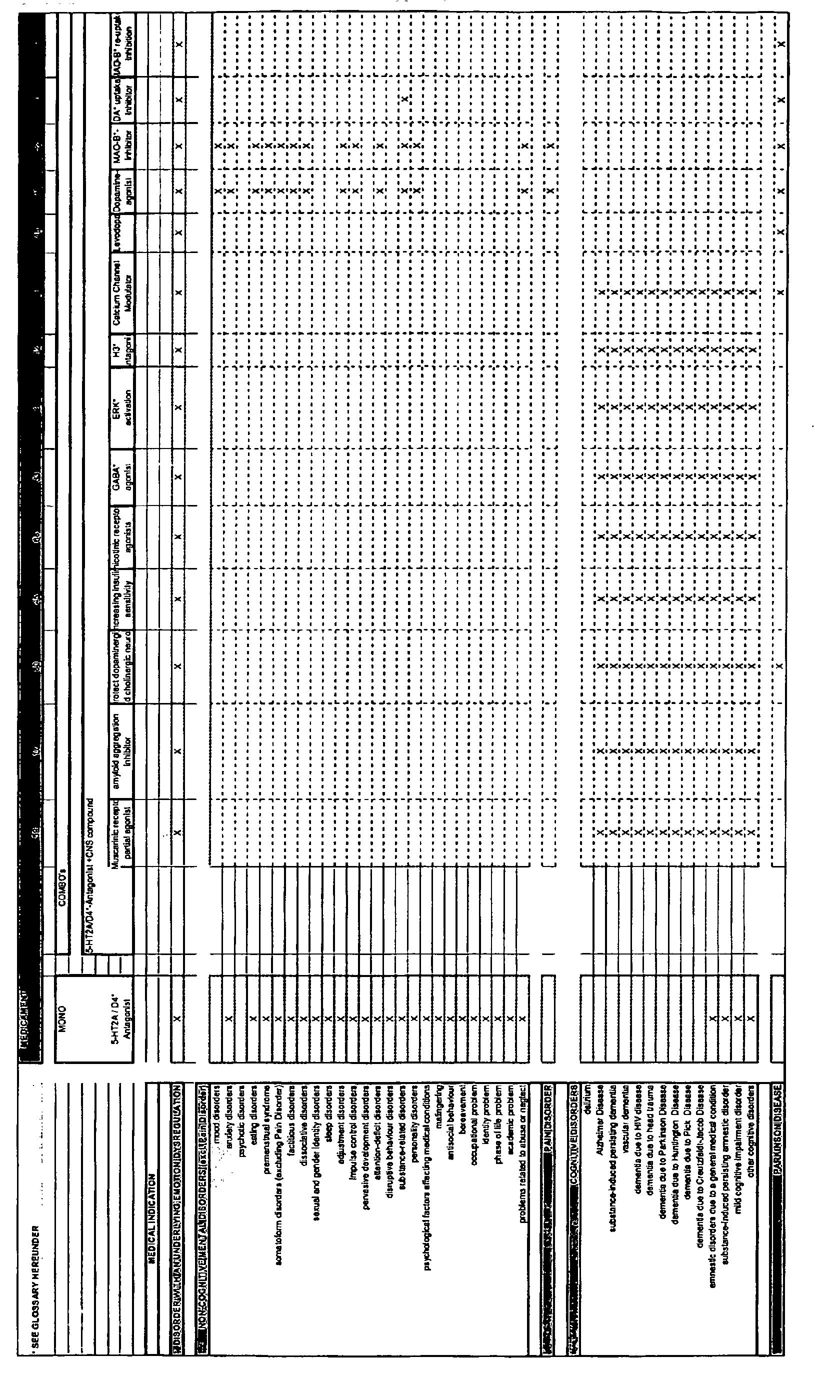

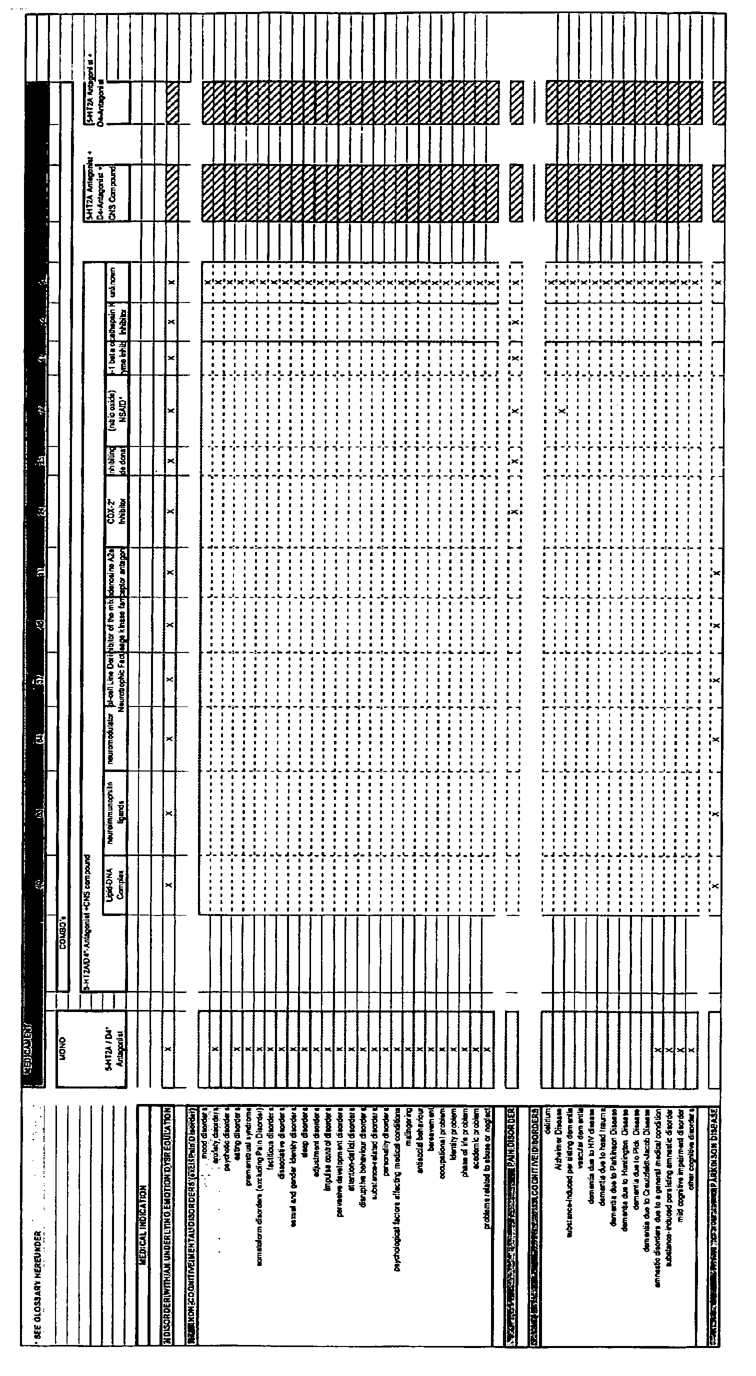

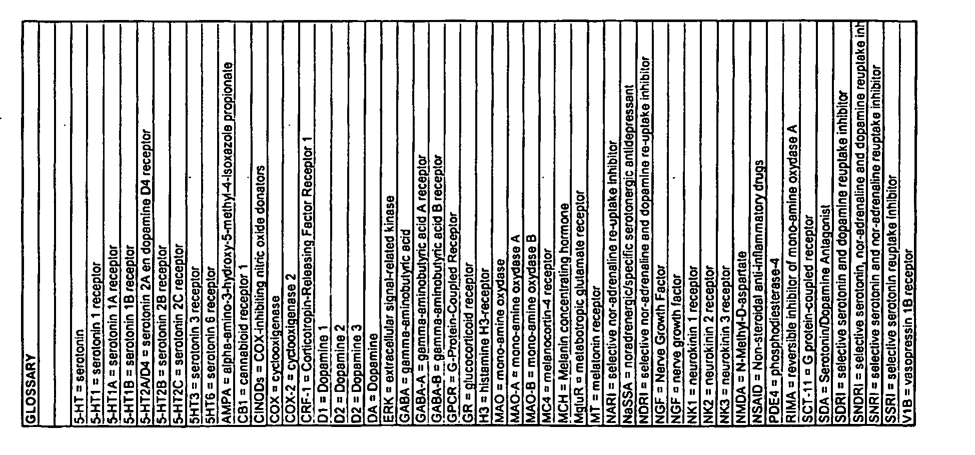

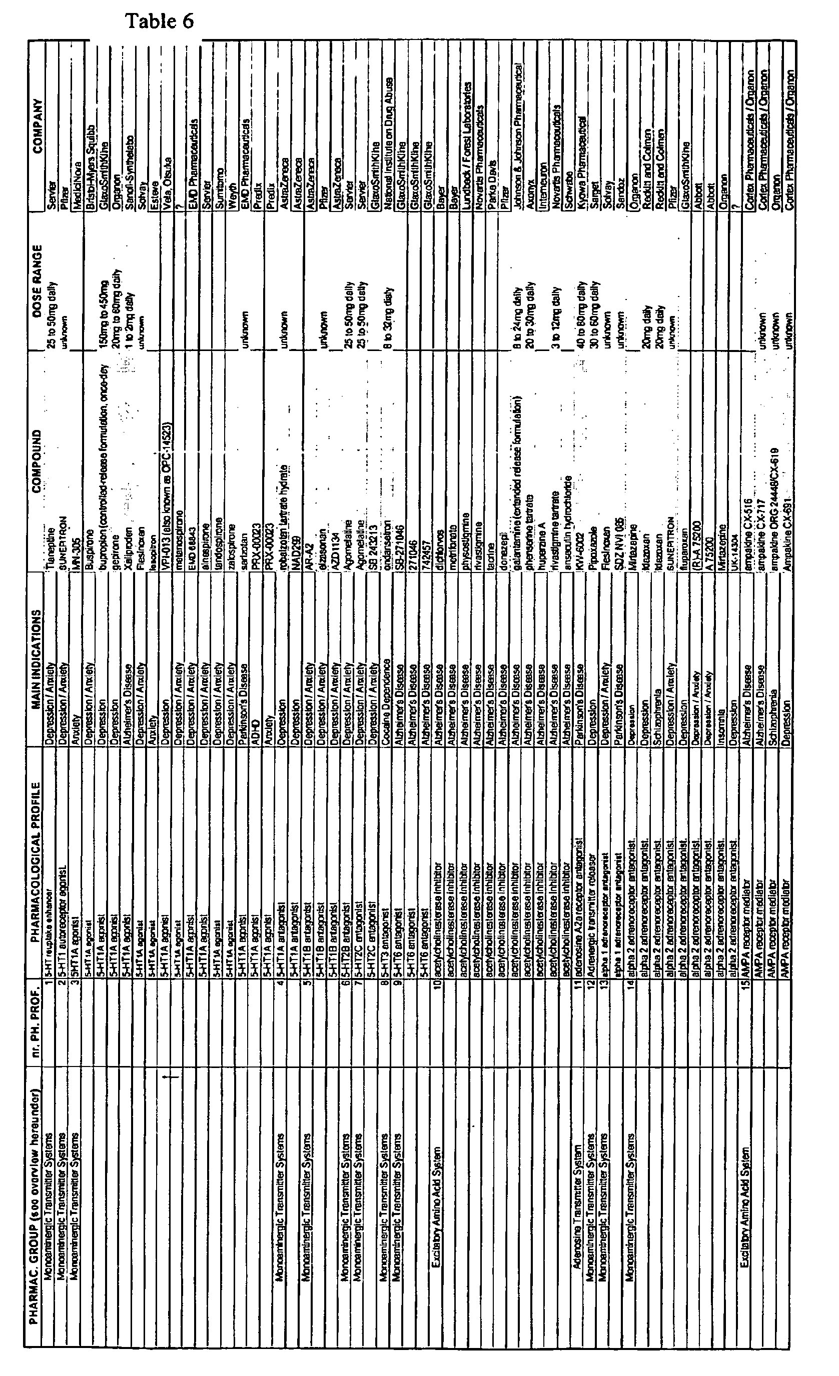

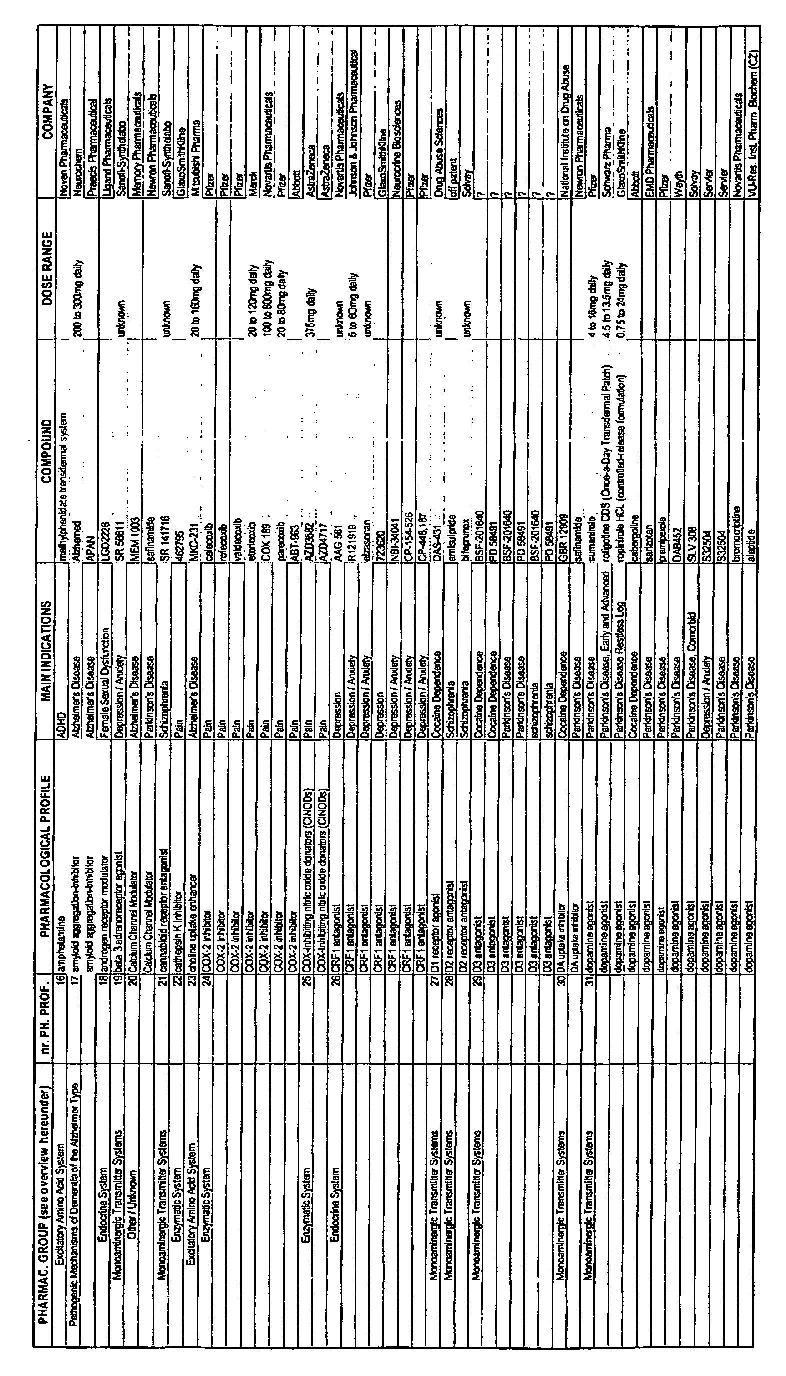

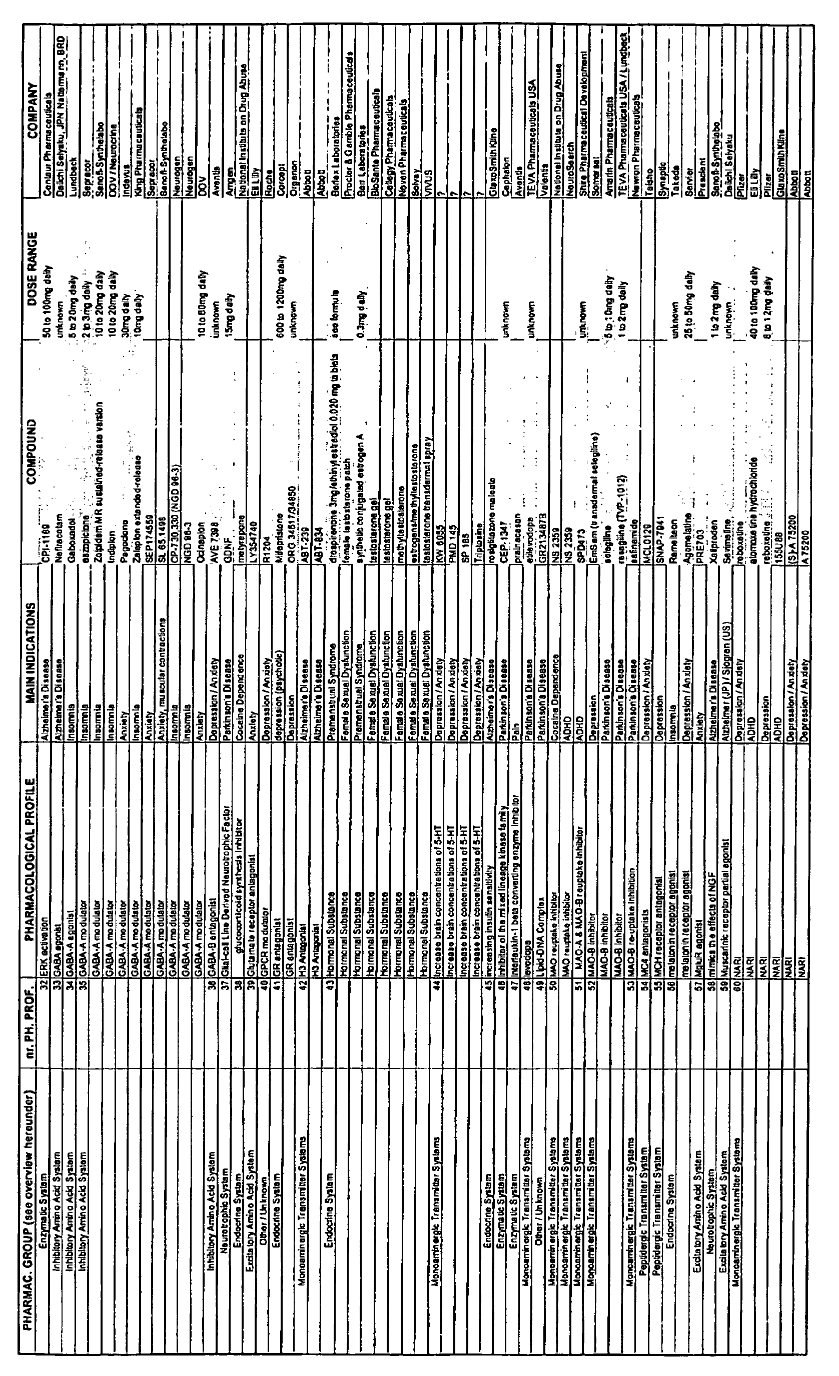

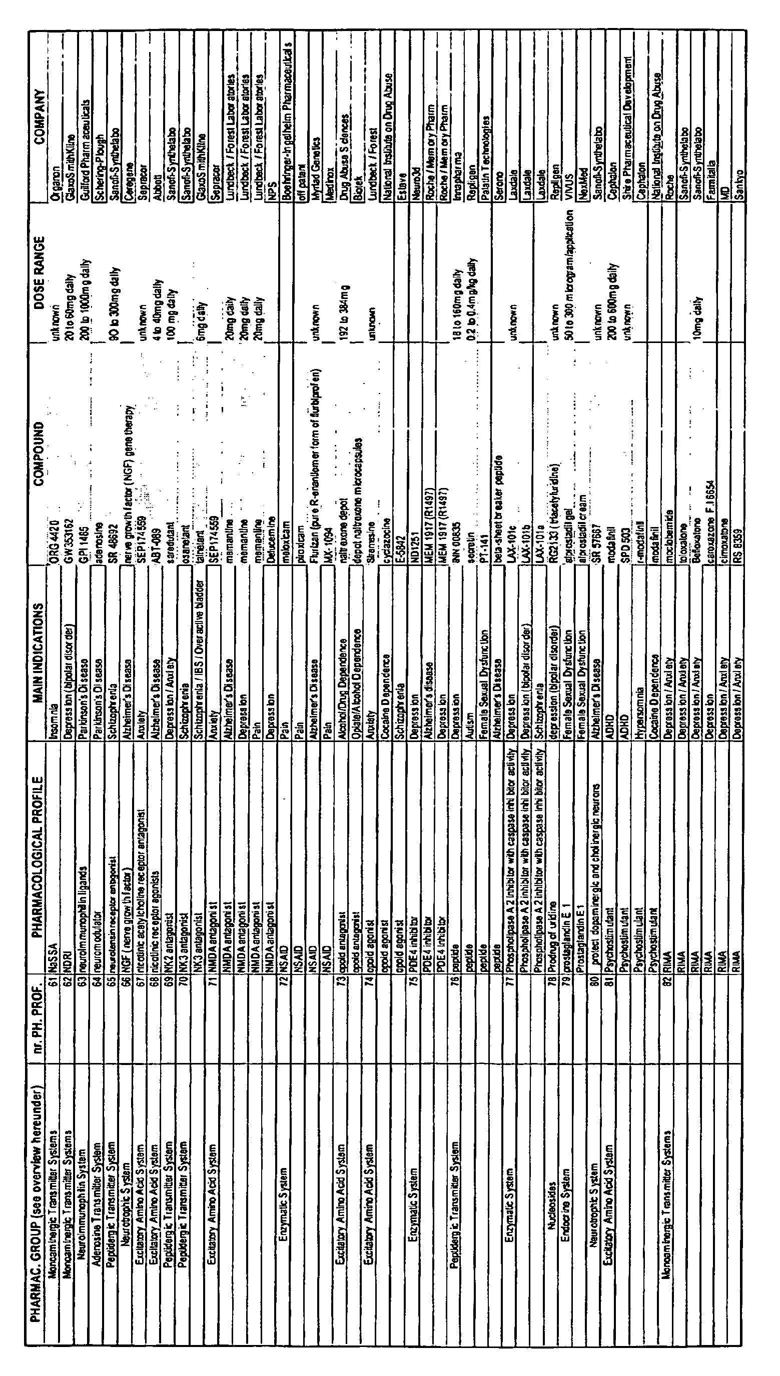

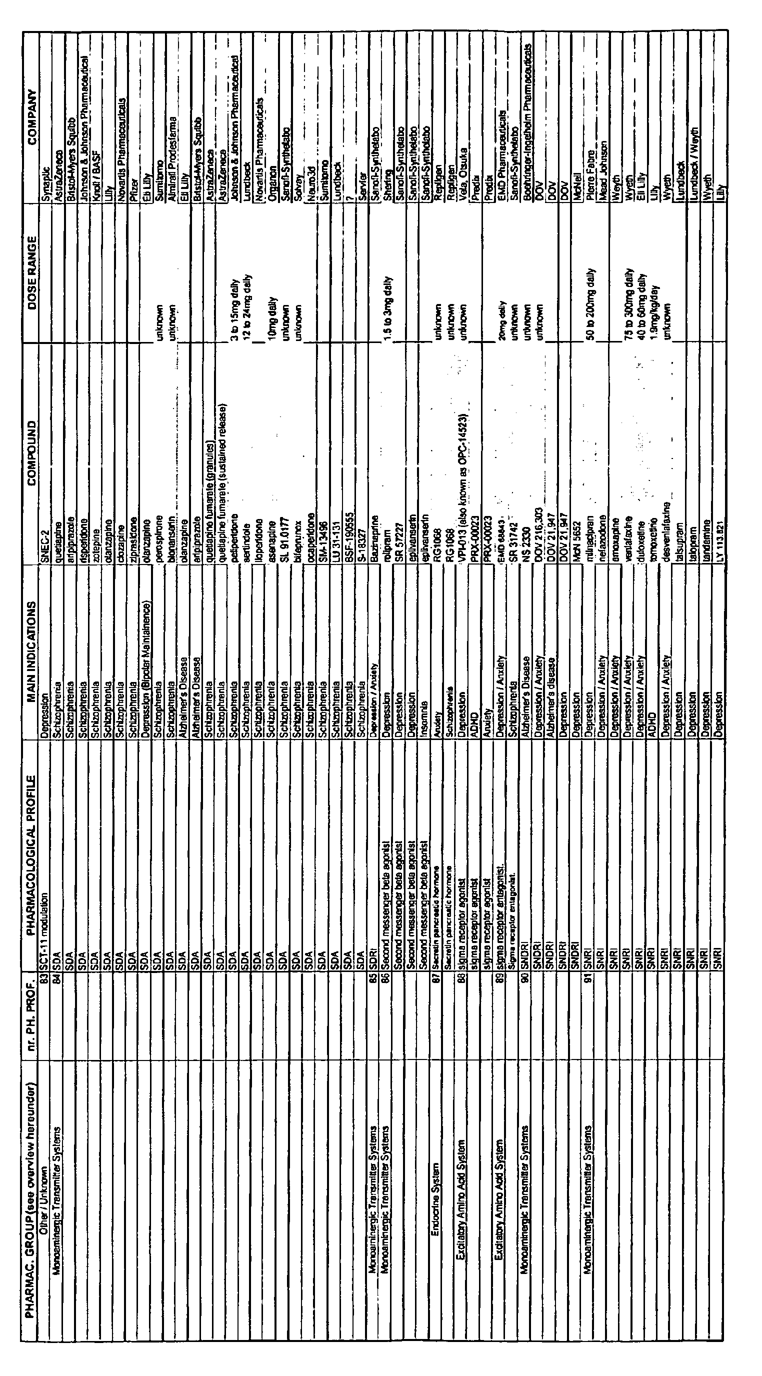

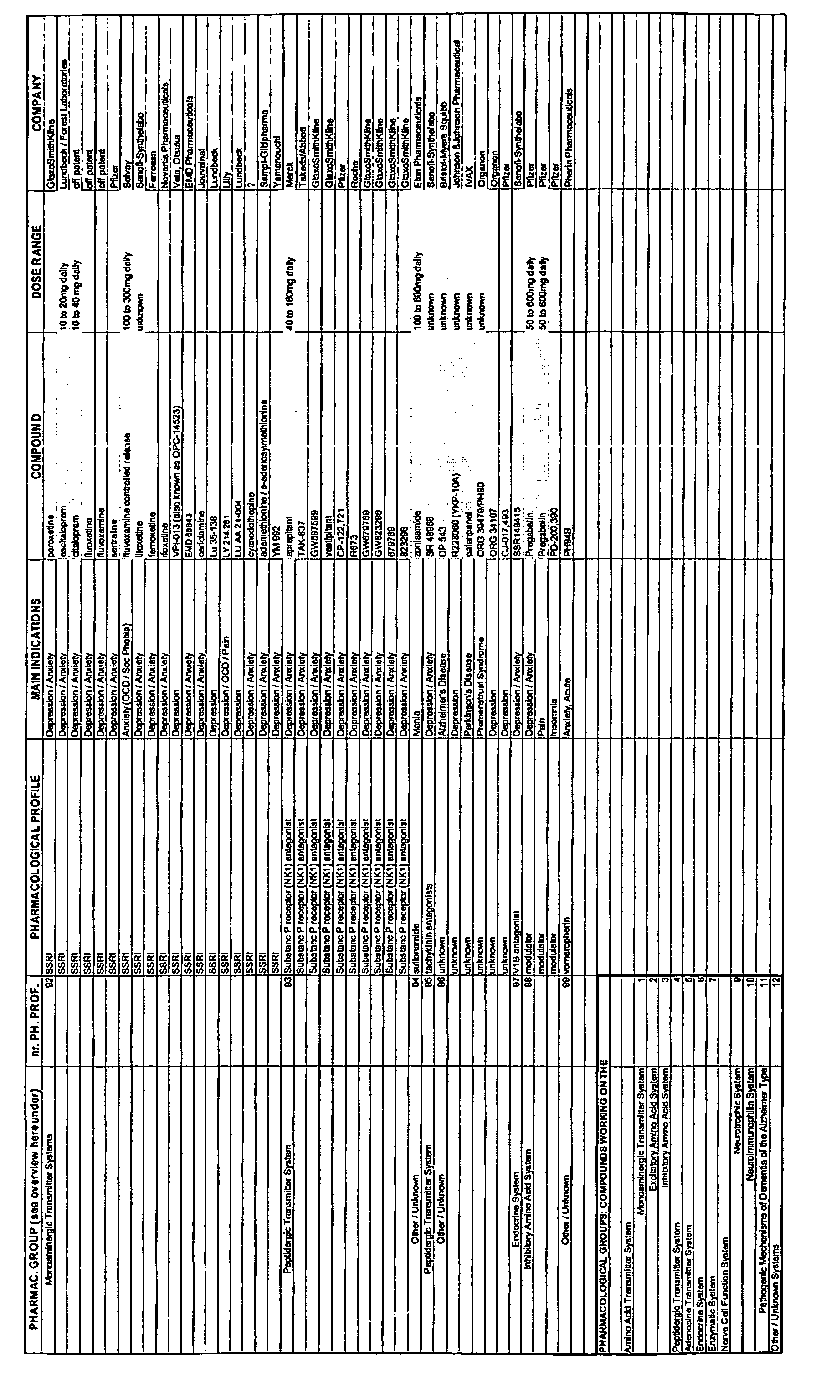

### Examples

### Example 1: Measuring pKi values of test compounds

In Table 1, the pKi values of test compounds are given for each of the dopamine receptors, 5HT receptors, adrenergic receptors and the histamine1 receptor. The affinity of test compounds for the respective receptors has been performed according to conventional procedures known in the art.

An indication "0" means that no affinity has been measured between the test compound and the receptor.

The columns displaying the pKi values for the D4 and the 5-HT2A receptor are filled with dark grey. pKi values between 8 and 9 and higher than 9 are represented by light grey boxes.

### Example 2: Foregoing pipamperon-citalopram treatment in major depressive disorder: a placebo and active controlled period finding clinical trial

Table 2 represents the set-up of a clinical trial comprising for treatment groups:
Group Pic - Active / Day 0 represents the group receiving 10 mg citalopram, twice a day, starting the first day (Day 0) of active treatment in the clinical trial. This administration regime is also indicated as the mono therapy.
Group Pip - Active / Day 0 represents the group receiving a combination of 4 mg pipamperon and 10 mg citalopram, twice a day, starting the first day (Day 0) of active treatment in the clinical trial. This administration regime is also indicated as the non-foregoing combo therapy.
Group Pip - Active / Day 4 represents the group receiving 4 mg pipamperon, twice a day, starting the first day (Day 0) of active treatment in the clinical trial, followed by a combination of 4 mg pipamperon and 10 mg citalopram, twice a day, starting the fifth (Day 4) day of active treatment in the clinical trial. This administration regime is also indicated as the foregoing therapy with combination therapy starting after **4** days of active treatment.
Group Pip - Active / Day 7 represents the group receiving 4 mg pipamperon, twice a day, starting the first day (Day 0) of active treatment in the clinical trial, followed by a combination of 4 mg pipamperon and 10 mg citalopram, twice a day, starting the eight (Day 7) day of active treatment in the clinical trial. This administration regime is also indicated as the foregoing therapy with combination therapy starting after 7 days of active treatment.

All subjects also undergo a placebo (PLC) run-in therapy, administered during a period of about 7 days before the active treatment starts.

During daily (D), weekly (W) or monthly (M) visits, several parameters are measured. Under NECT is to be understood: Neuronal E-clinical Trial = Vesalius Expert development for this trial which includes the bottom-up measurement of:
- In- and exclusion-criteria
- Functional status evaluation
- Medical history
- (Pre-)treatment signs & symptoms
- DSM-IV rules for diagnosis & efficacy
- HDRS-28 (Hamilton Depression Rating Scale - 28 items)
- Medical resource utilisation
- Pre-trial & Concomittant medication
- Drug administration
- (Serious) Adverse events
- Admission to the acute and extension phase of treatment
- Right flow of the trial

### Example 3: combo pipamperon-citalopram: therapeutic use in Major Depression

### Purpose

Pipamperon (1'-[3-(p-Fluorobenzoyl)propyl]-[1,4'-bipiperidine]-4'-carboxamide), the active ingredient of Dipiperon (Janssen-Cilag B.V), administered to patients in a dose ranging between 8 and 12 mg is claimed via its specific pharmacological properties to be a booster of the antidepressant effect of the selective serotonin re-uptake inhibitor citalopram. Preferably, pipamperon is administered daily at least 4-5 days before administering said antidepressant. The mechanism of boosting of pipamperon has to deal with (i) the selective affinity for the dopamine-4 (D4) receptor with a pKi value equal to or higher than 8 towards the D4 receptor and less than 8 towards other dopamine receptors, and (ii) the selective affinity for the 5-HT2A receptor with a pKi value equal to or higher than 8 towards the 5-HT2A receptor and less than 8 towards other 5HT receptors. This semi-naturalistic open label study investigated the efficacy and tolerability of the combo pipamperon - citalopramin the treatment of patients with major depression.

### Details

- Design:: Semi-naturalistic i.e. inclusion of every 'natural' patient in an outpatient practice but without concomitant use of mood enhancing drugs, open label
- Control:: No
- Phase:: Phase IIa - preliminary Proof of Concept
- Location:: Belgium - Research Centre ANIMA, Alken
- End Points :: Assessment scale scores, Hamilton Depression Rating Scale 17 items, Reduction, Response, Remission
- Medication:: Exclusion of mood stabilisers, antipsychotics (typical and atypical) and other antidepressants

### Subjects

| **Type** | **No.** | **Sex** | **Age** |
|---|---|---|---|
| Patients | 23 | 10 male & 13 female | 23-80 (mean 47) years |

Characteristics: patients had a major depressive disorder according to DSM-IV criteria, with or without a chronic course and a treatment refractory state towards another SSRI then citalopram.

### Treatments

### PIP-CIT¹ add-on: citalopram from day minus 60-20 - pipamperon from DAY 0

| **Drug/Treatment** | **Dose** | **Route** | **Frequency** | **Duration** |
|---|---|---|---|---|
| Pipamperon¹ Citalopram¹ | + Pip.: 8-12 mg/day - Cit.: 20- 40 mg/day | PO | bid | 8 weeks |

| | | | | |
|---|---|---|---|---|
| 1. Pipamperon (Pip) and citalopram (Cit) dosage was adjusted according to clinical response. | | | | |

### PIP-CIT¹ fore-going 1-5: pipamperon from day 0 - cital from day 1-5

| **Drug/Treatment** | **Dose** | | **Route** | **Frequency** | **Duration** |
|---|---|---|---|---|---|
| Pipamperon¹ | + Pip.: 8-12 mg/day - | Cit.: 20- | PO | bid | 8 weeks |
| Citalopram¹ | 40 mg/day | | | | |

| | | | | | |
|---|---|---|---|---|---|
| 1. Pipamperon (Pip) and citalopram (Cit) dosage was adjusted according to clinical response. | | | | | |

### PIP-CIT¹ fore-going 6-8: pipamperon from day 0 - citalopram from day 6-8

| **Drug/Treatment** | **Dose** | | **Route** | **Frequency** | **Duration** |
|---|---|---|---|---|---|
| Pipamperon¹ | + Pip.: 8-12 mg/day - | Cit.: 20- | PO | bid | 8 weeks |
| Citalopram¹ | 40 mg/day | | | | |

| | | | | | |
|---|---|---|---|---|---|
| 1. Pipamperon (Pip) and citalopram (Cit) dosage was adjusted according to clinical response. | | | | | |

### Results

| | PIP-CIT add-on | PIP-CIT foregoing | |
|---|---|---|---|
| | **After 20-60 DAYS (mean 33) (n = 5)** | **1-5 DAYS (mean 4) (n =15)** | **6-8 DAYS (mean 7) (n = 3)** |
| **Mean Used Medication** | | | |
| Pipamperone | 9mg/day | 10mg/day | 11mg/day |
| Citalopram | 30mg/day | 26mg/day | 30mg/day |
| **Depression scale scores** | | | |
| HDRS 17-item total score | | | |
| baseline | 29 | 23 | 28 |
| endpoint (week 8) | 4 | 5 | 11 |
| diminishment at week 8 | -25 (+8/-9) | -18 (+8/-8) | -17 (+17/-17) |
| % reduction at week 8 | 86 (+14/-12) | 80 (+20/-30) | 61 (+39/-61) |
| response¹ at week 8 | 5 (100%) | 15 (100%) | 2 (67%) |
| remission² at week 8 | 4 (80%) | 10 (67%) | 1 (33%) |

| | | | |
|---|---|---|---|
| 1. Response = ≥50% reduction in HDRS 17-item score; 2. Remission = HDRS 17-item score < 8 | | | |

Notably, the results obtained are highly significant since the variability in every group is distributed evenly around the mean.

### Add-on PIP-CIT

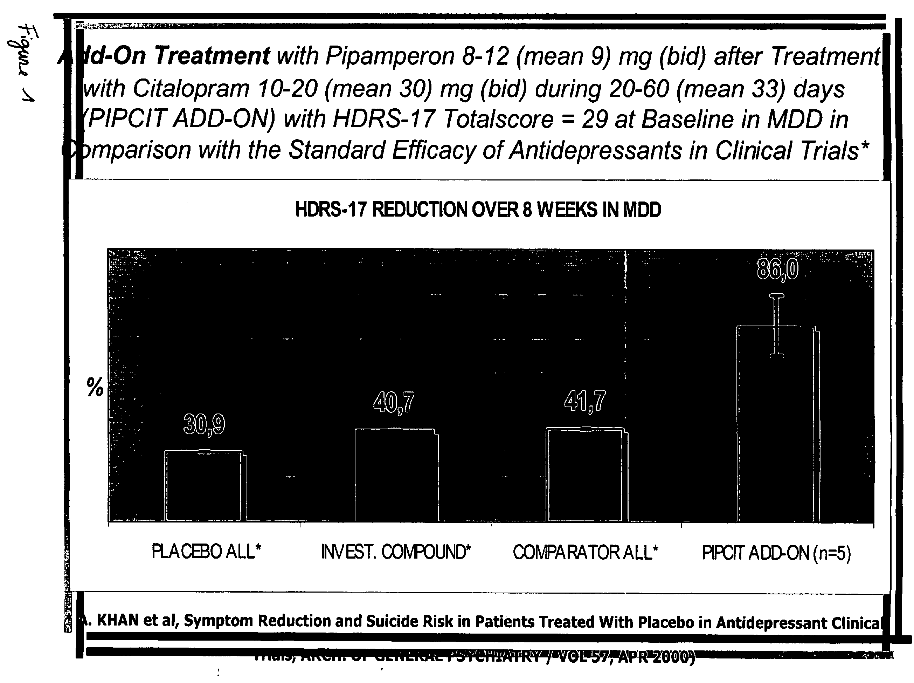

Figure 1 schematically depicts the "add-on" treatment with pipamperon 8-12 (mean 9) mg (bid) after treatment with citalopram 10-20 (mean 30) mg (bid) during 20-60 (mean 33) days (PIPCIT ADD-ON) with HDRS-17. Totalscore is 29 at baseline in MDD in comparison with the standard efficacy of antidepressants in clinical trials according to Khan et al. (2000), in "Symptom Reduction and Suicide Risk in Patients Treated With Placebo in Antidepressant Clinical Trials" (Arch. of General Psychiatry, Vol. 57, April 2000).

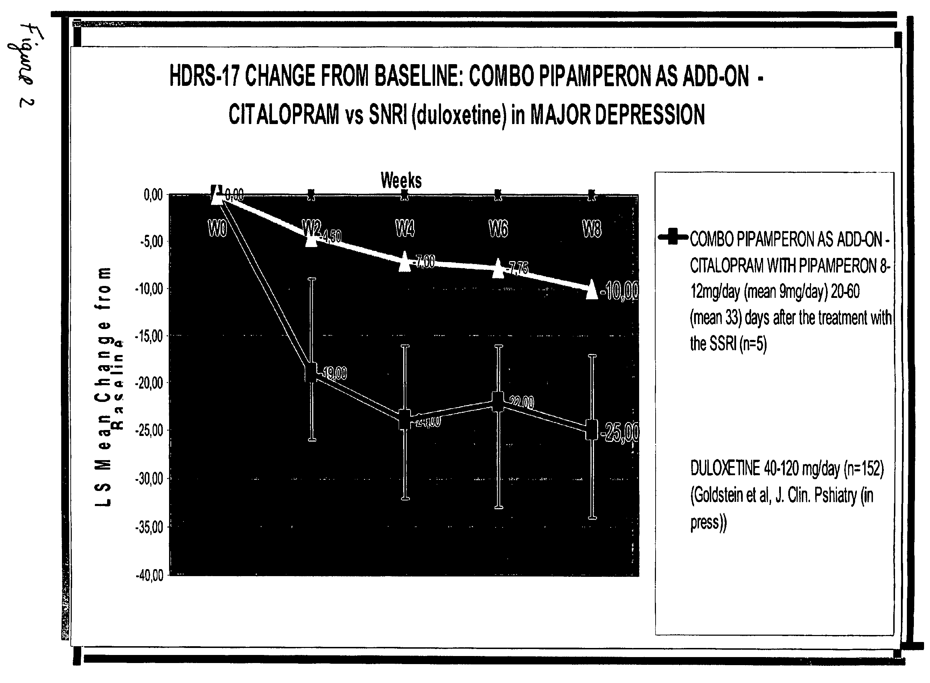

Figure 2 schematically depicts the HDRS-17 change from baseline in the combo pipamperon as "add-on" to citalopram vs SNRI (duloxetine) in Major Depression. Treatment with pipamperon 8-12 (mean 9 mg/day) during 20-60 (mean 33) days after treatment with SSRI (n=5). The SNRI (duloxetine) treatment was 40-120 mg/day (n = 152) according to Goldstein *et al.,* (Clin. Psychiatry, in press).

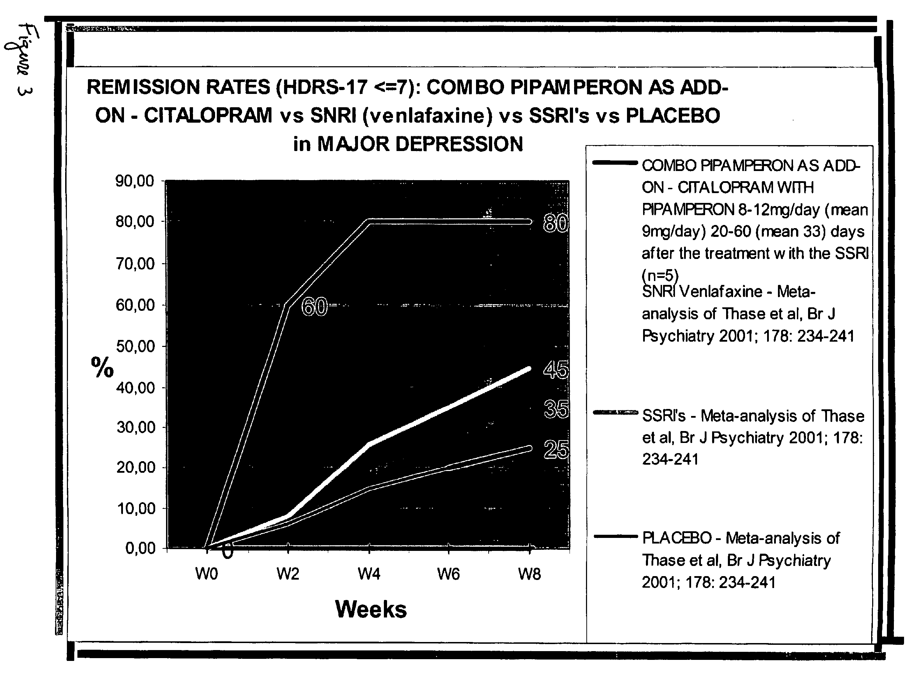

Figure 3 schematically depicts the remission rates (HDRS-17 <=7) with the combo pipamperon as "add-on" to citalopram vs SNRI (venlafaxine) vs SSRIs vs placebo in Major Depression. Treatment with pipamperon 8-12 (mean 9 mg/day) during 20-60 (mean 33) days after treatment with SSRI (n=5). Treatment with the SNRI venlafaxine is acording to a meta-analysis of Thase et al. (Br. J. Psychiatry (2001) 178:234-241). Treatment with SSRIs is according to a meta-analysis of Thase et al. (Br. J. Psychiatry (2001) 178:234-241). Treatment with placebo is according to a meta-analysis of Thase et al. (Br. J. Psychiatry (2001) 178:234-241).

### Fore-going 1-5 PIP-CIT

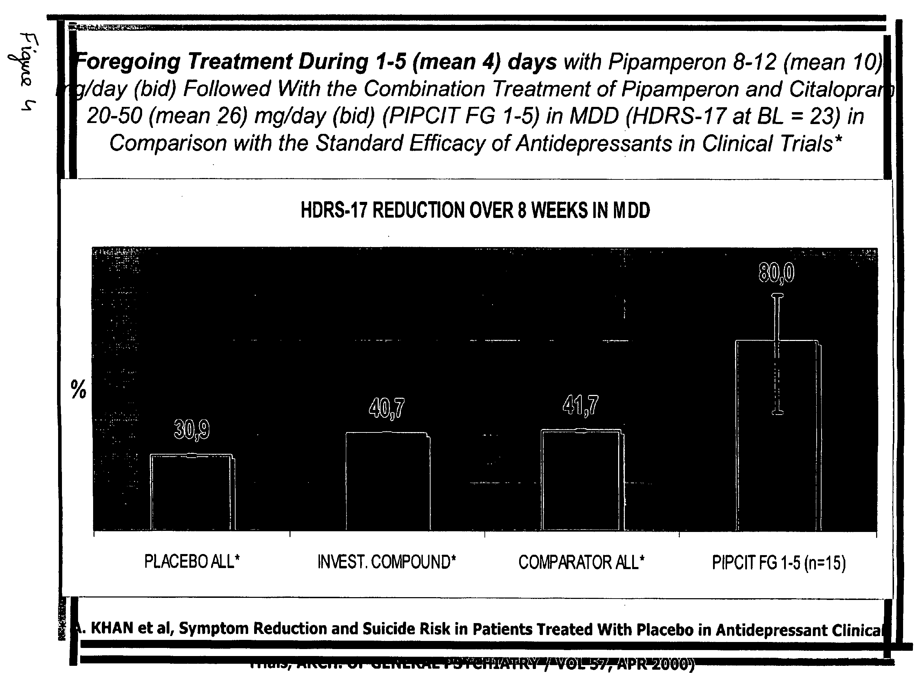

Figure 4 schematically depicts the "fore-going" treatment during 1-5 (mean 4) days with pipamperon 8-12 (mean 10) mg (bid), followed with the combination treatment of pipamperon and citalopram 20-50 (mean 26) mg/day (bid) (PIPCIT FG 1-5) in MDD (HDRS-17 at BL = 23) in comparison with the standard efficacy of antidepressants in clinical trials according to Khan et al. (2000), in "Symptom Reduction and Suicide Risk in Patients Treated With Placebo in Antidepressant Clinical Trials" (Arch. of General Psychiatry, Vol. 57, April 2000).

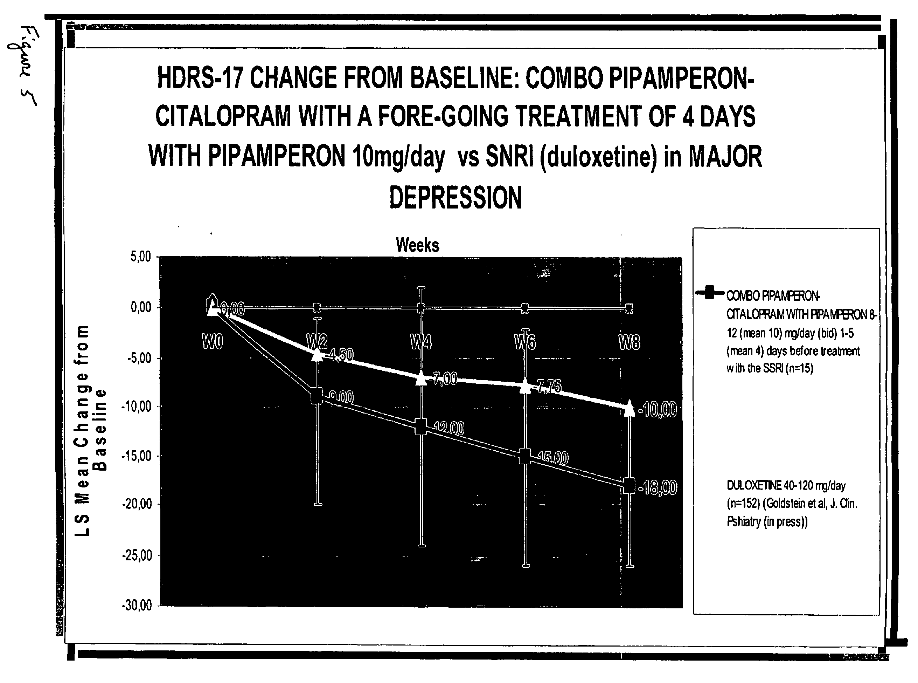

Figure 5 schematically depicts the HDRS-17 change from baseline in the combo pipamperon-citalopram treatment with a "fore-going" treatment of 4 days with pipamperon (10 mg/day) vs SNRI (duloxetine) in Major Depression. Treatment with the combo pipamperon-citalopram with pipamperon 8-12 (mean 10 mg/day) (bid) 1-5 (mean 4) days before treatment with SSRI (n=15). The SNRI (duloxetine) treatment was 40-120 mg/day (n = 152) according to Goldstein *et al.,* (Clin. Psychiatry, in press).

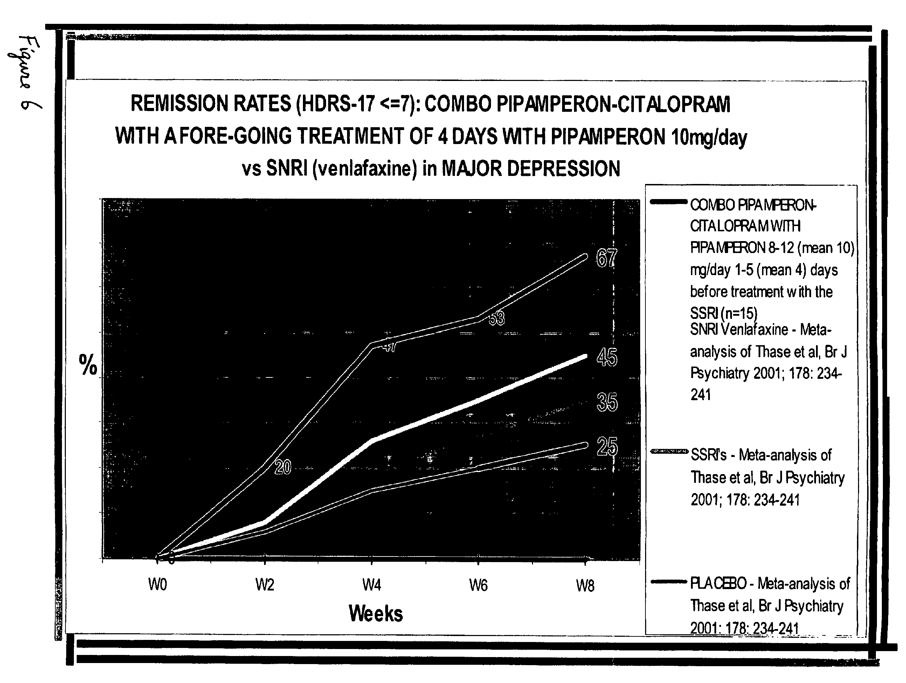

Figure 6 schematically depicts the remission rates (HDRS-17 <=7) with the combo pipamperon with a "fore-going" treatment of 4 days with pipamperon (10 mg/day) vs SNRI (venlafaxine) in Major Depression. Treatment with the combo pipamperon-citalopram was with pipamperon 8-12 (mean 10 mg/day) during 1-5 (mean 4) days before treatment with the SSRI (n=5). Treatment with the SNRI venlafaxine is acording to a meta-analysis of Thase et al. (Br. J. Psychiatry (2001) 178:234-241). Treatment with SSRIs is according to a meta-analysis of Thase et al. (Br. J. Psychiatry (2001) 178:234-241). Treatment with placebo is according to a meta-analysis of Thase et al. (Br. J. Psychiatry (2001) 178:234-241).

### Fore-going 6-8 PIP-CIT

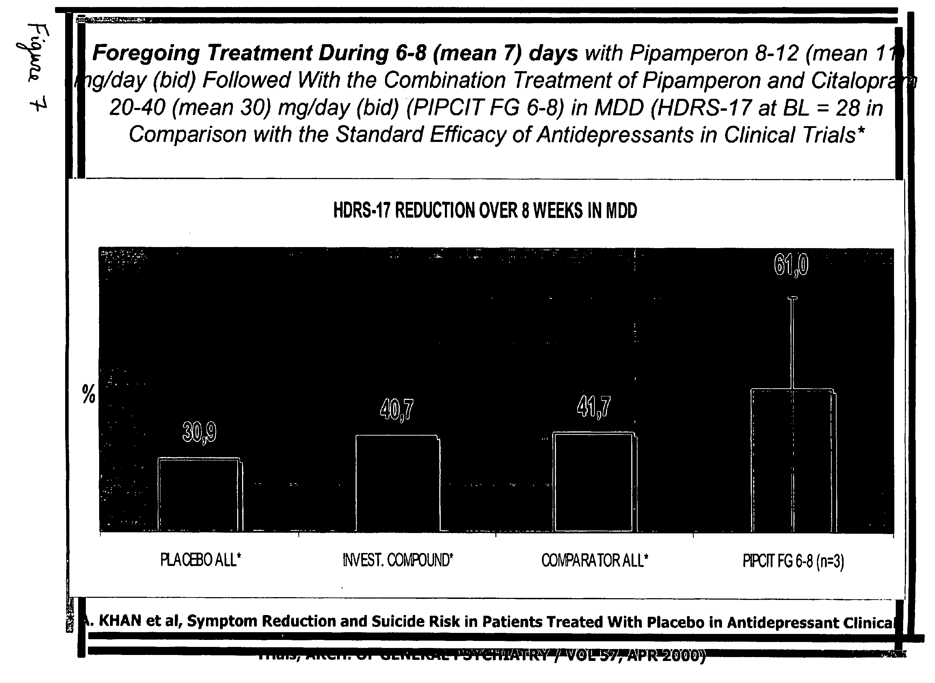

Figure 7 schematically depicts the "fore-going" treatment during 6-8 (mean 7) days with pipamperon 8-12 (mean 11) mg/day (bid), followed with the combination treatment of pipamperon and citalopram 20-40 (mean 30) mg/day (bid) (PIPCIT FG 6-8) in MDD (HDRS-17 at BL = 28) in comparison with the standard efficacy of antidepressants in clinical trials according to Khan et al. (2000), in "Symptom Reduction and Suicide Risk in Patients Treated With Placebo in Antidepressant Clinical Trials" (Arch. of General Psychiatry, Vol. 57, April 2000).

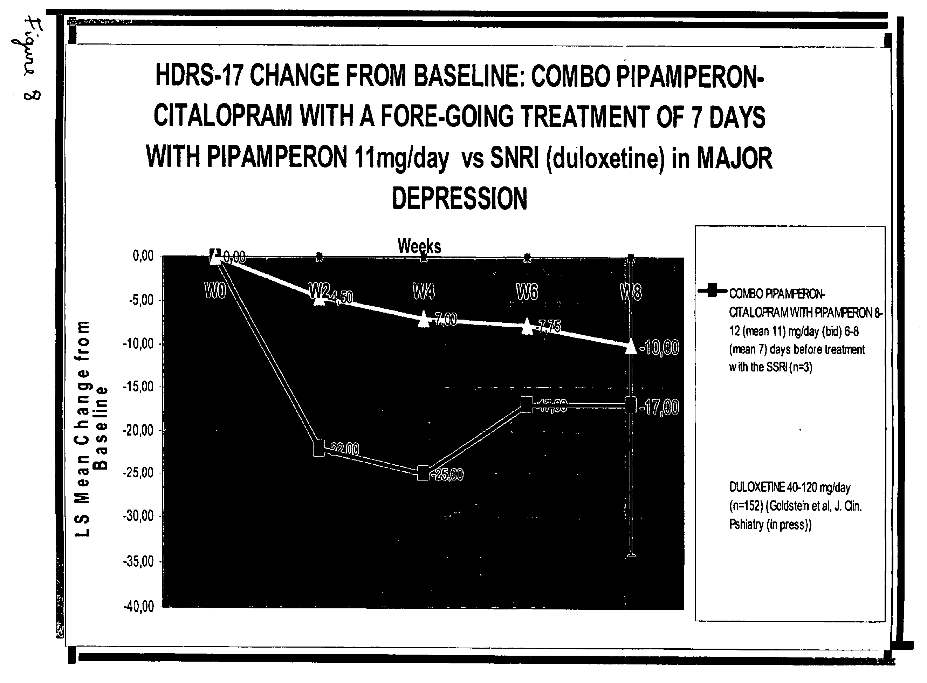

Figure 8 schematically depicts the HDRS-17 change from baseline in the combo pipamperon-citalopram treatment with a "fore-going" treatment of 7 days with pipamperon (11 mg/day) vs SNRI (duloxetine) in Major Depression. Treatment with the combo pipamperon-citalopram with pipamperon 8-12 (mean 11 mg/day) (bid) 6-8 (mean 7) days before treatment with SSRI (n=3). The SNRI (duloxetine) treatment was 40-120 mg/day (n = 152) according to Goldstein *et al.,* (Clin. Psychiatry, in press).

### Comparison "add-on" vs "fore-going"

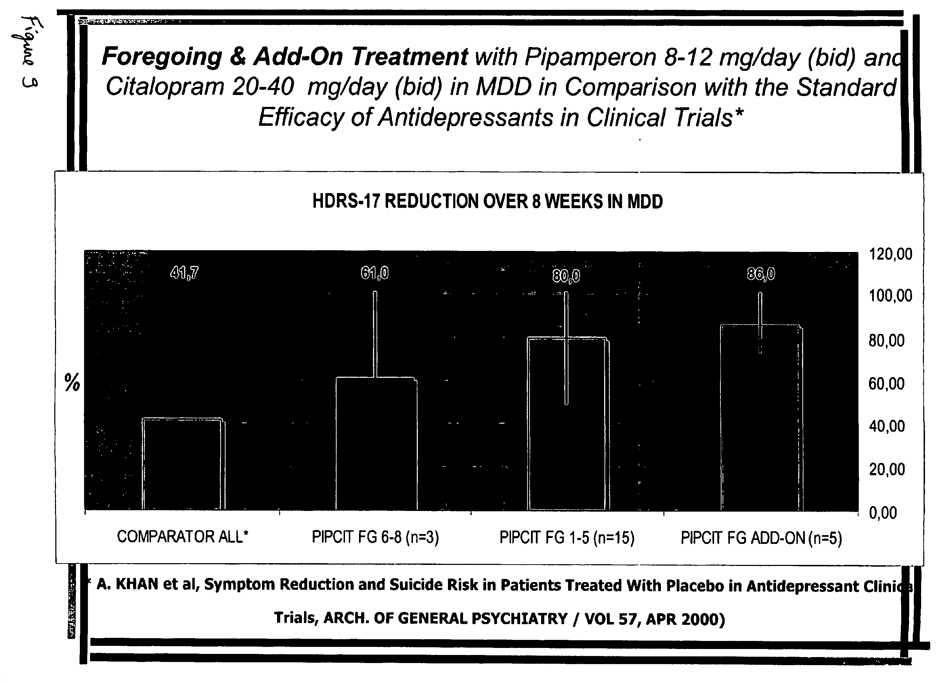

Figure 9 schematically depicts a comparison between "fore-going" and "add-on" treatments with pipamperon (8-12 mg/day; bid) and citalopram (20-40 mg/day; bid) in MDD in comparison with the standard efficacy of antidepressants in clinnical trials according to Khan et al. (2000), in "Symptom Reduction and Suicide Risk in Patients Treated With Placebo in Antidepressant Clinical Trials" (Arch. of General Psychiatry, Vol. 57, April 2000).

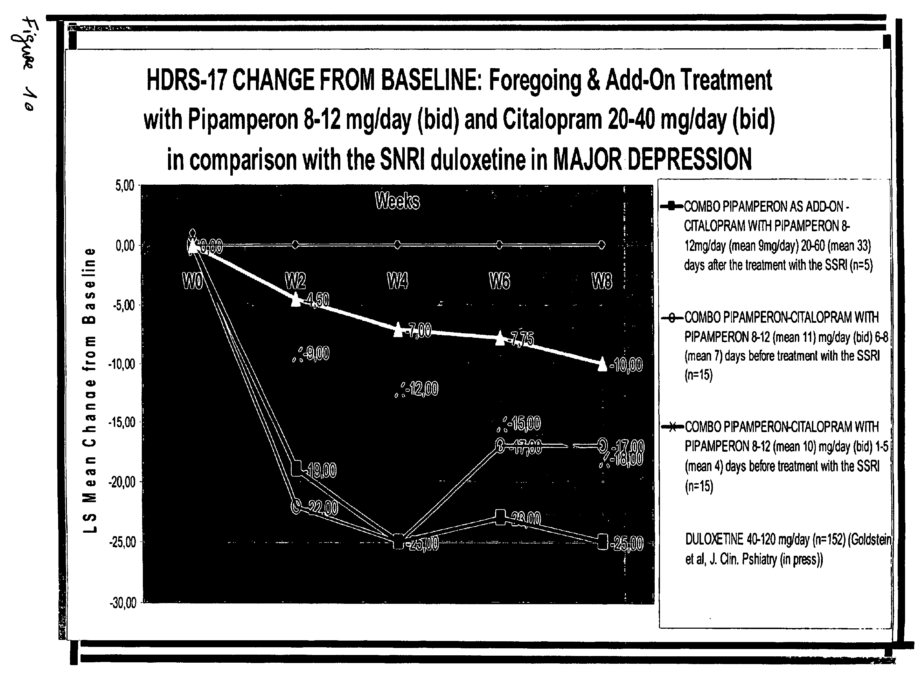

Figure 10 schematically depicts a comparison between "fore-going" and "add-on" treatments. In particular, the HDRS-17 change from baseline between "fore-going" and "add-on" treatment with pipamperon (8-12 mg/day; bid) and citalopram (20-40 mg/day; bid) in comparison with the SNRI duloxetine in Major Depression is depicted. Treatment with the combo pipamperon as "add-on" to citalopram, with pipamperon 8-12 mg/day (mean 9 mg/day) 20-60 (mean 33) days after treatment with the SSRI (n=5). Treatment with the combo pipamperon-citalopram, with pipamperon 8-12 mg/day (mean 11 mg/day; bid) 6-8 days (mean 7 days) before treatment with the SSRI (n = 15). Treatment with the combo pipamperon-citalopram, with pipamperon 8-12 mg/day (mean 10 mg/day; bid) 1-5 days (mean 4 days) before treatment with the SSRI (n = 15). The SNRI (duloxetine) treatment was 40-120 mg/day (n = 152) according to Goldstein et al., (Clin. Psychiatry, in press).

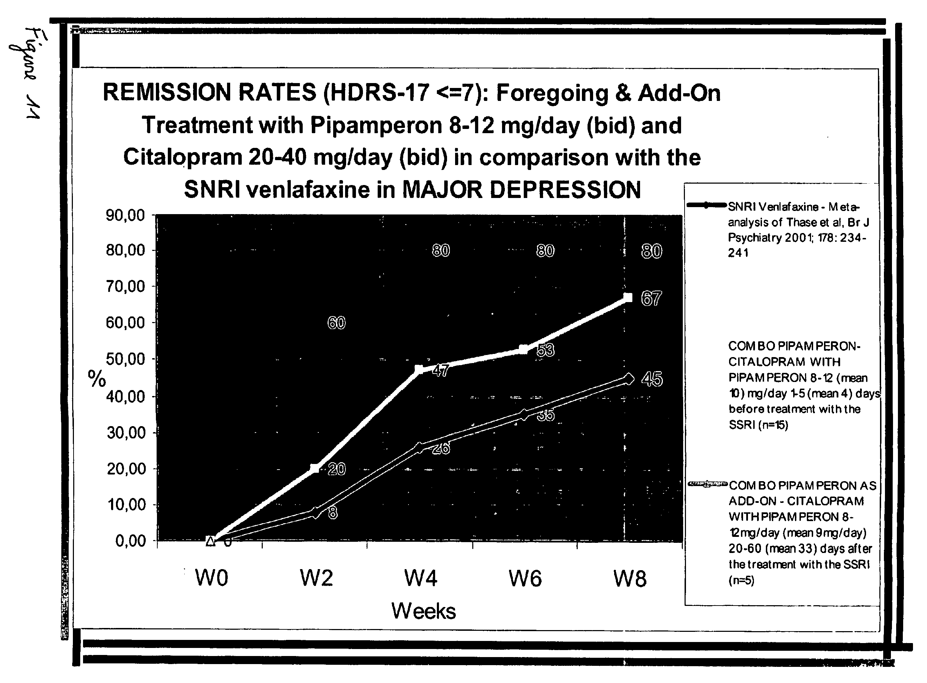

Figure 11 schematically depicts the remission rates (HDRS-17 <=7) in a comparison between "fore-going" and "add-on" treatment with pipamperon (8-12 mg/day; bid) and citalopram (20-40 mg/day; bid) in comparison with the SNRI venlafaxine in Major Depression. Treatment with the combo pipamperon-citalopram was with pipamperon 8-12 (mean 10 mg/day) during 1-5 (mean 4) days before treatment with the SSRI (n = 15). Treatment with the SNRI venlafaxine is acording to a meta-analysis of Thase et al. (Br. J. Psychiatry (2001) 178:234-241). Treatment with pipamperon as "add-on" to citalopram, with pipamperon 8-12 (mean 9 mg/day) during 20-60 (mean 33) days after treatment with SSRI (n = 5).

The intention-to-treat / last-observation-carried-forward analysis showed a high therapeutic efficacy according HDRS 17-item in all the treatment groups. This was especially true for the 'add-on' group probably caused by the longer treatment with an active antidepressant (+33 days). The huge therapeutic effect observed in the 'PIP-CIT 1-5' group present for at a mean dosage of pipamperon of 10 mg per day and administered the first four days of treatment without an active antidepressant, indicates the boosting effect of pipamperon on the SSRI citalopram at an extremely and thus unconventional low dose. Only 1 patient discontinued treatment due to a lost of follow-up.

### Adverse Events

| **Side effects (patients)** | **PIP-CIT add-on** | **PIP-CIT foregoing** | |
|---|---|---|---|
| | **After 20-60 DAYS (mean 33) (n = 5)** | **1-5 DAYS (mean 4) (n=15)** | **6-8 DAYS (mean 7) (n = 3)** |
| Discontinued treatment due to adverse events | 0 | 0 | 0 |
| By system: | | | |
| body as a whole | 0 | 0 | 0 |
| central and peripheral nervous system | 1 (20%) | 4(26.6%) | 0 |
| gastrointestinal | 1(20%) | 5(33%) | 2(66.6%) |
| musculoskeletal | 1(20%) | 3(20%) | 0 |
| psychiatric | 0 | 0 | 0 |
| respiratory | 0 | 1 (6.6%) | 0 |
| skin and appendages | 1(20%) | 2(13.3%) | 1(33.3%) |
| vascular | 0 | 1(6.6%) | 0 |
| urinary | 0 | 1(6.6%) | 0 |

Laboratory parameters, ECG, bodyweight and vital signs were not measured since this was a naturalistic study.

### Assessment

### Outcome

**Efficacy:** the 4-day fore-going combo pipamperon 8-12mg/d - citalopram 20-40mg/day is comparable to the add-on combo pipamperon-citalopram.

**Efficacy:** the 4-day fore-going combo pipamperon 8-12mg/d - citalopram 20-40mg/day is larger than the 7-day fore-going combo pipamperon 8-12mg/d - citalopram 20-40mg/day.

**Efficacy:** the combo pipamperon 8-12mg/d - citalopram 20-40mg/day is larger than the in the art known antidepressants SSRIs.

### Tolerability

**Tolerability:** the 4-day fore-going treatment is comparable to the 7-day fore-going combo is comparable to add-on combo pipamperon-citalopram.

**Tolerability:** no discontinued treatment due to adverse events.

### Study Messages

The boosting effect of pipamperon at an extremely unconventional low dose on a SSRI is indicated since the efficacy of the 'add-on' and '4-day fore-going' combo 'pipamperon 8 -12 mg/d - citalopram 20 - 40 mg/day' is in this study as twice higher as known in the art in the treatment of patients with major depression.

The combo pipamperon-citalopram is generally well tolerated in patients with depression i.e. at least no specific added adverse events were occurring by adding pipamperon at the doses used in the study.

### Example 4: combo pipamperon-citalopram: therapeutic use in Obsessive-Compulsive Disorder (OCD).

### Purpose

Pipamperon (1'-[3-(p-Fluorobenzoyl)propyl]-[1,4'-bipiperidine]-4'-carboxamide), the active ingredient of Dipiperon (Janssen-Cilag B.V), administered to a patient in a dose ranging between 8 and 12 mg is claimed via its specific pharmacological properties to be a booster of the effect of the selective serotonin re-uptake inhibitor citalopram towards OCD. Preferably, pipamperon is administered daily at least 4-5 days before administering said antidepressant. The mechanism of boosting of pipamperon has to deal with (i) the selective affinity for the dopamine-4 (D4) receptor with a pKi value equal to or higher than 8 towards the D4 receptor and less than 8 towards other Dopamine receptors, and (ii) the selective affinity for the 5-HT2A receptor with a pKi value equal to or higher than 8 towards the 5-HT2A receptor and less than 8 towards other 5HT receptors. This semi-naturalistic open label study investigated the efficacy and tolerability of the combo pipamperon - citalopram in the treatment of patients with OCD.

### Details

- Design:: Semi-naturalistic i.e. inclusion of every 'natural' patient in an outpatient practice but without concomitant use of mood enhancing drugs, open label
- Control:: No
- Phase:: Phase IIa - preliminary Proof of Concept
- Location:: Belgium - Research Centre ANIMA, Alken
- End Points :: Assessment scale scores, Yale-Brown Obsessive-Compulsive Scale, Reduction, Remission
- Medication:: Exclusion of mood stabilisers, antipsychotics (typical and atypical) and other antidepressants

### Subjects

| **Type** | **No.** | **Sex** | **Age** |
|---|---|---|---|
| Patients | 7 | 1 male & 7 female | 20-63 (mean 33) years |

Characteristics: patients had an obsessive-compulsive disorder according to DSM-IV criteria, with or without a chronic course and a treatment refractory state towards another SSRI then citalopram.

### Treatments

### PIP-CIT¹ ADD-ON: citalopram from DAY minus 730-60 - pipamperon from DAY 0

| **Drug/Treatment** | **Dose** | | **Route** | **Frequency** | **Duration** |
|---|---|---|---|---|---|
| Pipamperone¹ | + Pip.: 8-16 mg/day - | Cit.: 30-80 | PO | bid | 12 |
| Citalopram¹ | mg/day | | | | weeks |

| | | | | | |
|---|---|---|---|---|---|
| 1. Pipamperone (Pip) and Citalopram (Cit) dosage was adjusted according to clinical response. | | | | | |

### PIP-CIT¹ FORE-GOING 4-6: pipamperon from DAY 0 - citalopram from DAY 4-6

| **Drug/Treatment** | **Dose** | | **Route** | **Frequency** | **Duration** |
|---|---|---|---|---|---|
| Pipamperone¹ | + Pip.: 8-16 mg/day - | Cit.: 30-80 | PO | bid | 12 |
| Citalopram¹ | mg/day | | | | weeks |

| | | | | | |
|---|---|---|---|---|---|
| 1. Pipamperone (Pip) and Citalopram (Cit) dosage was adjusted according to clinical response. | | | | | |

### Results

| | |
|---|---|
| | **PIP-CIT add-on** |
| | after 730-60 DAYS (mean 241) (n = 6) |
| | with mean Cit. 54mg/d and Pip. 11 mg/d |
| | |
| | **PIP-CIT foregoing** |
| | 4-6 DAYS (mean 5) (n = 2) |
| | with mean Cit. 60mg/d and Pip. 10mg/d |

| **Y-BOCS score** | |
|---|---|
| Baseline | |
| Total | 31 |
| Obsessions | 18 |
| Compulsions | 13 |
| Endpoint (week 12) | |
| | |
| Total | 15 |
| | |
| diminishment | -16 (+16/-11) |
| | |
| % reduction | 53 |
| | |

| Obsessions | |
|---|---|
| total | 8 |
| diminishment | -10 (+9/-7) |
| % reduction | 57 |

| Compulsions | |
|---|---|
| total | 7 |
| diminishment | -6 (+7/-6) |
| % reduction | 45 |

| % Remission | |
|---|---|
| YBOCS score ≤8 | 29 |
| BOCS score ≤16 | 57 |

Notably, the results obtained are highly significant since the variability in every group is distributed evenly around the mean.

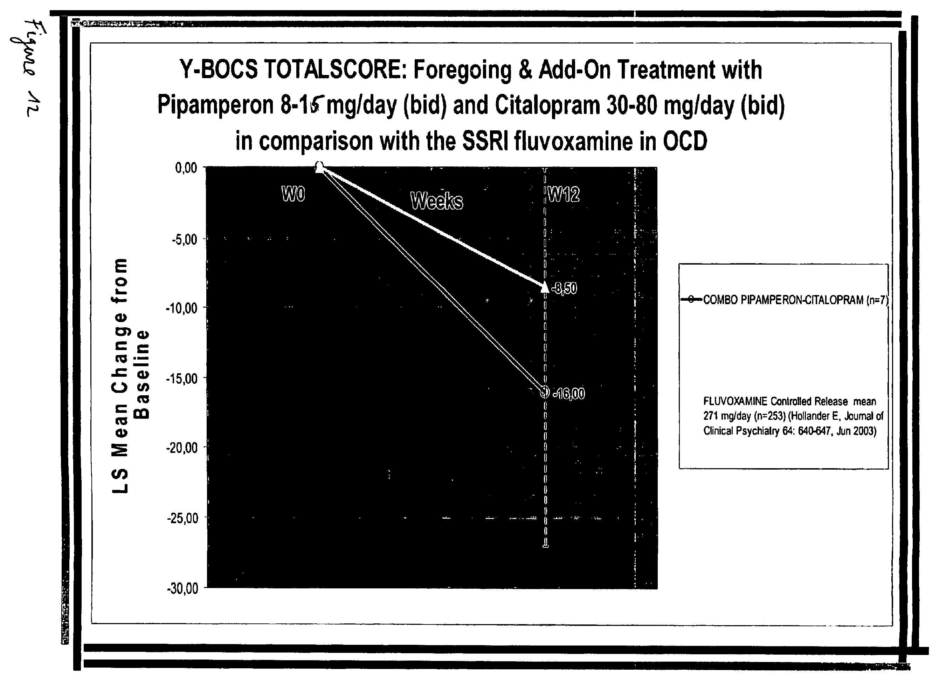

Figure 12 schematically depicts the Y-BOCS total score: "fore-going" and "add-on" treatment with pipamperon (8-15 mg/day; bid) and citalopram (30-80 mg/day; bid) in comparison with the SSRI fluvoxamine in OCD. Treatment with the combo pipamperon-citalopram (n = 7). Treatment with fluvoxamine (controlled release) mean 271 mg/day (n = 253) is according to Hollander et al. (2003).

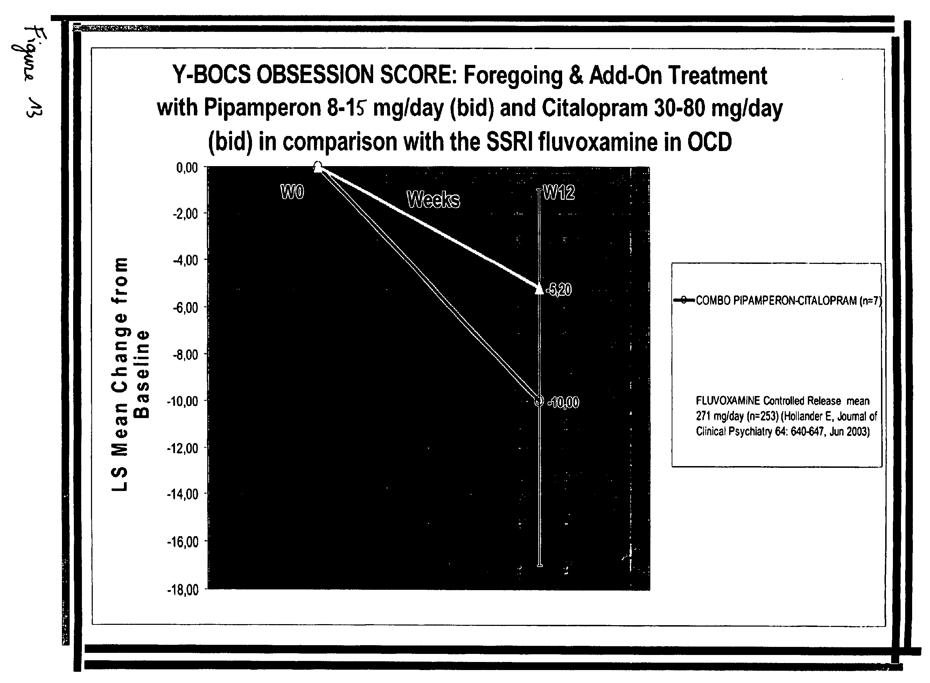

Figure 13 schematically depicts the Y-BOCS obsession score: "fore-going" and "add-on" treatment with pipamperon (8-15 mg/day; bid) and citalopram (30-80 mg/day; bid) in comparison with the SSRI fluvoxamine in OCD. Treatment with the combo pipamperon-citalopram (n = 7). Treatment with fluvoxamine (controlled release) mean 271 mg/day (n = 253) is according to Hollander et al. (2003).

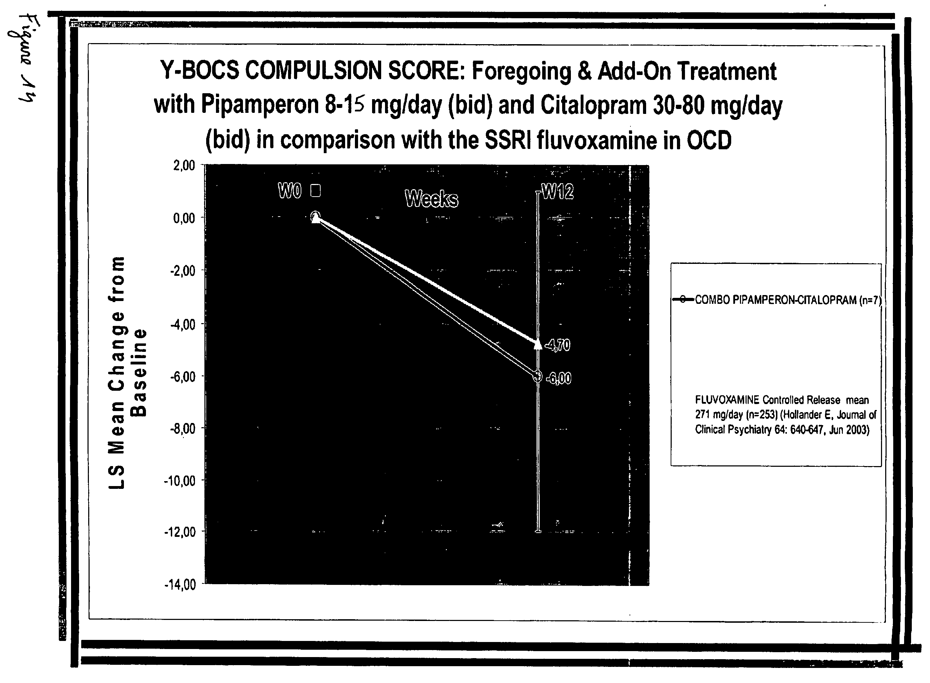

Figure 14 schematically depicts the Y-BOCS compulsion score: "fore-going" and "add-on" treatment with pipamperon (8-16 mg/day; bid) and citalopram (30-80 mg/day; bid) in comparison with the SSRI fluvoxamine in OCD. Treatment with the combo pipamperon-citalopram (n = 7). Treatment with fluvoxamine (controlled release) mean 271 mg/day (n = 253) is according to Hollander et al. (2003).

The intention-to-treat / last-observation-carried-forward analysis showed a high therapeutic efficacy according Y-BOCS total score, obsession and compulsion scores. This indicates the boosting effect of pipamperon on the SSRI citalopram at an extremely and thus unconventional low dose. No patient discontinued treatment.

### Assessment

**Efficacy:** the combo pipamperone 8-16mg/d - citalopram 30-80mg/day > the in the art known compounds effective towards OCD (Hollander E, Koran LM, Goodman WK, Greist JH, Ninan PT, et al. A double-blind, placebo-controlled study of the efficacy and safety of controlled-release fluvoxamine in patients with obsessive-compulsive disorder. Journal of Clinical Psychiatry 64: 640-647, Jun 2003 Mount Sinai School of Medicine, New York, New York, USA; Solvay Pharmaceuticals Inc., Marietta, Georgia, USA).

### Study Messages

The boosting effect of pipamperon at an extremely unconventional low dose on a SSRI is indicated since the efficacy of the 'add-on' and 'fore-going' combo 'pipamperon 8-15 mg/d - citalopram 30-80 mg/day' is in this study as twice higher as known in the art in the treatment of patients with obsessive-compulsive disorder.

### Example 5: combo pipamperon-citalopram: therapeutic use in Panic Disorder.

### Purpose

Preliminary examination of a "fore-going" and "add-on" treatment with pipamperon and citalopram in comparison with the SSRI in Panic Disorder.

### Results

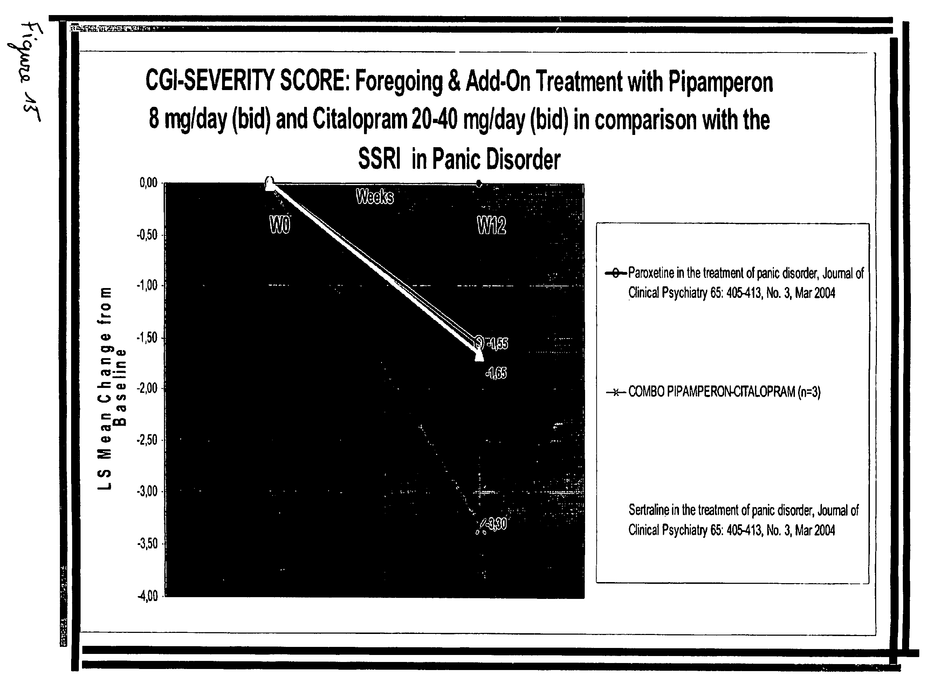

The results are indicated in Figure 15. Figure 15 schematically depicts the CGI-severity score: "fore-going" and "add-on" treatment with pipamperon (8 mg/day; bid) and citalopram (20-40 mg/day; bid) in comparison with the SSRI in Panic Disorder. Treatment with the combo pipamperon-citalopram (n = 3). Treatment with paroxetine is according to the Journal of Clinical Psychiatry (2004) 65: 405-413. Treatment with Sertraline is according to the Journal of Clinical Psychiatry (2004) 65: 405-413.

### Conclusion

Notably, although a small test group has been used (n = 3), the distribution around the mean is good. It will further be apparent from Figure 15 that the effect of the combo treatment of pipamperon and citalopram is twice as high as the standard treatments with paroxetine or sertraline.

### Example 6: POC process for mayor depressive disorder

Concept: Combo of the high selective 5-HT2A/D4 antagonist pipamperon with:
- a compound active towards the Amino Acid Transmitter, Peptidergic Transmitter, Adenosine Transmitter, Endocrine and/ or Enzymatic System;
- a fore-going admission during 4 days of pipamperon;
- a dose of pipamperon of 12 mg/day

Objectives: Demonstrating that this combo therapy has:
- the potency of being a treatment standard for depression by having an added value of reducing the total score of the Hamilton Depression Rating Scale - 17 items (HDRS-17) after 8 weeks of therapy with a least 20% more than reached with the conventional known antidepressants, i.e. 60% versus 40%. This stands for an added medium demission of 5 points on the total score of the HDRS-17 and by this will be very highly significant since the mean difference in all recent clinical trials between placebo and active treatment is 2.5;
- a more sustained therapeutic effect than the conventional mono therapy by preventing significant more relapses during 48 weeks following the acute treatment; and/or
- a complete neutral safety profile, e.g. there are no more adverse events in the combo therapy then in mono admission of the in the combo used antidepressant compound.

Process: the following different steps were implemented to reach out for these objectives (see also Tables 3 and 4):
(1) an naturalistic open label study (n=>20) on a depressive population with a normal variability of medical and psychiatric history, course of depression, earlier and concomitant therapy admitting the golden standard antidepressant citalopram 20-40 mg/day and a dose of 8-12 mg/day of pipamperon in a foregoing, simultaneous or add-on use.
(2) a 16 weeks placebo controlled randomised four armed study of each 36 patients with a mayor depressive disorder admitting:
   - from day 0: placebo or pipamperon (PIP) 10 mg/day or an active antidepressant compound or the combination of the last two;
   - from day 4: placebo or pipamperon 10 mg/day combined with an active antidepressant compound or an active antidepressant compound without pipamperon.

   By including rigorous control groups (placebo and active comparator; see Tables 3 and 4) this clinical trial is evaluated as a proof of concept of the added value of the combo and the foregoing treatment method since the inclusion/exclusion of:
   - a negative trial, i.e. no significant difference between the placebo and active treatment with the comparator;
   - a failed trial, i.e. no significant difference between the active and the studied treatment i.e. the combo.
(3) an active controlled randomised relapse prevention study following the POC trial during another 36 weeks with three arms of each 36 patients which is formed by:
   - continuation of the active mono therapy;
   - randomising the patients with a combo therapy in a group with an active mono therapy and with a continuation of the combo treatment.

## Claims

1. Use of pipamperone or a pharmaceutically acceptable salt thereof for the preparation of a medicament for treating a mood disorder or anxiety disorder, wherein said pipamperone or a pharmaceutically acceptable salt is to be administered to a patient in a daily dose ranging between 5 and 15 mg of the active ingredient, and wherein a second compound is to be administered simultaneously with, separate from or sequential to said pipamperone to augment the therapeutic effect of said second compound, said second compound is selected from the group consisting of: selective serotonin, nor-adrenaline and dopamine re-uptake inhibitor (SNDRI), selective serotonin and nor-adrenaline re-uptake inhibitor (SNRI) and selective serotonin re-uptake inhibitor (SSRI).

2. The use according to claim 1, wherein said pipamperon is to be administered daily at least one day before administering said second compound.

3. The use of pipamperone or a pharmaceutically acceptable salt thereof according to any of claims 1 to 2, wherein said second compound is a selective serotonin, nor adrenaline and dopamine re-uptake inhibitor (SNDRI) compound.

4. The use according to claim 3, wherein said selective serotonin, nor-adrenaline and dopamine re-uptake inhibitor (SNDRI) compound is selected from the group consisting of NS 2330, McN 5652, DOV 216,303 and DOV 21,947, or a pharmaceutically acceptable salt thereof.

5. A pharmaceutical composition comprising (a) pipamperone, and (b) a selective serotonin, nor-adrenaline and dopamine re-uptake inhibitor (SNDRI) compound, as a combined preparation for simultaneous, separate or sequential use for treating the underlying emotion dysregulation of mental disease or disorder which is chosen from the group consisting of mood disorders and anxiety disorders, wherein said pipamperone is to be administered to a patient in a daily dose of between 5 and 15 mg of the active ingredient.

6. The pharmaceutical composition according to claim 5, wherein said selective serotonin, nor-adrenaline and dopamine re-uptake inhibitor (SNDRI) compound is selected from the group consisting of NS 2330, McN 5652, DOV 216,303 and DOV 21,947, or a pharmaceutically acceptable salt thereof

7. The use of pipamperone or a pharmaceutically acceptable salt thereof according to any of claims 1 to 2, wherein said second compound is a selective serotonin and nor-adrenaline re-uptake inhibitor (SNRI) compound.

8. The use of pipamperone or a pharmaceutically acceptable salt thereof according to claim 7, wherein said selective serotonin and nor-adrenaline re-uptake inhibitor (SNRI) compound is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof.

9. The use of pipamperone or a pharmaceutically acceptable salt thereof according to claim 8, wherein said venlafaxine, or a pro-drug or an active metabolite thereof, or a pharmaceutically acceptable salt thereof, is to be administered in a daily dose ranging between 75 and 300 mg of the active ingredient.

10. The use of pipamperone or a pharmaceutically acceptable salt thereof according to claim 8, wherein said tomoxetine, or a pro-drug or an active metabolite thereof, or a pharmaceutically acceptable salt thereof, is to be administered in a daily dose ranging between 0.475 and 3.8 mg/kg of the active ingredient.

11. The use of pipamperone or a pharmaceutically acceptable salt thereof according to claim 8, wherein said milnacipran, or a pharmaceutically acceptable salt thereof, is to be administered in a daily dose ranging between 50 and 200 mg of the active ingredient.

12. The use of pipamperone or a pharmaceutically acceptable salt thereof according to claim 8, wherein said duloxetine, or a pharmaceutically acceptable salt thereof, is to be administered in a daily dose ranging between 40 and 60 mg of the active ingredient.

13. A pharmaceutical composition comprising (a) pipamperone and (b) a selective serotonin and nor-adrenaline re-uptake inhibitor (SNRI) compound as a combined preparation for simultaneous, separate or sequential use for treating the underlying emotion dysregulation of mental disease or disorder which is chosen from the group consisting of mood disorders and anxiety disorders, wherein said pipamperone is to be administered to a patient in a daily dose of between 5 and 15 mg of the active ingredient.

14. The pharmaceutical composition according to claim 13, wherein said selective serotonin and nor-adrenaline re-uptake inhibitor (SNRI) compound is selected from the group consisting of venlafaxine, tomoxetine, tandamine, talsupram, talopram, nefazodone, milnacipran, LY 113.821, duloxetine, desvenlafaxine and amoxapine, or a pharmaceutically acceptable salt thereof,

15. The pharmaceutical composition according to claim 14, wherein said venlafaxine, or a pharmaceutically acceptable salt thereof, provided in a unitary dose of between 75 and 300 mg of the active ingredient:

16. The pharmaceutical composition according to claim 14, wherein said tomoxetine, or a pharmaceutically acceptable salt thereof, is provided in a unitary dose of between 0.475 and 3.8 mg/kg of the active ingredient.

17. The pharmaceutical composition according to claim 14, wherein said milnacipran, or a pharmaceutically acceptable saft thereof, is provided in a unitary dose of between 50 and 200 mg of the active ingredient.

18. The pharmaceutical composition according to claim 14, wherein said duloxetine, or a pharmaceutically acceptable salt thereof, is provided in a unitary dose of between 40 and 60 mg of the active ingredient.

19. The use of pipamperone or a pharmaceutically acceptable salt thereof according to any of claims 1 to 2, wherein said second compound is a selective serotonin reuptake inhibitor (SSRI) compound.

20. The use of pipamperone or a pharmaceutically acceptable salt thereof according to claim 19, wherein said selective serotonin re-uptake inhibitor (SSRI) compound is selected from the group consisting of YM 992, VPI-013 (OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, cericlamine and ademethionine (preferably S-adenosylmethionine), or a pharmaceutically acceptable salt thereof.

21. The use of pipamperone or a pharmaceutically acceptable salt thereof according to claim 20, wherein said selective serotonin re-uptake inhibitor (SSRI) compound is uvoxamine (controlled release formulation), or a pharmaceutically acceptable salt thereof, is to be administered in a daily dose ranging between 100 and 300 mg of the active ingredient.

22. The use of pipamperone or a pharmaceutically acceptable salt thereof according to claim 20, wherein said selective serotonin re-uptake inhibitor (SSRI) compound is escitalopram, and is to be administered in a daily dose ranging between 10 and 20 mg of the active ingredient.

23. The use of pipamperone or a pharmaceutically acceptable salt thereof according to claim 20, wherein said selective serotonin re-uptake inhibitor (SSRI) compound is citalopram, and is to be administered in a daily dose ranging between 10 and 40 mg of the active ingredient.

24. A pharmaceutical composition comprising (a) pipamperone and (b) a selective serotonin re-uptake inhibitor (SSRI) compound as a combined preparation for simultaneous, separate or sequential use for treating the underlying emotion dysregulation of mental disease or disorder which is chosen from the group consisting of mood disorders and anxiety disorders, wherein said pipamperone is to be administered to a patient in a daily dose of between 5 and 15 mg of the active ingredient.

25. The pharmaceutical composition according to claim 24, wherein said selective serotonin re-uptake inhibitor (SSRI) compound is selected from the group consisting of YM 992, VPI-013 (also known as OPC-14523), sertraline, paroxetine, LY 214.281, LU AA 21-004, Lu 35-138, litoxetine, ifoxetine, fluvoxamine (controlled release formulation), fluvoxamine, fluoxetine, femoxetine, escitalopram, EMD 68843, cyanodothepine, citalopram, venlafaxine, milnacipran, duloxetine, cericlamine and ademethionine (preferably s-adenosylmethionine), or a pharmaceutically acceptable salt thereof.

26. The pharmaceutical composition according to claim 25, wherein said fluvoxamine (controlled release formulation), or a pharmaceutically acceptable salt thereof, is provided in a unitary dose of between 100 and 300 mg of the active ingredient.

27. The pharmaceutical composition according to claim 25, wherein said escitalopram, or a pharmaceutically acceptable salt thereof, is provided in a unitary dose of between 10 and 20 mg of the active ingredient.

28. The pharmaceutical composition according to claim 25, wherein said citalopram, or a pharmaceutically acceptable salt thereof, is provided in a unitary dose of between 10 and 40 mg of the active ingredient.

## Patentansprüche

1. Verwendung von Pipamperon oder eines pharmazeutisch verträglichen Salzes davon zur Darstellung von einem Medikament für die Behandlung einer affektiven Störung oder einer Angststörung, wobei das Pipamperon oder ein pharmazeutisch verträgliches Salz an einen Patienten in einer täglichen Dosis zu verabreichen ist, die in einem Bereich zwischen 5 und 15 mg des Wirkstoffes liegt, und wobei eine zweite Verbindung gleichzeitig mit, getrennt von oder aufeinanderfolgend zu dem Pipamperon zu verabreichen ist, um die therapeutische Wirkung von der zweiten Verbindung zu verstärken, und wobei die zweite Verbindung ausgewählt ist aus der Gruppe bestehend aus: selektive Serotonin-, Noradrenalin- und Dopamin-Wiederaufnahmehemmer (SNDRI), selektive Serotonin- und Noradrenalin-Wiederaufnahmehemmer (SNRI) und selektive Serotonin-Wiederaufnahmehemmer (SSRI).

2. Verwendung nach Anspruch 1, wobei das Pipamperon täglich zu verabreichen ist, mindestens einen Tag vor der Verabreichung der zweiten Verbindung.

3. Verwendung von Pipamperon oder eines pharmazeutisch verträglichen Salzes davon nach einem der Ansprüche 1 oder 2, wobei die zweite Verbindung eine selektive Serotonin-, Noradrenalin- und Dopamin-Wiederaufnahmehemmer- (SNDRI-) Verbindung ist.

4. Verwendung nach Anspruch 3, wobei die selektive Serotonin-, Noradrenalin- und Dopamin-Wiederaufnahmehemmer-(SNDRI-) Verbindung ausgewählt ist aus der Gruppe, die aus NS 2330, McN 5652, DOV 216,303 und DOV 21,947 oder einem pharmazeutisch verträglichen Salz davon besteht.

5. Pharmazeutische Zusammensetzung, die (a) Pipamperon und (b) eine selektive Serotonin-, Noradrenalin- und Dopamin-Wiederaufnahmehemmer- (SNDRI-) Verbindung als ein Kombinationspräparat umfasst, zur gleichzeitigen, getrennten oder aufeinanderfolgenden Verwendung bei der Behandlung der zugrunde liegenden Emotionsdysregulation der seelischen Erkrankung oder Störung, welche ausgewählt ist aus der Gruppe, die aus affektiven Störungen und Angststörungen besteht, wobei das Pipamperon an einen Patienten in einer täglichen Dosis von zwischen 5 und 15 mg des Wirkstoffes zu verabreichen ist.

6. Pharmazeutische Zusammensetzung nach Anspruch 5, wobei die selektive Serotonin-, Noradrenalin- und Dopamin-Wiederaufnahmehemmer- (SNDRI-) Verbindung ausgewählt ist aus der Gruppe, die aus NS 2330, McN 5652, DOV 216, 303 und DOV 21,947 oder einem pharmazeutisch verträglichen Salz davon besteht.

7. Verwendung von Pipamperon oder eines pharmazeutisch verträglichen Salzes davon nach einem der Ansprüche 1 oder 2, wobei die zweite Verbindung eine selektive Serotonin- und Noradrenalin-Wiederaufnahmehemmer- (SNRI-) Verbindung ist.

8. Verwendung von Pipamperon oder einem pharmazeutisch verträglichen Salz davon nach Anspruch 7, wobei die selektive Serotonin- und Noradrenalin-Wiederaufnahmehemmer- (SNRI-) Verbindung ausgewählt ist aus der Gruppe, die aus Venlafaxin, Tomoxetin, Tandamin, Talsupram, Talopram, Nefazodon, Milnacipran, LY 113.821, Duloxetin, Desvenlafaxin und Amoxapin oder einem pharmazeutisch verträglichen Salz davon besteht.

9. Verwendung von Pipamperon oder einem pharmazeutisch verträglichen Salz davon nach Anspruch 8, wobei das Venlafaxin oder eine Pro-Drug oder ein aktiver Metabolit davon, oder ein pharmazeutisch verträgliches Salz davon, in einer täglichen Dosis zu verabreichen ist, die in einem Bereich zwischen 75 und 300 mg von dem Wirkstoff liegt.

10. Verwendung von Pipamperon oder einem pharmazeutisch verträglichen Salz davon nach Anspruch 8, wobei das Tomoxetin oder eine Pro-Drug oder ein aktiver Metabolit davon, oder ein pharmazeutisch verträgliches Salz davon, in einer täglichen Dosis zu verabreichen ist, die in einem Bereich zwischen 0,475 und 3,8 mg/kg von dem Wirkstoff liegt.

11. Verwendung von Pipamperon oder einem pharmazeutisch verträglichen Salz davon nach Anspruch 8, wobei das Milnacipran oder ein pharmazeutisch verträgliches Salz davon in einer täglichen Dosis zu verabreichen ist, die in einem Bereich zwischen 50 und 200 mg von dem Wirkstoff liegt.

12. Verwendung von Pipamperon oder einem pharmazeutisch verträglichen Salz davon nach Anspruch 8, wobei das Duloxetin oder ein pharmazeutisch verträgliches Salz davon in einer täglichen Dosis zu verabreichen ist, die in einem Bereich zwischen 40 und 60 mg von dem Wirkstoff liegt.

13. Pharmazeutische Zusammensetzung, die (a) Pipamperon und (b) eine selektive Serotonin- und Noradrenalin-Wiederaufnahmehemmer- (SNDRI-) Verbindung als ein Kombinationspräparat umfasst, zur gleichzeitigen, getrennten oder aufeinanderfolgenden Verwendung bei der Behandlung der zugrunde liegenden Emotionsdysregulation der seelischen Erkrankung oder Störung, welche ausgewählt ist aus der Gruppe, die aus affektiven Störungen und Angststörungen besteht, wobei das Pipamperon an einen Patienten in einer täglichen Dosis von zwischen 5 und 15 mg des Wirkstoffes zu verabreichen ist.

14. Pharmazeutische Zusammensetzung nach Anspruch 13, wobei die selektive Serotonin- und Noradrenalin-Wiederaufnahmehemmer- (SNRI-) Verbindung ausgewählt ist aus der Gruppe, die aus Venlafaxin, Tomoxetin, Tandamin, Talsupram, Talopram, Nefazodon, Milnacipran, LY 113.821, Duloxetin, Desvenlafaxin und Amoxapin oder einem pharmazeutisch verträglichen Salz davon besteht.

15. Pharmazeutische Zusammensetzung nach Anspruch 14, wobei das Venlafaxin oder ein pharmazeutisch verträgliches Salz davon in einer Einheitsdosis von zwischen 75 und 300 mg des Wirkstoffs bereitgestellt wird.

16. Pharmazeutische Zusammensetzung nach Anspruch 14, wobei das Tomoxetin oder ein pharmazeutisch verträgliches Salz davon in einer Einheitsdosis von zwischen 0,475 und 3,8 mg/kg des Wirkstoffs bereitgestellt wird.

17. Pharmazeutische Zusammensetzung nach Anspruch 14, wobei das Milnacipran oder ein pharmazeutisch verträgliches Salz davon in einer Einheitsdosis von zwischen 50 und 200 mg des Wirkstoffs bereitgestellt wird.

18. Pharmazeutische Zusammensetzung nach Anspruch 14, wobei das Duloxetin oder ein pharmazeutisch verträgliches Salz davon in einer Einheitsdosis von zwischen 40 und 60 mg des Wirkstoffs bereitgestellt wird.

19. Verwendung von Pipamperon oder eines pharmazeutisch verträglichen Salzes davon nach einem der Ansprüche 1 oder 2, wobei die zweite Verbindung eine selektive Serotonin-Wiederaufnahmehemmer- (SSRI-) Verbindung ist.

20. Verwendung von Pipamperon oder eines pharmazeutisch verträglichen Salzes davon nach Anspruch 19, wobei die selektive Serotonin-Wiederaufnahmehemmer- (SSRI-) Verbindung ausgewählt ist aus der Gruppe, die aus YM 992, VPI-013 (OPC-14523), Sertralin, Paroxetin, LY 214.281, LU AA 21-004, Lu 35-138, Litoxetin, Ifoxetin, Fluvoxamin (Formulierung mit kontrollierter Wirkstofffreisetzung), Fluvoxamin, Fluoxetin, Femoxetin, Escitalopram, EMD 68843, Cyanodothepin, Citalopram, Venlafaxin, Milnacipran, Duloxetin, Cericlamin und Ademethionin (vorzugsweise S-Adenosylmethionin) oder einem pharmazeutisch verträglichen Salz davon besteht.

21. Verwendung von Pipamperon oder einem pharmazeutisch verträglichen Salz davon nach Anspruch 20, wobei die selektive Serotonin-Wiederaufnahmehemmer- (SRRI-) Verbindung Fluvoxamin (Formulierung mit kontrollierter Wirkstofffreisetzung) oder ein pharmazeutisch verträgliches Salz davon ist, die in einer täglichen Dosis zu verabreichen ist, welche in einem Bereich zwischen 100 und 300 mg von dem Wirkstoff liegt.

22. Verwendung von Pipamperon oder einem pharmazeutisch verträglichen Salz davon nach Anspruch 20, wobei die selektive Serotonin-Wiederaufnahmehemmer- (SRRI-) Verbindung Escitalopram ist und die in einer täglichen Dosis zu verabreichen ist, welche in einem Bereich zwischen 10 und 20 mg von dem Wirkstoff liegt.

23. Verwendung von Pipamperon oder einem pharmazeutisch verträglichen Salz davon nach Anspruch 20, wobei die selektive Serotonin-Wiederaufnahmehemmer- (SRRI-) Verbindung Citalopram ist und die in einer täglichen Dosis zu verabreichen ist, welche in einem Bereich zwischen 10 und 40 mg von dem Wirkstoff liegt.

24. Pharmazeutische Zusammensetzung, die (a) Pipamperon und (b) eine selektive Serotonin-Wiederaufnahmehemmer- (SSRI) Verbindung als ein Kombinationspräparat umfasst, zur gleichzeitigen, getrennten oder aufeinanderfolgenden Verwendung bei der Behandlung der zugrunde liegenden Emotionsdysregulation der seelischen Erkrankung oder Störung, welche ausgewählt ist aus der Gruppe, die aus affektiven Störungen und Angststörungen besteht, wobei das Pipamperon an einen Patienten in einer täglichen Dosis von zwischen 5 und 15 mg des Wirkstoffes zu verabreichen ist.

25. Pharmazeutische Zusammensetzung nach Anspruch 24, wobei die selektive Serotonin-Wiederaufnahmehemmer- (SSRI-) Verbindung ausgewählt ist aus der Gruppe, die aus YM 992, VPI-013 (ebenfalls bekannt als OPC-14523), Sertralin, Paroxetin, LY 214.281, LU AA 21-004, Lu 35-138, Litoxetin, Ifoxetin, Fluvoxamin (Formulierung mit kontrollierter Wirkstofffreisetzung), Fluvoxamin, Fluoxetin, Femoxetin, Escitalopram, EMD 68843, Cyanodothepin, Citalopram, Venlafaxin, Milnacipran, Duloxetin, Cericlamin und Ademethionin (vorzugsweise S-Adenosylmethionin) oder einem pharmazeutisch verträglichen Salz davon besteht.

26. Pharmazeutische Zusammensetzung nach Anspruch 25, wobei das Fluvoxamin (Formulierung mit kontrollierter Wirkstofffreisetzung) oder ein pharmazeutisch verträgliches Salz davon in einer Einheitsdosis von zwischen 100 und 300 mg des Wirkstoffs bereitgestellt wird.

27. Pharmazeutische Zusammensetzung nach Anspruch 25, wobei das Escitalopram oder ein pharmazeutisch verträgliches Salz davon in einer Einheitsdosis von zwischen 10 und 20 mg des Wirkstoffs bereitgestellt wird.

28. Pharmazeutische Zusammensetzung nach Anspruch 25, wobei das Citalopram oder ein pharmazeutisch verträgliches Salz davon in einer Einheitsdosis von zwischen 10 und 40 mg des Wirkstoffs bereitgestellt wird.

## Revendications

1. Utilisation de la pipampérone ou d'un sel pharmaceutiquement acceptable de celle-ci pour la préparation d'un médicament pour le traitement d'un trouble de l'humeur ou d'un trouble de l'anxiété, dans laquelle ladite pipampérone ou un sel pharmaceutiquement acceptable de celle-ci doit être administrée à un patient sous forme d'un dosage quotidien compris entre 5 et 15 mg de l'ingrédient actif, et dans laquelle un deuxième composé doit être administré simultanément à, séparément de ou séquentiellement à ladite pipampérone pour augmenter l'effet thérapeutique dudit deuxième composé, ledit deuxième composé étant sélectionné parmi le groupe constitué de l'inhibiteur du recaptage de la sérotonine, la noradrénaline et la dopamine sélectif (SNDRI), l'inhibiteur du recaptage de la sérotonine et la noradrénaline sélectif (SNRI) et l'inhibiteur du recaptage de la sérotonine sélectif (SSRI).

2. Utilisation selon la revendication 1, dans laquelle ladite pipampérone doit être administrée quotidiennement au moins un jour avant d'administrer ledit deuxième composé.

3. Utilisation de la pipampérone ou d'un sel pharmaceutiquement acceptable de celle-ci selon 1"une quelconque des revendications 1 ou 2, dans laquelle ledit deuxième composé est un composé inhibiteur du recaptage de la sérotonine, la noradrénaline et la dopamine sélectif (SNDRI).

4. Utilisation selon la revendication 3, dans laquelle ledit composé inhibiteur du recaptage de la sérotonine, la noradrénaline et la dopamine sélectif (SNDRI) est sélectionné parmi le groupe constitué de NS 2330, McN 5652, DOV 216,303 et DOV 21,947, ou un sel pharmaceutiquement acceptable de ceux-ci.

5. Composition pharmaceutique comprenant (a) de la pipampérone, et (b) un composé inhibiteur du recaptage de la sérotonine, la noradrénaline et la dopamine sélectif (SNDRI), en tant qu'une préparation combinée pour une utilisation simultanée, séparée ou séquentielle pour le traitement de la dérégulation des émotions sous-jacente d'une maladie ou d'un trouble mental qui est choisi(e) parmi le groupe constitué de troubles de l'humeur et de troubles de l'anxiété, dans laquelle ladite pipampérone doit être administrée à un patient sous forme d'un dosage quotidien compris entre 5 et 15 mg de l'ingrédient actif.

6. Composition pharmaceutique selon la revendication 5, dans laquelle ledit composé inhibiteur du recaptage de la sérotonine, la noradrénaline et la dopamine sélectif (SNDRI) est sélectionné parmi le groupe constitué de NS 2330, McN 5652, DOV 216,303 et DOV 21,947, ou un sel pharmaceutiquement acceptable de ceux-ci.

7. Utilisation de la pipampérone ou d'un sel pharmaceutiquement acceptable de celle-ci selon 1"une quelconque des revendications 1 ou 2, dans laquelle ledit deuxième composé est un composé inhibiteur du recaptage de la sérotonine et la noradrénaline sélectif (SNRI).

8. Utilisation de la pipampérone ou d'un sel pharmaceutiquement acceptable de celle-ci selon la revendication 7, dans laquelle ledit composé inhibiteur du recaptage de la sérotonine et la noradrénaline sélectif (SNRI) est sélectionné parmi le groupe constitué de venlafaxine, tomoxétine, tandamine, talsupram, talopram, néfazodone, milnacipran, LY 113.821, duloxétine, desvenlafaxine et amoxapine, ou un sel pharmaceutiquement acceptable de ceux-ci.

9. Utilisation de la pipampérone ou d'un sel pharmaceutiquement acceptable de celle-ci selon la revendication 8, dans laquelle ladite venlafaxine ou un promédicament ou un métabolite actif de celle-ci, ou un sel pharmaceutiquement acceptable de celle-ci, doit être administrée sous forme d'un dosage quotidien compris entre 75 et 300 mg de l'ingrédient actif.

10. Utilisation de la pipampérone ou d'un sel pharmaceutiquement acceptable de celle-ci selon la revendication 8, dans laquelle ladite tomoxétine ou un promédicament ou un métabolite actif de celle-ci, ou un sel pharmaceutiquement acceptable de celle-ci, doit être administrée sous forme d'un dosage quotidien compris entre 0,475 et 3,8 mg/kg de l'ingrédient actif.

11. Utilisation de la pipampérone ou d'un sel pharmaceutiquement acceptable de celle-ci selon la revendication 8, dans laquelle ledit milnacipran ou un sel pharmaceutiquement acceptable de celui-ci, doit être administré sous forme d'un dosage quotidien compris entre 50 et 200 mg de l'ingrédient actif.

12. Utilisation de la pipampérone ou d'un sel pharmaceutiquement acceptable de celle-ci selon la revendication 8, dans laquelle ladite duloxétine ou un sel pharmaceutiquement acceptable de celle-ci, doit être administrée sous forme d'un dosage quotidien compris entre 40 et 60 mg de l'ingrédient actif.

13. Composition pharmaceutique comprenant (a) de la pipampérone et (b) un composé inhibiteur du recaptage de la sérotonine et la noradrénaline sélectif (SNRI), en tant qu'une préparation combinée pour une utilisation simultanée, séparée ou séquentielle pour le traitement de la dérégulation des émotions sous-jacente d'une maladie ou d'un trouble mental qui est choisi(e) parmi le groupe constitué de troubles de l'humeur et de troubles de l'anxiété, dans laquelle ladite pipampérone doit être administrée à un patient sous forme d'un dosage quotidien compris entre 5 et 15 mg de l'ingrédient actif.

14. Composition pharmaceutique selon la revendication 13, dans laquelle ledit composé inhibiteur du recaptage de la sérotonine et la noradrénaline sélectif (SNRI) est sélectionné parmi le groupe constitué de venlafaxine, tomoxétine, tandamine, talsupram, talopram, néfazodone, milnacipran, LY 113.821, duloxétine, desvenlafaxine et amoxapine, ou un sel pharmaceutiquement acceptable de ceux-ci.

15. Composition pharmaceutique selon la revendication 14, dans laquelle ladite venlafaxine ou un sel pharmaceutiquement acceptable de celle-ci, est pourvue sous forme de dosage unitaire compris entre 75 et 300 mg de l'ingrédient actif.

16. Composition pharmaceutique selon la revendication 14, dans laquelle ladite tomoxétine ou un sel pharmaceutiquement acceptable de celle-ci, est pourvue sous forme de dosage unitaire compris entre 0,475 et 3,8 mg/kg de l'ingrédient actif.

17. Composition pharmaceutique selon la revendication 14, dans laquelle ledit milnacipran ou un sel pharmaceutiquement acceptable de celui-ci, est pourvu sous forme de dosage unitaire compris entre 50 et 200 mg de l'ingrédient actif.

18. Composition pharmaceutique selon la revendication 14, dans laquelle ladite duloxétine ou un sel pharmaceutiquement acceptable de celle-ci, est pourvue sous forme de dosage unitaire compris entre 40 et 60 mg de l'ingrédient actif.

19. Utilisation de la pipampérone ou d'un sel pharmaceutiquement acceptable de celle-ci selon l'une quelconque des revendications 1 ou 2, dans laquelle ledit deuxième composé est un composé inhibiteur du recaptage de la sérotonine sélectif (SSRI).

20. Utilisation de la pipampérone ou d'un sel pharmaceutiquement acceptable de celle-ci selon la revendication 19, dans laquelle ledit composé inhibiteur du recaptage de la sérotonine sélectif (SSRI) est sélectionné parmi le groupe constitué de YM 992, VPI-013 (OCP-14523), sertraline, paroxétine, LY 214.281, LU AA 21-004, Lu 35-138, litoxétine, ifoxétine, fluvoxamine (formulation à libération contrôlée), fluvoxamine, fluoxétine, fémoxétine, escitalopram, EMD 68843, cyanodothépine, citalopram, venlafaxine, milnacipran, duloxétine, cériclamine et adéméthionine (de préférence S-adénosylméthionine), ou un sel pharmaceutiquement acceptable de ceux-ci.

21. Utilisation de la pipampérone ou d'un sel pharmaceutiquement acceptable de celle-ci selon la revendication 20, dans laquelle ledit composé inhibiteur du recaptage de la sérotonine sélectif (SSRI) est la fluvoxamine (formulation à libération contrôlée), ou un sel pharmaceutiquement acceptable de celle-ci, et doit être administrée sous forme d'un dosage quotidien compris entre 100 et 300 mg de l'ingrédient actif.

22. Utilisation de la pipampérone ou d'un sel pharmaceutiquement acceptable de celle-ci selon la revendication 20, dans laquelle ledit composé inhibiteur du recaptage de la sérotonine sélectif (SSRI) est l'escitalopram, et doit être administré sous forme d'un dosage quotidien compris entre 10 et 20 mg de l'ingrédient actif.

23. Utilisation de la pipampérone ou d'un sel pharmaceutiquement acceptable de celle-ci selon la revendication 20, dans laquelle ledit composé inhibiteur du recaptage de la sérotonine sélectif (SSRI) est le citalopram, et doit être administré sous forme d'un dosage quotidien compris entre 10 et 40 mg de l'ingrédient actif.

24. Composition pharmaceutique comprenant (a) de la pipampérone et (b) un composé inhibiteur du recaptage de la sérotonine sélectif (SSRI), en tant qu'une préparation combinée pour une utilisation simultanée, séparée ou séquentielle pour le traitement de la dérégulation des émotions sous-jacente d'une maladie ou d'un trouble mental qui est choisi(e) parmi le groupe constitué de troubles de l'humeur et de troubles de l'anxiété, dans laquelle ladite pipampérone doit être administrée à un patient sous forme d'un dosage quotidien compris entre 5 et 15 mg de l'ingrédient actif.

25. Composition pharmaceutique selon la revendication 24, dans laquelle ledit composé inhibiteur du recaptage de la sérotonine sélectif (SSRI) est sélectionné parmi le groupe constitué de YM 992, VPI-013 (également connu comme OPC-14523), sertraline, paroxétine, LY 214.281, LU AA 21-004, Lu 35-138, litoxétine, ifoxétine, fluvoxamine (formulation à libération contrôlée), fluvoxamine, fluoxétine, fémoxétine, escitalopram, EMD 68843, cyanodothépine, citalopram, venlafaxine, milnacipran, duloxétine, cériclamine et adéméthionine (de préférence S-adénosylméthionine), ou un sel pharmaceutiquement acceptable de ceux-ci.

26. Composition pharmaceutique selon la revendication 25, dans laquelle ladite fluvoxamine (formulation à libération contrôlée), ou un sel pharmaceutiquement acceptable de celle-ci, est pourvue sous forme de dosage unitaire compris entre 100 et 300 mg de l'ingrédient actif.

27. Composition pharmaceutique selon la revendication 25, dans laquelle ledit escitalopram, ou un sel pharmaceutiquement acceptable de celui-ci, est pourvu sous forme de dosage unitaire compris entre 10 et 20 mg de l'ingrédient actif.

28. Composition pharmaceutique selon la revendication 25, dans laquelle ledit citalopram, ou un sel pharmaceutiquement acceptable de celui-ci, est pourvu sous forme de dosage unitaire compris entre 10 et 40 mg de l'ingrédient actif.
